**Europäisches Patentamt**

(19) **European Patent Office**

**Office européen des brevets**

(11) Publication number : **0 471 457 A2**

# (12) EUROPEAN PATENT APPLICATION

(21) Application number : **91306646.0**

(22) Date of filing : **22.07.91**

(51) Int. Cl.⁵ : **C12N 15/86, A61K 39/135, C12N 15/42**

(30) Priority : **24.07.90 US 556593**

(43) Date of publication of application :
**19.02.92 Bulletin 92/08**

(84) Designated Contracting States :
**AT BE CH DE DK ES FR GB GR IT LI LU NL SE**

(71) Applicant : **NOVAGENE, INC.**
**P.O. Box 270275**
**Houston Texas 77277 (US)**
(71) Applicant : **Baylor College of Medicine**
**Texas Medical Center 1200 Moursund Avenue**
**Houston Texas 77030 (US)**
(71) Applicant : **ELI LILLY AND COMPANY**
**Lilly Corporate Center**
**Indianapolis Indiana 46285 (US)**

(72) Inventor : **Dimarchi, Richard Dennis**
**3815 Wolf Creek Circle**
**Carmel, Indiana 46032 (US)**
Inventor : **Kit, Saul**
**11935 Wink Drive**
**Houston, Texas 77024 (US)**
Inventor : **Little, Sheila Parks**
**4629 Sunset Avenue**
**Indianapolis, Indiana 46208 (US)**
Inventor : **Gale, Charles**
**6627 East Pleasant Run Parkway, South Drive**
**Indianapolis, Indiana 46219 (US)**
Inventor : **Kit, Malon**
**11935 Wink Drive**
**Houston, Texas 77024 (US)**

(74) Representative : **Hudson, Christopher Mark et al**
**Erl Wood Manor**
**Windlesham Surrey GU20 6PH (GB)**

(54) **Herpesvirus-based viral vector which expresses a foot & mouth disease virus epitope.**

(57)    The present invention relates to a herpesvirus-based viral vector which expresses a foot and mouth disease epitope on the surface of virus-infected cells and on the surface of the virus particles. The viral vector of the present invention is useful, <u>inter alia</u>, as either a live or killed viral vaccine against foot and mouth disease.

EP 0 471 457 A2

FIELD OF THE INVENTION

The present invention relates to a herpesvirus-based viral vector which expresses a foot and mouth disease epitope on the surface of virus-infected cells and on the surface of the virus particles. The viral vector of the present invention is useful, inter alia, as either a live or killed viral vaccine against foot and mouth disease.

BACKGROUND OF THE INVENTION

I. Foot and Mouth Disease

Foot and mouth disease (hereinafter "FMD") is a highly contagious virus disease of domestic and wild cloven-hoofed animals. It also produces a persistent, inapparent infection in ruminants (Pereira, In: Viral Dieases of Food Animals, Gibbs ed., Vol. 2, Academic Press Inc., N.Y., pp. 333-363 (1981)). FMD is economically the most important virus disease of cattle. It is enzootic in most South American countries, where hundreds of outbreaks are recorded each year. FMD is also endemic in India and in many countries of the Middle East and Africa. FMD can spread rapidly due to its short incubation period and contagious nature and has a dramatic impact on livestock production and trade. The importance of FMD as a world-wide problem has long been recognized. Hence, International programs have been established, by the Office International de Epizootics, the Food and Agriculture Organization, the Pan American Health Organization, and the International Association of Biological Standardization, for the study and control of the disease.

The causative agent of FMD, i.e., FMD virus (hereinafter "FMDV") is classified in the Aphthovirus genus (from the Greek Aphtha "vesicles in the mouth"), within the family Picornaviridae. The Picornavirus family is characterized by particles with naked nucleocapsids approximately 25 nm in diameter and displaying icosahedral symmetry. The capsids are constructed from four major structural proteins, VP1 to VP4. Within the capsid is the virus genome represented by a single linear molecule of single-stranded, positive polarity RNA, about 8,000 nucleotides in length (Pereira, In: Viral Diseases of flood Animals, Gibbs ed., Vol. 2, Academic Press Inc., N.Y., pp. 333-363 (1981))). Aphthoviruses are distinguished from the other genera of the family Picornaviridae by certain physico-chemical properties, such as lability below pH 5.0 and relatively high buoyant density in CsCl (1.43 to 1.45), by possessing cross-reactive antigens common to all members of the genus, and by the capacity to cause FMD in cattle, buffalo, pigs, sheep, goats, and at least 27 other cloven-hoofed animal species.

Each of the polypeptides, VP1, VP2, and VP3, have molecular weights of about 26,000 daltons and are exposed on the capsid surface. On the other hand, VP4, a polypeptide having a molecular weight of about 8,000 daltons, is an internal protein which possesses an N-terminal myristic acid.

The protein referred to here as VP1 has been referred to by other workers as VP3 (Kleid et al, Science, 214: 1125-1129 (1981)). However, the most commonly accepted nomenclature is VP1. Thus, for purposes herein, this protein is designated VP1.

VP1 contains antigenic determinants able to induce neutralizing antibodies and to protect both swine and cattle from FMDV infection (Bachrach et al, J. Immmunol 115:1636-1651 (1975); and DiMarchi et al, Science, 232:639-641 (1986)). The immunogenic regions of VP1 are situated at amino acids 140 to 160 and amino acids 200 to 213 (Strohmaier et al, J. Gen. Virol., 59:395-306 (1982); Fox et al, J. Gen. Virol., 70:625-637 (1989); and Acharya et al, Nature, 337:709-716 (1989)). The immunogenic portion of VP1 forms a protrusion on the virus surface. The cell attachment site on the virus is also located in this region, probably at the arginine-glycine-aspartic acid residues at amino acids 145 to 147 and the amino acids at the C-terminal region of VP1 (amino acids 203 to 213).

The strategies employed to control FMD have depended on the prevalence of disease in a particular geographical region. In disease-free areas, efforts have been made to prevent introduction of FMD by prohibition or restriction of the importation of livestock and animals. The United States and Australia forbid the importation of potentially FMDV-infective materials, such as fresh meat. Even so, migrating birds may play a role in carrying the virus from one country to another, as from France and Holland to England. Furthermore, feral animals represent a natural reservoir of FMDV.

In the past, if disease has arisen due to the breakdown of restrictions, spread of disease has been promptly stopped by slaughter and destruction of all exposed and in-contact animals, their carcasses have been destroyed, the premises have been disinfected, and the movements of animal products, feedstuffs, and vehicles into or out of the premises have been restricted. FMDV can survive in meat products, so importation of whole carcasses has been forbidden from countries where FMD is endemic. Strict quarantines have been established and the areas have not been presumed to be safe until susceptible animals fail to develop FMD symptoms for 30 days.

Currently, livestock in several parts of the world where FMD is endemic are being treated with conventional killed vaccines. There are no commercially available live attenuated vaccines for FMD. The killed vaccine approach, although generally effective, has not been without problems. Compulsory regular vaccination combined with slaughter in Western European countries over the past 25 years covering more than 60 million cattle has reduced the incidence of the disease. However, occasions in which the virus was not completely inactivated or insufficiently attenuated have given rise to FMD outbreaks. Nucleotide sequence analyses have shown that recent outbreaks of FMD in Italy and Germany are traceable to vaccine which had escaped from laboratories.

Killed vaccines are prepared from chemically (usually binary-imine)-inactivated viruses. Protection is afforded by the induction of humoral antibodies. The majority of FMD vaccines are produced from virus cultivated either in surviving tongue epithelial tissue collected from animals slaughtered for meat, or in cell lines (e.g., baby hamster kidney) grown in suspension cultures. However, killed vaccines do not confer long-lasting immunity. One explanation for this is that the inactive vaccine virus is less competent than live virus with respect to the induction of a T cell response and the induction of T cell memory (Collen et al, J. Gen. Virol., 70:395-403 (1989)). In endemic areas, vaccination consists of 2 inoculations of inactivated FMDV two to four weeks apart. Further, livestock must be revaccinated two or three times each year to maintain protective levels of immunity (Pereira, In: Viral Diseases of Food Animals, Gibbs ed., Vol. 2, Academic Press Inc., N.Y., pp. 333-363 (1981)). Also, the killed vaccines are not highly purified and require adjuvants to enhance the antibody response. For example, inactivated vaccines may be adsorbed on to aluminum hydroxide and adjuvanted with saponin. Freund's complete adjuvant is an excellent adjuvant but cannot be used routinely on commercial herds because it causes too severe an inflammatory reaction. Following repeated vaccinations, hypersensitivity reactions may disadvantageously occur, requiring immediate treatment with adrenalin.

Another problem with present control measures for FMD involves the remarkable antigenic variability of FMDV (Dopazo et al, Proc. Natl. Acad. Sci., USA, 85:2041-2049 (1988)). Like other RNA viruses, FMDV has a high mutation rate. Seven serotypes and over 65 subtypes are known, necessitating either expensive polyvalent vaccines or vaccines appropriate to particular areas which may have to be changed rapidly to meet new strains arising in the field. The evolution of antigenic variants has been mimicked in vitro in studies where FMDV populations under antibody pressure have acquired electrophoretically altered VP1 and resistance to neutralization by antiviral sera (Carillo et al, Virol., 171:599-601 (1989); and Bolwell et al, J. Gen Virol., 70:45-57 (1989)). Viruses isolated from the field are often composed of mixed populations of antigenically distinguishable variants. Consequently, another potential source of antigenic variation is the selection involved in adaptation of virus field strains to growth in cultured cells. Thus, although much has been achieved with available vaccines, there is still a real need for alternative methods for producing antigens (de la Torre et al, J. Virol., 62:2050-2058 (1988)).

Synthetic peptides have been reported and are perceived as one alternative to conventional vaccines (Murdin, Vaccine, 4:210-211 (1986)). Several years ago, peptides corresponding to region 141 to 160 and 200 to 213 of the VP1 polypeptide of FMDV have been synthesized. The peptides have the ability to produce neutralizing antibodies in guinea pigs and rabbits (Bittle et al, Nature 298:30-33 (1982)).However, these peptides were found to protect only one of four cattle that received them (PCT Patent Publication No. 83/03547). Further, the synthetic peptides were found to exhibit only one-tenth the immunogenicity of killed virus particles.

Many of the synthetic peptide vaccines described to date are effective only in combination with proteins and Freund's adjuvant. A novel, completely synthetic virus peptide vaccine, consisting of a synthetic activator of T cells and macrophage, covalently linked to an amphophilic alpha-helical T-cell epitope has been developed (Wiesmuller et al, Vaccine, 7:29-33 (1989)). This low molecular weight vaccine is composed of a synthetic VP1 (135 to 154) amino acid sequence and the adjuvant tripalmitoyl-S-glyceryl-cysteinyl-serine, the synthetic analogue of the N-terminal portion of an E. coli lipoprotein. This vaccine was found to induce a long-lasting high protection against FMD in guinea pigs after a single administration without any additional protein or carrier, but has not been evaluated in cattle.

A peptide has been prepared in which amino acids 141 to 158 of serotype O of VP1, or the equivalent sequence of other serotypes, is linked through proline-proline-serine to amino acids 200 to 213, and on which cysteines were present at or near the amino and carboxy termini of the peptides, so as to permit polymerization and/or cyclization (Dimarchi et al, Science, 232:639-641 (1986)). This peptide, which is free of any carrier protein, has been found to elicit an unforeseen markedly enhanced neutralization response against FMDV (U.S. Patent 4,732,971). Also, this peptide has been found to induce protection in cattle with a single vaccination and provides complete protection after a repeat immunization. While the synthetic peptide vaccine offers many advantages, it requires adjuvants, and has not yet been perfected sufficiently to protect animals consistently with a single vaccination.

II. Recombinant Foot and Mouth Disease Vaccines

With the advent of recombinant DNA technology, other approaches to the production of FMDV antigens have become available. The DNA sequence coding capsid protein VP1 of FMDV (strain A12) has been ligated to a plasmid designed to express a chimeric protein from the E. coli tryptophan promoter-operator system (Kleid et al, Science, 214:1125-1129 (1981)). An insoluble protein is produced by this method and has been found to induce neutralizing antibodies in cattle and swine. Unfortunately, however, the VP1 produced in E. coli has been of very disappointing antigenicity, approximating that of VP1 produced by detergent extraction and gel electrophoresis of intact virus particles. It is believed that the VP1 produced in E. coli, does not have its chain folded in such a way as to present the important antigenic sites to the immune system.

Proteins consisting of one, two, or four copies of the amino acids 137 to 162 of FMDV attached to the N-terminus of E. coli β-galactosidase have been expressed in E. coli (Brockhuijen et al, J. Gen. Virol., 68:3137-3143 (1987)). In guinea pigs, the fusion protein emulsified in incomplete Freund's adjuvant and containing one copy of the FMDV determinant has been found to elicit only low levels of neutralizing antibody, whereas protective levels have been found to be elicited by the emulsified fusion proteins containing two or four copies of the FMDV determinant. Single inoculations of the fusion proteins containing two or four FMDV determinants have been found sufficient to protect guinea pigs and Deutsche Landrasse pigs against challenge infection. Choice of adjuvant was found to be important in that the substitution of aluminum hydroxide for incomplete Freund's adjuvant gave poor results.

The construction of bacterial expression systems comprising bacterial plasmids or bacteriophage lambda DNA fused to cDNA sequences encoding FMDV proteins has also been disclosed (U.S. Patent 4,743,554).

Finally, two recombinant vaccinia viruses expressing the FMDV VP1 epitope have been constructed to improve the presentation of FMDV epitopes to the immune system (Clarke et al, Nature 330:381-384 (1987); and Newton et al, In: Vaccines 87, Chanock et al eds., Cold Spring Harbor Laboratory, pp. 12-21 (1987)). In the first construction, advantage was taken of the fact that when human hepatitis B virus core antigen (HBcAg) is expressed in microbial cells, it self-assembles into 27 nm particles that are immunogenic in laboratory animals. Hence, the oligodeoxyribonucleotide sequence corresponding to FMDV VP1 142 to 160 was synthesized and then fused to the amino terminus of human HBcAg. This construct was inserted into the vaccinia thymidine kinase (hereinafter "tk") gene sequence adjacent to the vaccinia p11 K promoter sequence to obtain a tk⁻ recombinant vaccinia virus which expressed the FMDV epitope as a fusion protein with HBcAg. The ability of the FMD-HBcAg fusion protein to self-assemble into regular 27 nm core-like particles was confirmed by electron microscopic examination of immune complexes. The FMD-HBcAg fusion protein, on a weight basis, was found to be 30-40 times more immunogenic than the peptide/β-galactosidase fusion protein containing tandem copies of the FMDV epitope, 500 times more immunogenic than free synthetic peptide, almost as immunogenic as inactivated virus particles, and protected guinea pigs from FMDV challenge. However, the poor yield in the vaccinia virus expression system gives rise to insufficient material for detailed scientific investigations or commercial purposes.

The second recombinant virus consisted of a tk⁻ vaccinia virus expressing a fusion protein of the FMDV epitope and the human influenza virus hemagglutinin on the surface of vaccinia virus-infected cells. In this construct, the human influenza virus hemagglutin coding sequence, including the signal peptide, was cloned behind the vaccinia virus p11 K promoter and inserted into the vaccinia virus tk gene. Then, the human influenza virus hemagglutinin sequence downstream from the amino terminal end of the hemagglutinin and containing the immunogenic A site was replaced with the FMD VP1 epitope. To date, efficacy studies in animals have not been reported on this recombinant virus.

The present invention represents the first disclosure of the presentation of an FMDV viral epitope (e.g., VP1 amino acids 141 to 158 linked to amino acids 200 to 213) by fusion of such to the N-terminal of a major glycoprotein gene (e.g., gIII) of a non-human herpesvirus (e.g., bovine herpesvirus), such that the FMDV epitope is expressed not only on the surface of infected cells, but also on the surface of infectious viral particles.

III. Herpesviruses

Alpha herpesviruses have the special ability to enter into a dormant state, which does not occur with other families of viruses. That is, as an animal recovers from the initial generalized infection, alpha herpesviruses retreat to portions of the nervous system where they become quiescent and impervious to the body's immune system. This dormant infection, i.e., latency, may be unexpectedly reactivated, resulting in recrudescence of disease or in a contagious condition known as the carrier state, wherein the infected animal shows no outward symptoms of the disease but can transmit or "shed" infectious alpha herpesviruses intermittently, so as to cause the spread of infection.

Examples of alpha herpesviruses which infect animals that are susceptible to FMD include bovine herpesvirus type 1, more commonly known as infectious bovine rhinotracheitis virus (hereinafter "IBRV"); caprine herpesvirus (hereinafter "CapHV-1"), also known as bovid herpesvirus-6; bovine mammillitis virus, also known as bovid herpesvirus-2 (hereinafter "BHV-2"); and pseudorabies virus.

In addition to alpha herpesviruses, herpesviruses of the beta, gamma-1 and gamma-2 subfamilies which exhibit lymphotropic characteristics are known.

Examples of gamma-2 herpesviruses which infect animals that are susceptible to FMD include bovine herpesvirus type-4 (hereinafter "BHV-4); and malignant catarrhal fever (MCF) virus, also known as alcelaphine herpesvirus type 1 (hereinafter "AHV-1").

## A. Infectious Bovine Rhinotracheitis

IBRV has been associated with respiratory, reproductive, enteric, ocular, central nervous system, neonatal, mammary, and dermal infections of cattle (U.S. Patents 4,569,840, 4,703,011 and 4,824,667, which are incorporated by reference herein in their entirety). In natural outbreaks of the respiratory form of the disease, conjunctivitis is also prominent. Infectious pustular vulvovaginitis and balanoposthitis are likewise caused by IBRV. The spread of the disease in naturally and artificially bred cattle poses a serious problem, especially with the widespread use of frozen semen. Recurrent shedding of virus from infected bulls also constitutes a significant threat to the artificial insemination industry in the United States and to the worldwide distribution of bovine germ plasma. The incrimination of IBRV as an etiologic agent of oophoritis and salpingitis with resulting infertility and sterility adds to the seriousness of the infection. IBRV is widely recognized as a cause of abortion, stillbirths, and infertility. Meningoencephalitis is another of the sequels to IBRV infection.

IBRV infection of species other than cattle have been described. Natural infections occur in swine, goats, water buffalo, wildebeests, ferrets, and mink. Experimental infections have been established in swine fetuses, goats, mule deer, ferrets, and rabbits. Thus, IBRV infects many of the hosts that are also susceptible to FMDV.

Currently, three types of conventional vaccines are being employed to control IBR: (1) killed virus vaccines, (2) subunit vaccines, and (3) modified live virus (hereinafter "MLV") vaccines (U.S. Patents 3,925,544; 4,291,019; and 3,634,587). Killed IBR vaccines are produced by treating IBRV with chemical and/or physical agents, Subunit IBR vaccines are prepared by solubilizing IBRV-infected cell cultures with nonionic detergents. Early MLV vaccines were obtained by attenuating IBRV by rapid passage in bovine cell cultures, by adaption of IBRV to porcine or canine cell cultures, by adaption to growth in cell cultures at a low temperature (30°C), or by selection of heat-stable virus particles (56°C for 40 min). These MLV IBR vaccines have usually been administered parenterally to cattle. More recently, specialized types of MLV vaccines designed for intranasal administration have also been obtained. These specialized MLV vaccines are attenuated by serial passages in rabbit cell cultures or by treatment of IBRV with nitrous acid followed by selection for temperature-sensitive mutants. A temperature-sensitive virus is one that replicates efficiently at about 30°C to 38°C, but not at about 39°C to 41°C (Todd et al, J. Am. Vet. Med. Assoc., 159:1370-1374 (1971); Kahrs et al, J. Am. Vet. Med. Assoc 163:437-441 (1973); Zygraich et al, Res. Vet. Sci., 16:328-335 (1974); and U.S. Patents 3,907,986 and 4,132,775)).

Although the extensive use of conventional IBR vaccines has reduced the severity of IBR, all of the current vaccines have serious disadvantages. The killed and subunit vaccines are expensive to produce, they may require several injections with adjuvants, they do not prevent latent infections by virulent strains of IBRV with spread of disease-causing virus from animal to animal, and they are generally inferior to MLV vaccines in providing long-term protection. Further, in the case of killed IBR vaccines, some infectious particles may survive the killing process and cause disease.

With regard to conventional MLV vaccines, there has been concern as to their safety, especially if the vaccine virus itself produces latency and may be shed and transmitted to susceptible cattle. Vaccination with conventional MLV vaccines has also failed to prevent latent infections following exposure to virulent IBRV (Narita et al, Am. J. Vet. Res., 41:1995-1999 (1980)). Utilization of intramuscularly (hereinafter "IM") administered MLV IBR vaccines has been especially hampered by the hazards of vaccine-induced abortions. In addition, with conventional vaccines, which are attenuated only by serial passage of IBR in tissue culture cells, there is the danger of reversion to virulence.

The specialized MLV IBR vaccines developed for intranasal (hereinafter "IN") inoculation have been found to be immunogenic and safe for pregnant cattle and can prevent abortions in pregnant cows which have been challenge-exposed to virulent IBRV. However, they have a disadvantage in that IN vaccination of large numbers of cattle is inconvenient and potentially dangerous to animal handlers. Additionally, when administered IM, one temperature-sensitive IBRV vaccine has been found to replicate poorly or not at all at normal body temperatures (Zygraich et al, Res. Vet. Sci., 16:328-335 (1974)), while another IBR vaccine has been found to be insufficiently attenuated for IM administration to pregnant cows although safe when given IN (Todd et al, J. Am. Vet. Med. Assoc., 163:437-444 (1973)). Screening to identify pregnant animals prior to immunization is often not desirable or cost effective. Hence, development of vaccines which can be safely administered either IM or IN in stressed feedlot cattle, in breeding bulls, and in pregnant cows would facilitate disease prevention, be more cost effective, and be compatible with current management regimens has been desired.

## B. Bovine herpesvirus type-4

BHV-4 includes a large number of antigenically related isolates recovered from cattle with diverse clinical diseases, as well as from apparently healthy cattle and bovine fetuses. In contrast to BHV-1 and BHV-2, to date, BHV-4 has not been established as an etiological agent of a distinct disease entity and its pathogenic role remains unclear. However, several BHV-4 isolates have been recovered from genital diseases. Further, post-partum metritis has been observed in cows experimentally infected with BHV-4. These observations suggest that BHV-4 is associated with reproductive problems in cattle (Dubuisson et al, Vet. Microbiol., 21:97-114 (1989)). BHV-4 has been classified as a bovine cytomegalovirus because of its characteristic maturation process. However, DNA restriction endonuclease analyses have shown that BHV4 isolates, unlike cytomegaloviruses of other species, contain group B DNA genomes and encode a virus-specific TK (Ehlers et al, J. Gen Virol., 66:55-68 (1985); and Kit et al, Virus Res., 4:197-212 (1986)). These features are shared with the lymphotropic primate herpesviruses, saimiri and ateles. BHV-4 also possesses a tropism for lymphoid cells in the natural and experimental host. This is a characteristic property of viruses belonging to the gamma herpesvirinae subfamily. The association between BHV-4 and bovine lymphoid tissue, and its transport through lymphatic and hematogenous routes, is believed to explain the great diversity of tissues and symptoms from which BHV-4 has been recovered.

## C. Pseudorabies virus

Pseudorabies, a highly contagious disease of swine and other livestock, such as cattle, sheep, and goats, is caused by Herpesvirus suis (hereinafter "pseudorabies virus" or "PRV"). In swine, the disease causes respiratory illness and encephalitis which may progress to death. Other common consequences of infection in swine are abortions, neonatal demise, reduced litter size, and slower growth rates. In other livestock, most notably cattle, PRV infection almost invariably proceeds to a lethal encephalitis (U.S. Patents 4,514,497 and 4,609,548, which are incorporated herein by reference in their entirety).

Pseudorabies has become a major threat and cause of economic loss to the swine industry throughout the world. There is also considerable alarm over the spread of pseudorabies to cattle and other farm animals. Within the last ten years, economic losses have escalated because of the emergence of more virulent strains of PRV, and the widespread dissemination of the disease. Today, it is estimated that 8.0% of the 80 million hogs on farms in the United States are infected, in comparison to less than 0.8% a decade ago. Thus, PRV infects many of the hosts that are also susceptible to FMDV.

Previously, MLV PRV vaccines have been produced by multiple passages of the virus in chick and/or monkey tissue culture cells (Skoda et al, Acta Virol.,8:1-9 (1964); and Bartha, Magy. Allatorv. Lapja, 16:42-45 (1961)). During tissue culture passages, mutations accumulate as the virus adapts to its new environment. These undefined mutations adversely affect virus reproduction in the natural host, resulting in virus attenuation.

A problem with the above-described MLV PRV vaccines is that the animal often becomes a carrier of the dormant vaccine virus. As a result, usage of these vaccines can result in two undesirable situations which impede their safety and effectiveness. First, abortions, stillbirth, and fatal infections in newborns can be caused by some vaccine viruses as they are shed by vaccinated carriers. Second, the repeated circulation of vaccine virus within a herd can result in a reversal of the process of attenuation such that the vaccine virus reverts to the pathogenic parent strain. Under such circumstances, widespread vaccination will undesirably promote the dissemination of the disease.

In addition to the above-described disadvantages, the previously known PRV vaccines, while substantially minimizing symptoms of illness, do not prevent the animal from acquiring a dormant infection with pathogenic field strains. Thus, despite vaccination, an animal may become a carrier of the disease and transmit it to susceptible animals. These carriers of the disease, when moved between farms and market, will shed not only the dormant vaccine virus as discussed above, but also the disease virus. This results in the undesirable transmission of the disease across geographic barriers and state boundaries.

## D. Caprine herpesvirus

CapHV-1 is distributed world-wide and causes a generalized disease in young kids, mainly affecting the digestive tract (Kao et al, In: Immunity to Herpesvirus Infections in Domestic Animals, Report EUR 9737 EN, Pastoret et al, eds., Commission of the European Communities, Luxembourg, pp 93-97 (1985). Infection of adult goats remains inapparent or may cause abortion, vulvovaginitis or balanoposthitis. Although the restriction endonuclease map of CapHV-1 and BHV-1 differ significantly, the genome structures are identical. In addition, CapHV-1 and BHV-1 share a high degree of base sequence homology, suggesting a common ancestry (Engels et al, J. Gen. Virol., 68:2019-2023 (1987)). An antigenic relationship also exists between CapHV-1 and BHV-1.

## E. Alcelaphine herpesvirus type 1

AHV-1 is a herpesvirus of the wildebeest, Connochaetes Taurinus, which is an apparent carrier of the virus (Mushi et al, Res. Vet. Sci., 29:168-171 (1980); and Osorio et al, Am. J. Vet. Res., 46:2104-2109 (1985)). In free-living animals, the virus spreads through ocular and nasal secretions. As a result, all wildebeest calves over the age of 7 months are infected. AHV-1 infection of ruminants other than wildebeests, such as deer and cattle, leads to a fatal lymphoproliferative and degenerative disease known as malignant catarrhal fever. Economic losses in Africa and zoological parks can be severe. The disease is a world-wide phenomenon. Epidemiological evidence indicates that sheep are a second carrier of infection. However, attempts to isolate a virus capable of inducing malignant catarrhal fever from sheep have repeatedly failed. AHV-1 possess a double-stranded DNA genome with characteristics similar to that of BHV-4 and herpesvirus ateles. This indicates that AHV-1 should be included within the gamma-2 group of herpesviruses (Bridgen et al, J. Gen. Virol., 70:1141-1150 (1989)). Inactivated vaccines against malignant catarrhal fever have been prepared, but have not been found to be effective (Plowright et al, Res. Vet. Sci., 19:159-166 (1975)).

## F. Bovine herpesvirus type 2

BHV-2 is closely related to herpes simplex virus. BHV-2 causes bovine herpesvirus mammillitis, a seasonal disease of cattle seen primarily in October and November before cattle are confined in barns for the winter. BHV-2 causes swelling, inflammation and ulceration of the teats and udder in domestic cattle. Virus-induced lesions are usually restricted to the skin of the teats and udder despite the presence of the virus in internal tissues, the ability of the virus to replicate to high titers in bovine internal organ tissues in vitro, and the ability of the virus to establish latent infections (Letchworth et al, Infection and Immunity, 43:1072-1079 (1984)); and Catsrucci et al, Arch. Virol., 72:75-81 (1982)). Disease induced by BHV-2 is not a major problem in cattle. The virus encodes a thymidine kinase (hereinafter "TK") activity (Sheppard et al, J. Gen. Virol., 70:3067-3071 (1989)). Some strains produce no disease at all. Further, strains have not been shown to be transmitted to people or other animals in field situations.

## IV. Recombinant Herpesvirus Vaccines

Recently, recombinant DNA technology has been used to develop new IBR and PRV vaccines which overcome many of the problems that have limited the usefulness of conventional vaccines. Many experiments utilizing animal model systems have demonstrated that herpesvirus encoded tk genes are important for virulence (Klein, Arch. Virol., 72:143-160 (1982)). These studies have shown that tk⁻ HSV-1 mutants have reduced pathogenicity in mice, rabbits, and guinea pigs and are less likely to be reactivated from latency, but, nevertheless, protect animals against fatal infection from virulent tk⁺ viruses and reduce the probability that tk⁺ HSV-1 infection would establish latency in immunized animals. The tk⁻ mutants of HSV-2, Herpes tamarinus, and PRV are also less virulent than parental tk⁺ strains. Furthermore, restoration of tk⁺ function by the marker transfer techniques of recombinant DNA methodology causes a reversion to virulence (Kit et al, J. Med. Virol., 12:25-36 (1983); Kit et al, Am. J. Vet. Res., 46:1359-1367 (1985); and Stanberry et al, J. Virol., 55:322-328 (1985)). In addition to the above model experiments with laboratory animals, the attenuated properties of tk⁻ deletion mutants of PRV for natural hosts has been demonstrated (U.S. Patents 4,514,497; 4,690,548; 4,711,850; and 4,753,884; Kit et al, Am. J. Vet. Res., 46:1359-1367 (1985); and Kit et al, Am. J. Vet. Res., 48:780-793 (1987)). Furthermore, a mutagen-induced tk⁻ IBRV virus which replicates equally well at 39.1°C or 34.5°C has been shown to be safe and efficacious as an IBR vaccine after IN or IM or intravenous immunization of calves and after intravaginal or IM immunization of pregnant cows (Kit et al, Virol., 130:381-389 (1983); Kit et al, Arch. Virol., 86:63-84 (1985); Kit et al, Vaccine, 4:55-61 (1986); and U.S. Patent 4,569,840)).

tk⁻ herpesviruses with deletions in the tk gene are superior to spontaneous or mutagen-induced tk⁻ mutants for the following three reasons. First, herpesvirus deletion mutants have absolutely no TK-inducing activity. This is inappropriate for a vaccine because mutants with partial TK activity are often virulent. Second, tk⁻ herpesvirus deletion mutants cannot revert to tk⁺. In contrast, spontaneous or mutagen-induced tk⁻ mutants, especially those with partial TK activity, can revert to tk⁺ and, thus, to restored virulence. Third, tk⁻ herpesvirus deletion mutants can be distinguished from virulent field strains and from conventional vaccine strains by their tk⁻ phenotype, by their restriction endonuclease patterns, and by their Southern blotting molecular hybridization patterns (Kit et al, Am. J. Vet. Res., 46:1359-1367 (1985)). These distinctions have practical importance. For example, if a vaccinated animal develops disease, it is important to know whether the vaccine virus caused the disease or whether infection by a virulent field strain did so.

Based on these considerations, an IBRV mutant and a PRV mutant, both of which fail to produce any functional TK as a result of a deletion in the tk gene have been constructed (U.S. Patents 4,703,011, 4,824,667, 4,514,497 and 4,609,548). These mutants, designated IBRV(NG)dltk (ATCC No. VR-2112) and PRV(BUK-dl 3) (ATCC No. VR-2075), respectively, replicate equally well at 39.1°C or 34.5°C. Thus, they are not temperature-sensitive. In addition to the tk gene deletion in IBRV(NG)dltk, there is an insertion of a synthetic 40-mer oligonucleotide sequence (hereinafter "NG") with stop condons in all three reading frames in place of the deleted tk sequence. The insertion of the NG sequence provided further assurance that the mutant virus would produce absolutely no TK activity, and that the inactivation of the tk gene was irreversible. Also, the NG sequence provides a marker by which the genetically engineered IBRV(NG)dltk virus can be distinguished from all conventional IBRV vaccine strains.

## V. Eukaryotic Viral Vectors

The development and use of eukaryotic viral expression systems has become widespread in recent years. The limited capacity of microbial systems to express authentic and functional eukaryotic proteins is one factor which fuels these developments. Another is the desire to be able to study diverse aspects of gene expression with highly manipulatable, and yet physiological, model systems (Stephens et al, Biochem. J., 248:1-11 (1987)). Consequently, eukaryotic viral vector systems have been developed for studies of cellular regulation, for the introduction of genes into mammalian cells, for cloning propagation, and for high level expression of foreign genes. The foreign genes propagated and expressed in eukaryotic virus vectors may be useful to generate immunogens used as vaccines, to make antigens to be used in diagnostic kits, for the synthesis of protein hormones, lymphokines, and other immuno-modulators, as DNA probes in diagnostic kits, for the development of transgenic animals, and for gene replacement therapy. Cloning vectors derived from animal viruses have been described in the art (Rigby, J. Gen. Virol., 64:255-266 (1983)).

Eukaryotic-based viral vectors have been developed utilizing Simian virus 40 (Mulligan et al, Science, 209:1422-1427 (1980); and Clarke et al, Gene, 65:23-30 (1988)); bovine papilloma virus (Stephens et al, Biochem J. 248:1-11 (1987)); human papovavirus BK (Manservigi et al, Virol 167:284-288 (1988)); baculoviruses (Matsuura et al, J. Gen. Virol., 68:1233-1250 (1987); Kang et al, J. Gen. Virol., 68:2607-2613 (1987); Makino et al, J. Gen. Virol., 70:1417-1425 (1989); Jeong et al, J. Virol., 62:3874-3878 (1988); and van Wyke Coelingh et al, Virol., 160:465-472 (1987)); Autographa californica nuclear polyhedroses (insect) virus (Hu et al, J. Virol., 61:3617-3620 (1987)); Bombyx mori nuclear polyhedrosis (insect) virus (Marmumuto et al, J. Gen. Virol., 68:2599-2606 (1987)); adeno-associated virus (La Face et al, Virol 162:483-486 (1988)); adenoviruses (Berkner, Biotechniques, 6:616-629 (1988); Prevec al, J. Gen Virol., 70:429-434 (1989); and McDermott et al, Virol., 169:244-247 (1989)); and retroviruses (Eglitis et al, Biotechniques, 6:608-614 (1988)). Herpesvirus-based vector systems include the herpes simplex virus (Smith et al, Proc. Natl. Acad. Sci. USA, 81:5867-5870 (1984); and Geller et al, Science 241:1667-1669 (1988)); herpesvirus saimiri (Desrosiers et al, Mol. Cell. Biol,. 5:2796-2803 (1985); and Grassmann et al, J. Virol., 63:1818-1821 (1989)); and Epstein-Barr virus (Story et al, J. Virol., 63:3837-3843 (1989)).

The use of viral-based vectors as vaccines is advantageous over subunit vaccines because adjuvants are not required, a single vaccination suffices for immunization, and replication of the viral-based vectors within the host amplifies the amount of foreign antigen being expressed thereby, often increasing both cell-mediated and humoral immune responses to the foreign antigen. In addition, the expression of foreign antigens within infected cells of the immunized host provides for post-translational modifications of the foreign antigen and thus antigen presentation more closely resembling that occurring during natural infection with the pathogen from which the foreign antigen is derived. Further, the use of a safe, live-attenuated virus as a vector allows for the immunization against the pathogen from which the foreign antigen is derived.

The most extensively developed and exploited eukaryotic virus-based vectors are the vaccinia virus-based vectors (Mackett et al, Proc. Natl. Acad. Sci. USA, 79:7415-7419 (1982); Panicali et al, Proc. Natl. Acad. Sci. USA, 79:4927-4931 (1982); and Tartaglia et al, Crit. Rev. Immun., 10:12-30 (1990)). Vaccinia virus-based vectors which have been described include those which contain inserts of foreign DNA derived from hepatitis B virus (Smith et al, Nature 302:490-492 (1983)); rabies (Brochier et al, J. Gen Virol., 70:1601-1604 (1989)); malaria (Smith et al, Science, 224:397-399 (1984)); influenza (Smith et al, Proc. Natl. Acad. Sci. USA, 80:7155-7159 (1983); Chambers et al, Virol 167:414-421 (1988); and Smith et al, Virol., 160:336-345 (1987)); herpes simplex virus (Paoletti et al, Proc. Natl. Acad. Sci. USA 81:193-197 (1984); Gillespie et al, J. Clin. Microbiol., 23:283-288 (1986); and McLaughlin-Taylor et al, J. Gen. Virol., 69:1731-1734 (1988)); murine cytomegalovirus (Jonicet al, J. Virol., 62:1653-1658 (1988)); Epstein Barr virus (Stewart et al, J. Gen. Virol., 70:1231-1237 (1989)); equine herpesvirus-1 (Guo et al, J. Virol., 63:4189-4198 (1989)); vesicular stomatitis virus (Mackett et al, Science 227:433-435 (1985)); human respiratory syncytial virus (King et al, J. Virol., 61:2885-2890 (1987)); rotavirus SA11 (Andrew et al, J. Virol., 61:1054-1060 (1987)); Rinderpest (Yilma et al, Sciences, 242:1058-1061 (1988)); Lassa fever virus (Morrison et al, Virol., 171:179-188 (1989)); human immunodeficiency virus envelope proteins (Chakrabarti et al, Nature 320:535-537 (1986)); and human T-cell leukemia virus (Shida et al, EMBO J., 6:3379-3384 (1987)). A vaccinia-based viral vector which expresses the coding gene sequences of the PRV g92 gene has been described (European Patent Publication No. 0162738). Racoon poxvirus recombinants expressing rabies virus glycoproteins (Esposito et al, Virol., 165:313-316 (1988)) and fowl pox virus recombinants (Taylor et al, Vaccine, 6:497-503 (1988)) are alternatives to vaccinia poxvirus recombinants. As already noted, two recombinant vaccinia viruses expressing the FMDV VP1 epitopes have been reported (Newton et al, In: Vaccine 87, Chanock et al eds., Cold Spring Harbor Laboratory, pp. 12-21 (1987)).

Viral-based vector vaccines offer several significant advantages over conventional vaccines. First, immunization against more than one disease is possible with a single virus strain. This creates economy of production. Secondly, for many vaccines, two or more different MLVs cannot be employed in combination in a single formulation because the replication of the viruses in the host causes mutual interference, and thereby impairs immunization. This situation is circumvented with the viral-based vector because there is only a single replicating genome. Third, vaccines against causative agents of infectious diseases, such as viruses and bacteria, for which vaccines have not previously been available or feasible, may for the first time be developed using viral-based vectors which express the antigens of the causative agents of infectious disease. This is because the only component of the causative agents of infectious disease which is carried by the viral-based vector is the gene coding for the antigen of the causative agent of infectious disease which is responsible for eliciting immunity. Other components of the causative agents of infectious disease that are responsible for the pathobiology of the disease are eliminated from the viral-based vector.

The vaccinia-based viral vectors are disadvantageous because: (1) they infect humans and therefore can pose a health hazard to the public; (2) there is no purpose served in vaccinating animals against smallpox, i.e., the disease that vaccinia protects against; (3) potential recombination events between vaccinia and indigenous animal pox viruses might regenerate virulence of the vaccinia virus which will cause smallpox-like disease in humans; and (4) vaccinia virus induces morbidity in cattle, the preferred species to be protected from FMD and by the herpesviruses of the present invention. Indeed, the traditional way to make smallpox vaccine is to induce vaccinia lesions in cattle.

Before recombinant vaccinia virus vaccines are widely used, ecological research in the following areas is badly needed: (1) the ecology of orthopox viruses; and (2) possible interactions between genetically engineered vaccinia viruses released into the environment and wild viruses which may be resident in both target and non-target species in a wide selection of habitats (Kaplan, Arch. Virol., 106:127-139 (1989)).

In the present invention, non-human, mammalian herpesviruses can be used as viral vectors. IBRV, PRV, BHV-2, BHV-6 and AHV-1 are typical herpesvirus with a genome consisting of linear double-stranded DNA molecules approximately 135-140 kilobases (hereinafter "kb") in size. Like vaccinia and the aforementioned other herpesviruses, IBRV can tolerate nucleotide sequence deletions of 4.0 kb or more, and can tolerate foreign DNA insertions of 5.0-10 kb or more. IBRV-based viral vectors and PRV-based viral vectors are advantageous over vaccinia-based viral vectors for vaccination of cattle and/or pigs because: (1) they protect cattle and/or pigs against disease; and (2) they are host limiting, i.e., they do not infect humans. Furthermore, as indicated above, natural infections of IBRV occur in swine, goats, water buffalo, and wildebeests, and natural infections of PRV occur in cattle, goats, and sheep, so that the potential exists for also using IBRV-based viral vectors and PRV-based viral vectors in these species. Similarly, BHV-4, AHV-1 and some attenutated strains of BHV-2 have potential as viral-based vectors with these animal species.

The vaccinia-based viral vectors developed in the art (Newton et al, In: Vaccines 87, Cold Spring Harbor Laboratory, Chanock et al, eds., pp. 12-21 (1987)) differ greatly from the viral vectors of the present invention in that: (1) they are not non-human, mammalian herpesvirus-based viral vectors and, thus, do not possess the advantageous characteristics thereof; (2) they require activation of the foreign gene insert, i.e., the FMD VP1 fusion proteins, by a vaccinia gene promoter rather than, e.g., an IBRV or PRV promoter, such as the IBRV gIII promoter or the PRV gIII promoter; (3) the vaccinia recombinants utilize human influenza hemagglutinin and human hepatitis B virus core protein genes for the construction of FMD VP1 fusion proteins, whereas a natural herpesvirus glycoprotein gene, e. g., the IBRV gIII gene or the PRV gIII gene, is used in the present invention in the construction of FMD VP1 fusion glycoproteins; (4) one vaccinia virus recombinant utilizes a human influenza virus hemagglutinin signal sequence to direct the transportation of the FMD VP-fusion protein to the cell membrane, and the second vaccinia recombinant generates an insoluble 27 nm particle that is not found in the cell membrane at all, whereas, in the present invention, the signal sequence, e. g., the bovine growth hormone signal sequence, is placed directly adjacent to the FMD VP1 epitope sequence and directs the FMDV VP1-fusion glycoprotein to the surface of infected cells; (5) the FMDV VP1 epitope does not become a part of the vaccinia virus particle in the vaccinia recombinants, whereas the FMDV VP1 epitope is incorporated together with the glycoprote in in the envelope of herpesvirus particles in the present invention; (6) the FMDV sequences are inserted downstream from the signal sequence and the ATG translational start signal in the human influenza hemagglutinin gene in the vaccinia recombinant, whereas the FMDV VP1 sequences are linked directly in phase to the signal sequence at the N-terminal of the glycoprotein gene in the present invention; (7) the foreign DNA is inserted in the tk gene of the vaccinia recombinants whereas, preferably, in a glycoprotein gene in the present invention; and (8) the FMDV VP1 amino acids 142 to 151 are inserted in the vaccinia recombinants, whereas, preferably, the superior FMDV epitope consisting of VP1 amino acids 141 to 158 linked to amino acids 200 to 213 are inserted into the herpesvirus-based viral vectors of the present invention. Hence, the presentation of the FMDV epitope to the immune system is quite different in the present invention from that of the vaccinia recombinants.

Prior to U.S. Patent 4,703,011 and U.S. Patent Application S.N. 07/116,197, filed November 3, 1987, and U.S. Patent Application S.N. 07/148,725, filed March 4, 1988, it was not possible to develop IBRV-based viral vectors because there was no knowledge of the location of suitable insertion sites, i.e., non-essential IBRV genes, which could be employed for the insertion of foreign genes. U.S. Patent 4,703,011 and U.S. Patent Application S.N. 07/116,197, filed November 3, 1987, however, identified the location of the IBRV tk and IBRV gIII genes on the physical map of IBRV DNA and demonstrated that the genes are not essential for virus replication in cultured cells. Hence, suitable insertion sites for foreign genes became known for the first time. Furthermore, U.S. Patent 4,703,011 and U.S. Patent Application S.N. 07/116,197, filed November 3, 1987, described for the first time the nucleotide sequences of the coding regions, the promoter regions, the translation start and stop signals, and the polyadenylation signals of the IBRV tk and IBRV gIII genes. In addition, U.S. Patent Application S.N. 07/148,725, filed March 4, 1988, demonstrated for the first time that the putative promoter sequences of the IBRV gIII gene could be linked to a foreign DNA sequence to drive the expression of the foreign DNA. This provided a functional demonstration that the IBRV gIII gene promoter had been accurately identified.

Moreover, prior to U.S. Patents 4,514,497 and 4,711,850 and U.S. Patent Application S.N. 06/875,703, filed April 29, 1986, it was not possible to develop PRV-based viral vectors because there was no knowledge of the location of suitable insertion sites, i.e., non-essential PRV genes, which could be employed for the insertion of foreign genes. U.S. Patents 4,514,497 and 4,711,850, however, describe the location of the PRV tk and PRV gIII genes on the physical map of PRV DNA and demonstrated that the genes are not essential for virus replication in cultured cells. Hence, suitable insertion sites for foreign genes became known for the first time. Furthermore, U.S. Patents 4,514,497 and 4,711,850 describe the nucleotide sequences of the coding regions, the promoter regions, the translation start and stop signals, and the polyadenylation signals of the PRV tk gene, as well as the restriction nuclease cleavage sites of a 2.4 Kbp NcoI to MluI DNA fragment which contains the PRV gIII gene. Moreover, U.S. Patent Application S.N. 06/857,703, filed April 29, 1986, demonstrated for the first time that the putative promoter sequences of the PRV gIII gene could be linked to a foreign DNA sequence to drive the expression of the foreign DNA. This provided a functional demonstration that the PRV gIII gene promoter had been accurately identified.

Recently, PRV-based viral vectors containing insertions of foreign DNA in non-essential glycoprotein genes have been described (Whealy et al, J. Virol., 62:4184-4194 (1988); Whealy et al, J. Virol., 63:4055-4059 (1989); and Thomsen et al, Gene, 57:261-265 (1987)).

More specifically, the PRV gIII gene has been used as the site of insertion of the foreign gene encoding the human immunodeficiency virus type 1 (hereinafter "HIV-1") envelope glycoprotein (Whealy et al, J. Virol., 62:4184-4194 (1988)). Three infectious PRV mutants expressing fused viral envelope proteins composed of PRV gIII and HIV-1 envelope glycoproteins gp120 and gp41 were produced. However, the level of HIV-1 gp120 and gp41 expression was very low, perhaps due to the instability or altered processing of the hybrid mRNA. In addition, two of the fused proteins were poorly detected on the cell surface and were not detected at all on the surface (envelope) of the virus particles. The third fused protein was not at all detected on the cell surface or on the surface (envelope) of the virus particles, but instead was secreted.

Furthermore, the PRV gIII gene has been used as the site of insertion of the foreign gene encoding the herpes simplex virus glycoprotein gC (Whealy et al, J. Virol., 63:4055-4059 (1989)). In this case, the PRV gIII gene and regulatory sequences were replaced by the entire herpes simplex virus gC gene and its regulatory sequences. The herpes simplex virus gC promoter was operable in the PRV genome and herpes simplex virus gC was produced in infected cells, albeit at a low level. However, the herpes simplex virus gC was only detected on the cell surface, i.e., such was not detected on the surface (envelope) of the virus particles.

Moreover, a cDNA encoding for human tissue plasminogen activator has been cloned downstream from the promoter of the PRV gX glycoprotein gene and flanked by downstream pRV DNA (Thomsen et al, Gene, 57:261-265 (1987)). Since PRV gX is normally secreted into the medium of infected tissue culture cells, only the medium of tissue culture cells infected by this PRV-based viral vector was found to contain human tissue plasminogen activator, i.e., human tissue plasminogen activator was not detected on the cell surface or on the surface (envelope) of the virus particles.

PCT Patent Publication No. 89/01040 also describes recombinant fusion proteins comprising an antigenic amino acid sequence fused to pRV gX. In particular, a recombinant PRV having the E. coli β-galactosidase gene inserted into the pRV gX gene which contains a deletion therein is disclosed. Also disclosed is a recombinant PRV having a portion of the Plasmodium falciparum cirumsporozoite gene (encoding amino acids 146-210 or amino acids 19-412) inserted between DNA encoding the PRV gX promoter and a portion of the PRV gX gene (encoding amino acids 1-269) and DNA encoding another portion of the PRV gX gene (amino acids 356-498, including the polyadenylation signal). With these recombinant PRV, the fusion protein was not detected on the surface on infected cells or on the surface of virus particles.

Prior to U.S. Patent 4,732,971, it was not known that FMDV VP1 amino acids 141 to 158 of the serotype O, or the equivalent sequence of other serotypes, could be linked together through amino acid bridges, such as proline-proline-serine, to amino acids 200 to 213 without a carrier, so as to obtain a markedly enhanced neutralization response in cattle against FMDV, to induce protection in cattle with a single vaccination, and to complete protection after repeat immunization. Therefore, it was not possible to incorporate this type of an FMDV VP1 epitope into a virus-based vector so as to obtain amplified expression of the FMDV VP1 epitope.

In a preferred embodiment of the present invention, the chemical synthesis of oligonucleotides encoding the bovine growth hormone signal sequence and the FMDV VP1 epitope sequences, the in phase linkage of the sequences, and the ligation of the IBRV gIII promoter to the coding nucleotide sequence of the bovine growth hormone-FMDV VP1 fusion have been described for the first time. Further, the insertion in phase of these sequences to the coding sequences of IBRV gIII gene modified so as to remove the IBRV gIII signal sequence, and thus avoid duplicate signal sequences, is described for the first time. Also, it has been determined for the first time in the present invention that in this IBRV-based viral vector, the FMDV VP1-IBRV gIII fusion protein is expressed from the IBRV gIII promoter on the surface of recombinant virus-infected cells and on the surface of the recombinant virus particles. Furthermore, it is shown for the first time in the present invention that the recombinant IBRV expressing the FMDV VP1 epitope elicits neutralizing antibodies to both IBRV and FMDV in cattle and pigs, protects the cattle from challenge by virulent IBRV, and protects cattle from challenge with virulent FMDV.

SUMMARY OF THE INVENTION

An object of the present invention is to provide a herpesvirus-based viral vector which expresses the major FMDV epitope.

Another object of the present invention is to provide a herpesvirus-based viral vector wherein the major FMDV epitope is expressed on the surface of infected cells and on the surface of the virus particles.

A further object of the present invention is to provide a herpesvirus-based viral vector wherein animals vaccinated with such develop antibodies to FMDV and are protected from FMDV disease.

A still further object of the present invention is to provide a herpesvirus vaccine effective in controlling the spread of herpesvirus disease in its various manifestations.

An additional object of the present invention is to provide a herpesvirus vaccine, wherein the vaccine can be safely and efficaciously administered intramuscularly, intranasally or intravaginally.

Another object of the present invention is to provide a herpesvirus vaccine, wherein the vaccine can be administered safely to calves or piglets and to pregnant cows or sows in all stages of pregnancy.

Still another object of the present invention is to provide a herpesvirus vaccine, wherein the animal vaccinated with such is less likely to become a carrier of either the herpesvirus vaccine strain or any herpesvirus field strain.

A further another object of the present invention is to provide a herpesvirus-based viral vector which is distinguishable from any herpesvirus field strain and from other herpesvirus vaccine strains.

A still further object of the present invention is to provide a herpesvirus-based viral vector which fails to produce any functional TK activity as a result of a deletion and/or insertion mutation in the tk gene.

Yet another object of the present invention is to provide a herpesvirus-based viral vector virus which cannot revert to tk$^+$, and is easily distinguished from a tk$^+$ virus.

Still another object of the present invention is to provide a herpesvirus-based viral vector which can replicate efficiently at temperatures ranging from 34.5°C to 40°C, i.e., inclusive of temperature-resistant viruses.

Other objects of the present invention will be apparent from the detailed description of the invention provided hereinafter.

In one embodiment of the present invention, the above described objects have been met by a herpesvirus-based viral vector which expresses an FMDV epitope on the surface of virus-infected cells and on the surface of virus particles.

In another embodiment, the above-described objects have been met by a vaccine against FMD comprising:

(A) a pharmaceutically effective amount of a herpesvirus-based viral vector which expresses an FMDV epitope on the surface of virus-infected cells and on the surface of virus particles; and

(B) a pharmaceutically effective carrier or diluent.

In still another embodiment, the above described objects have been met by a herpesvirus-based viral vector which expresses an FMDV epitope on the surface of virus-infected cells and on the surface of virus particles produced by a process comprising:

(1) Constructing a hybrid plasmid comprising a cloning vector and a herpesvirus glycoprotein gene sequence and flanking sequences of a non-essential herpesvirus genetic locus;

(2) Constructing a hybrid DNA sequence encoding a signal sequence-FMDV epitope sequence;

(3) Inserting the signal sequence-FMDV epitope sequence of Step (2) into the resulting hybrid plasmid of Step (1) so as to obtain a hybrid plasmid containing the signal sequence-FMDV epitope sequence as the N-terminal sequence of the herpesvirus glycoprotein gene;

(4) Deleting N-terminal herpesvirus glycoprotein gene sequences unidirectionally from the resulting hybrid plasmid of Step (3) so as to remove the non-essential herpesvirus glycoprotein signal sequence, and so as to obtain a hybrid plasmid comprising DNA encoding a fusion protein comprising the signal sequence-FMDV epitope sequence in phase with the herpesvirus glycoprotein sequence and said flanking sequences;

(5) Constructing a recombinant herpesvirus which contains a DNA sequence encoding a promoter adjacent to a selectable gene inserted in said non-essential herpesvirus genetic locus and which expresses the selectable gene from the promoter;

(6) Co-transfecting the DNA of the resulting recombinant herpesvirus of Step (5) and the resulting hybrid plasmid of Step (4) into herpesvirus host cells; and

(7) Selecting for a recombinant herpesvirus which does not express the selectable gene so as to obtain a herpesvirus-based viral vector which expresses an FMDV epitope on the surface of virus-infected cells and on the surface of virus particles.

In yet another embodiment of this invention, the hybrid DNA sequence of Step (2) encodes a signal sequence-FMDV epitope dimer so as to produce, in Step (7) a herpesvirus-based viral vector which expresses an FMDV epitope dimer on the surface of virus-infected cells and on the surface of virus particles.

## BRIEF DESCRIPTION OF THE DRAWINGS

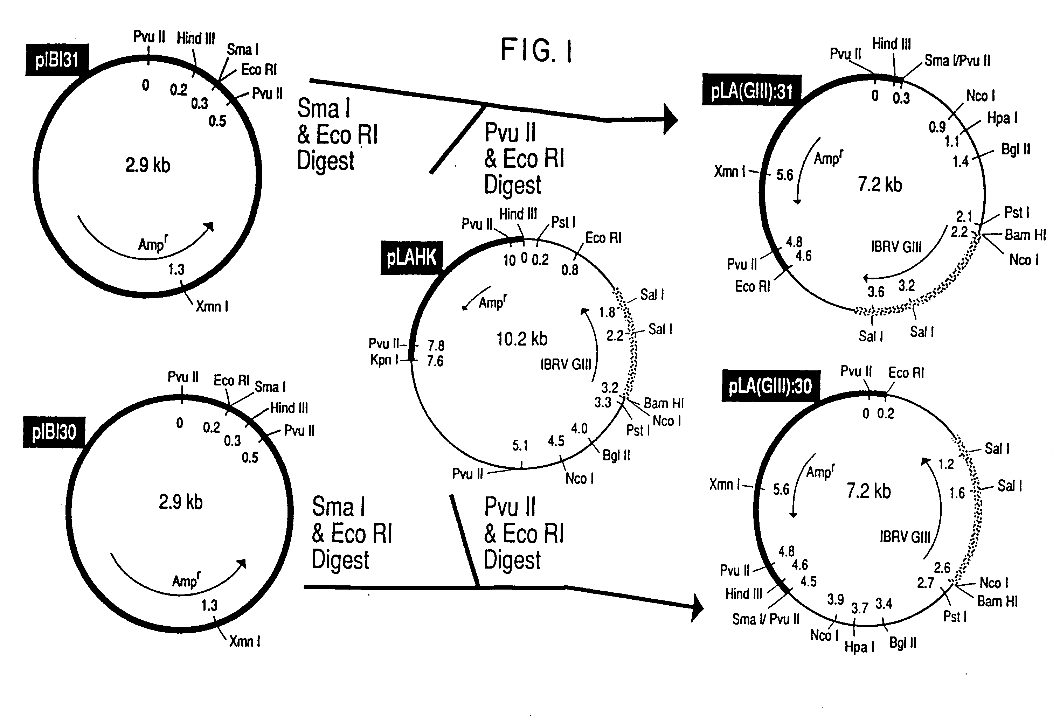

Figure 1 schematically illustrates, by example, the restriction endonuclease maps of the plasmids used to construct pLA(GIII):31 and pLA(GIII):30. These plasmids were prepared by subcloning the 4.3 kb EcoRI to PvuII fragments (map position 0.8 to 5.1 kb) from plasmid pLAHK into the EcoRI/SmaI sites of plasmids pIBI31 and pIBI30, respectively.

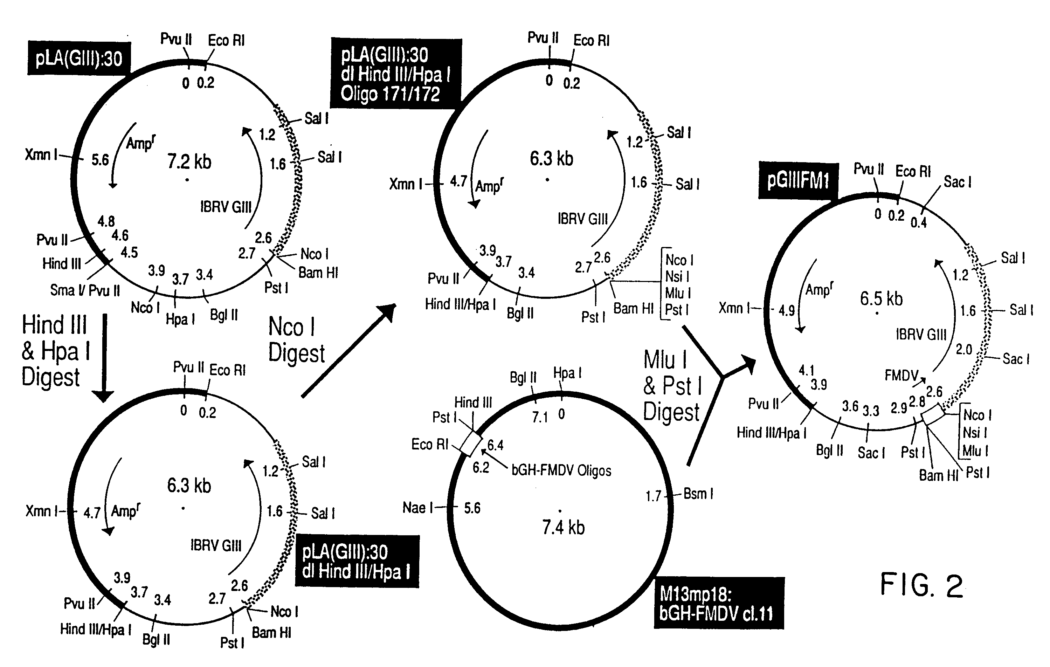

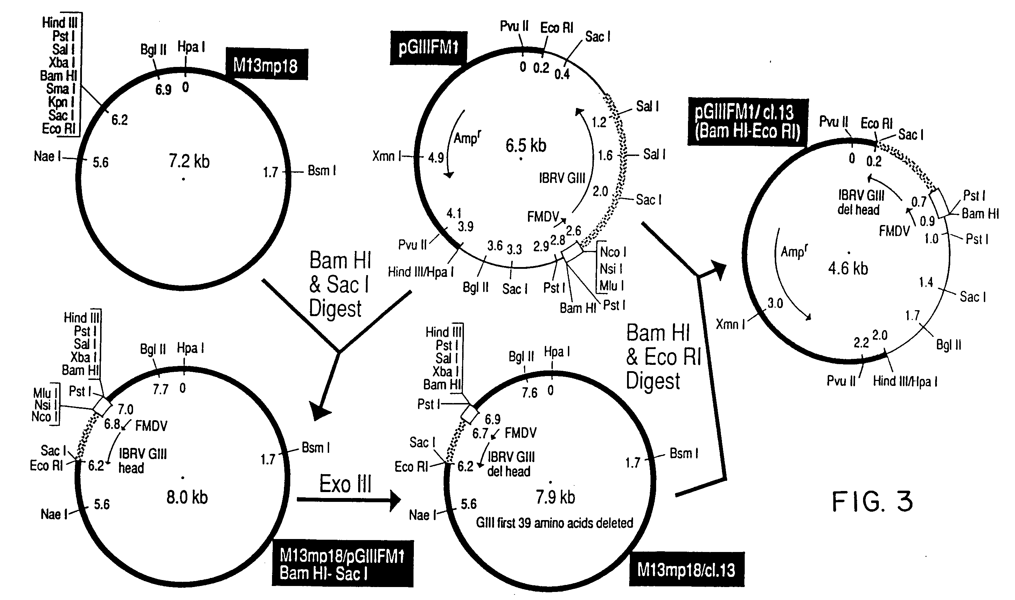

Figure 2 schematically illustrates, by example, the restriction endonuclease maps of the plasmids used to construct pGIIIFM1. This plasmid contains the bovine growth hormone (bGH) signal and FMDV epitope sequence inserted downstream from the IBRV gIII promoter and adjacent to the coding sequence of the IBRV gIII gene. Figure 2 shows that a HindIII/HpaI deletion mutant was prepared from plasmid pLA(GIII):30. Then, a 32-mer oligonucleotide cassette sequence, which contains NcoI cohesive ends and PstI, M1uI, and NsiI restriction sites, was inserted at the NcoI (translational start) site of the IBRV gIII gene to produce plasmid pLA(GIII):30 d1 HindIII/HpaI Oligo 171/172. The bGH-FMDV sequence which had been cloned in phage M13mp18 (Figure 3) was then transferred into the oligonucleotide cassette sequence of pLA(GIII):30 d1 HindIII/HpaI Oligo 171/172 to obtain pGIIIFM1.

Figure 3 schematically illustrates, by example, the restriction endonuclease maps of the plasmids used to obtain pGIIIFM1/cl.13 (BamHI-EcoRI). This plasmid was derived from pGIIIFM1 (Figure 2) by deleting the IBRV gIII sequences encoding the first amino acids of IBRV gIII. A small 793 bp BamHI to SacI fragment from pGIIIFM1 containing the bGH-FMDV sequence and the 5′ end of the coding sequence of the IBRV gIII gene was first transferred to the cloning cassette of phage M13mp18 to give M13mp18/pGIIIFM1 BamHI-SacI. The 793 bp IBRV sequence was then subjected to unidirectional exonuclease III digestion to remove sequences encoding the 39 N-terminal amino acids (including the signal sequence) from the IBRV gIII gene. The resulting deletion mutant was designated M13mp18/cl.13. The BamHI-EcoRI fragment of pGIIIFM1 was next replaced by the BamHI-EcoRI fragment of M13mp18/cl.13 to give pGIIIFM1/cl.13 (BamHI-EcoRI).

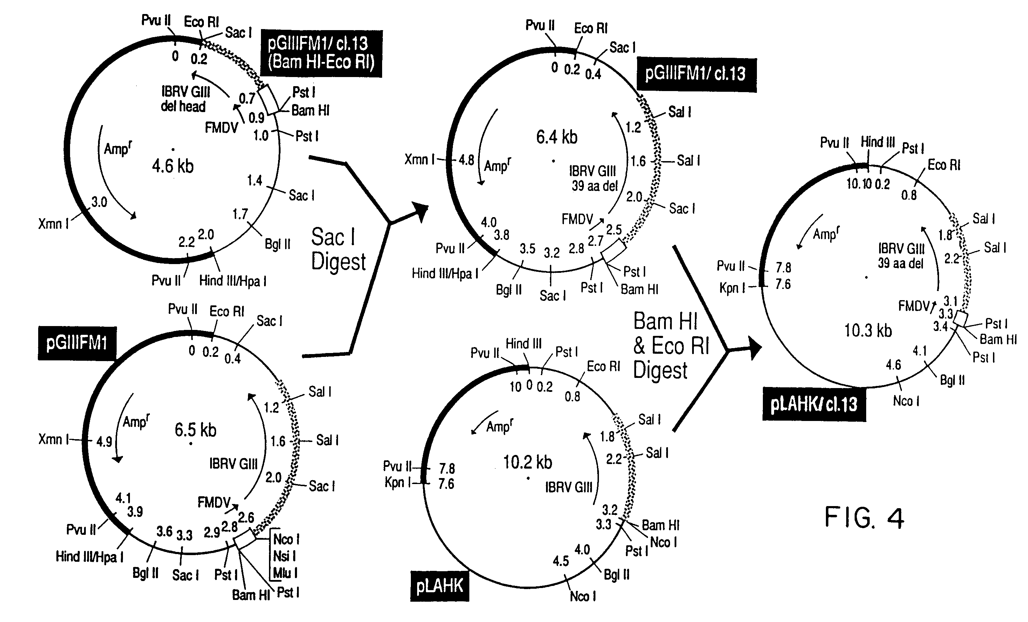

Figure 4 schematically illustrates, by example, the restriction endonuclease maps of the plasmids used in the derivation of pLAHK/cl.13 from pGIIIFM1/cl.13 (BamHI-EcoRI) and pGIIIFM1. Plasmid pLAHK/cl.13 has long (greater than 1 kb) DNA sequences from pLAHK that are 5' and 3' to the bGH-FMDV-gIII (del 39 amino acids) sequences. The bGH-FMDV sequences are in phase with the partially deleted IBRV gIII sequences so that a fusion protein of bGH-FMDV-(del)IBRV gIII can be expressed from the IBRV gIII promoter.

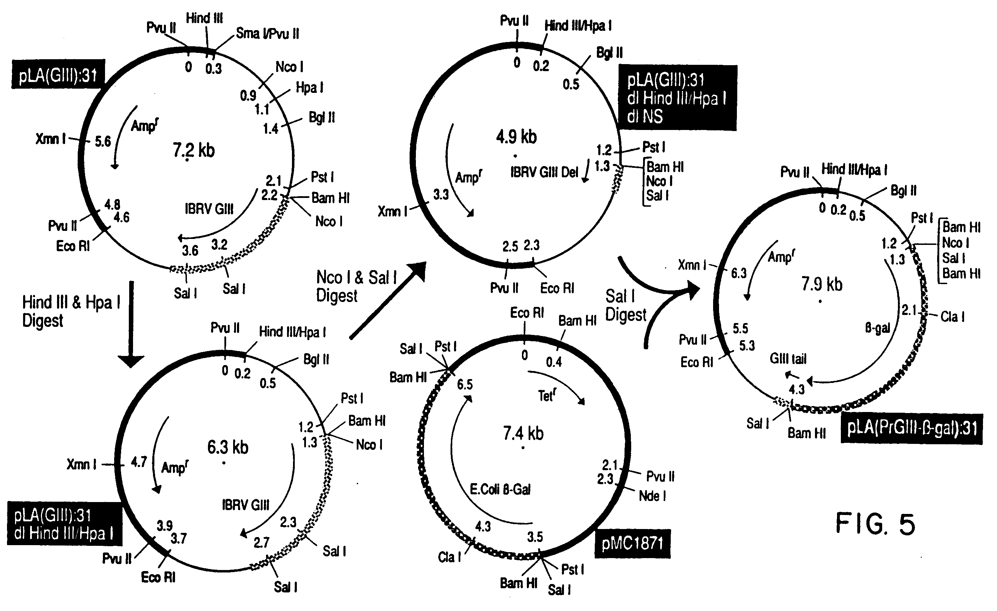

Figure 5 schematically illustrates, by example, the restriction endonuclease maps of plasmids used to construct pLA(PrGIII β-gal):31, which expresses the E. coli β-gal gene from an IBRV gIII promoter. The IBRV gIII gene was subcloned from pLA(GIII):31 (Figure 1) and HindIII to HpaI sequences were deleted to give pLA-(GIII):31 dl HindIII/HpaI (Figure 5). This plasmid was further modified by deleting NcoI to SalI sequences (1.3 to 2.7 map units). An adaptor made from Oligo 138 and Oligo 139 was then ligated at the deletion site to provide a cloning site for the insertion of the β-gal gene in phase with the gIII start codon (ATG) at the NcoI site of pLA-(GIII):31 dl HindIII/HpaI dl NS. The E. coli β-gal coding sequences from pMC1871 (map units 3.5 to 6.5) were then transferred to the SalI site of pLA(GIII):31 dl HindIII/HpaI dl NS to give pLA(PrGIII-β-gal):31.

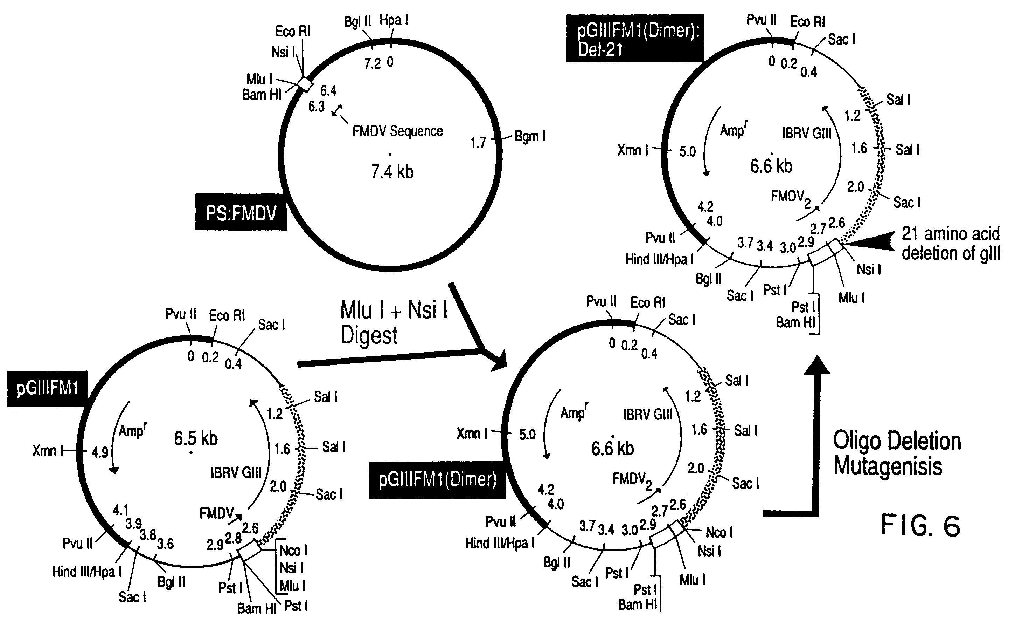

Figure 6 illustrates, by example, the restriction endonuclease maps of the plasmids used to construct plasmid pGIIIFM1(Dimer):Del 21. This plasmid contains the bGH signal sequence, and a dimer of the FMDV epitope sequence 3' to the IBRV promoter. Downstream from this sequence is the coding sequence of the IBRV gIII gene from which the N-terminal signal sequence has been removed- by oligo deletion mutagenesis. The bGH-FMDV-FMDV sequences are in phase with the IBRV gIII sequence and are expressed from the IBRV gIII promoter.

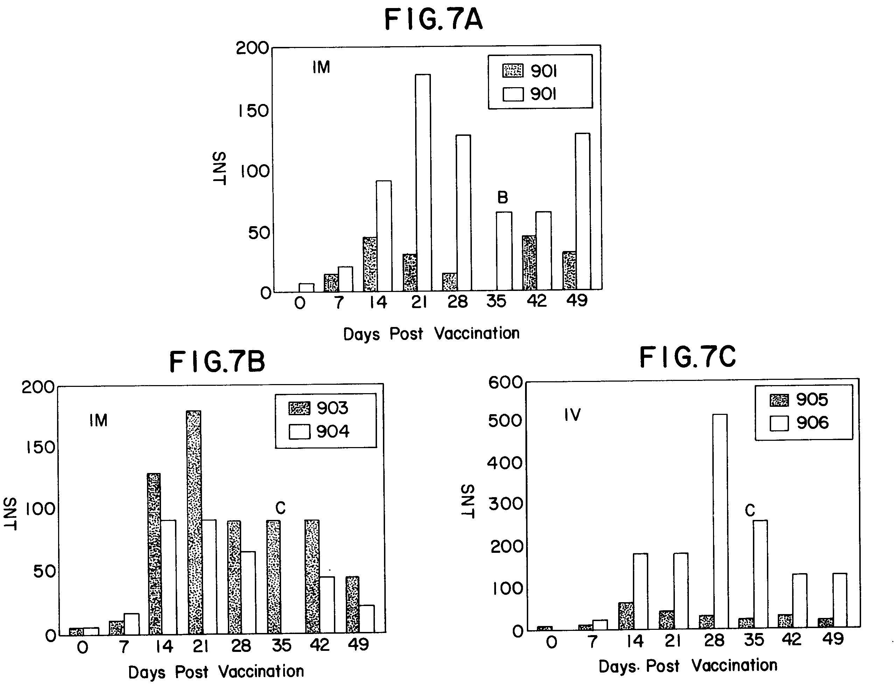

Figure 7 shows the anti-FMDV serum neutralization titers (SNT) obtained after vaccinating cattle with IBRV-FMDV cl.1A intramuscularly (IM) or intravenously (IV). Animals were boosted (B) with the recombinant virus or challenged (C) with virulent IBRV(Cooper) at 35 days post vaccination.

## DETAILED DESCRIPTION OF THE INVENTION

As discussed above, in one embodiment of the present invention, the above described objects have been met by a herpesvirus-based viral vector which expresses an FMDV epitope on the surface of virus-infected cells and on the surface of virus particles.

In another embodiment, the above-described objects have been met by a vaccine against FMD comprising:

(A) a pharmaceutically effective amount of a herpesvirus-based viral vector which expresses an FMDV epitope on the surface of virus-infected cells and on the surface of virus particles; and

(B) a pharmaceutically effective carrier or diluent.

In still another embodiment, the above described objects have been met by a herpesvirus-based viral vector which expresses an FMDV epitope on the surface of virus-infected cells and on the surface of virus particles produced by a process comprising:

(1) Constructing a hybrid plasmid comprising a cloning vector and a herpesvirus glycoprotein gene sequence and flanking sequences of a non-essential herpesvirus genetic locus;

(2) Constructing a hybrid DNA sequence encoding a signal sequence-FMDV epitope sequence;

(3) Inserting the signal sequence-FMDV epitope sequence of Step (2) into the resulting hybrid plasmid of Step (1) so as to obtain a hybrid plasmid containing the signal sequence-FMDV epitope sequence as the N-terminal sequence of the herpesvirus glycoprotein gene;

(4) Deleting N-terminal herpesvirus glycoprotein gene sequences unidirectionally from the resulting hybrid plasmid of Step (3) so as to remove the non-essential herpesvirus glycoprotein signal sequence, and so as to obtain a hybrid plasmid comprising DNA encoding a fusion protein comprising the signal sequence-FMDV epitope sequence in phase with the herpesvirus glycoprotein sequence and said flanking sequences;

(5) Constructing a recombinant herpesvirus which contains a DNA sequence encoding a promoter adjacent to a selectable gene inserted in said non-essential herpesvirus genetic locus and which expresses the selectable gene from the promoter;

(6) Co-transfecting the DNA of the resulting recombinant herpesvirus of Step (5) and the resulting hybrid plasmid of Step (4) into herpesvirus host cells; and

(7) Selecting for a recombinant herpesvirus which does not express the selectable gene so as to obtain a herpesvirus-based viral vector which expresses an FMDV epitope on the surface of virus-infected cells and on the surface of virus particles.

In yet another embodiment of this invention, the hybrid DNA sequence of Step (2) encodes a signal sequence-FMDV epitope dimer so as to produce, in Step (7) a herpesvirus-based viral vector which expresses an FMDV epitope dimer on the surface of virus-infected cells and on the surface of virus particles.

In the present invention, a herpesvirus-based viral vector has been developed which expresses the epitopes of FMDV on the surface of virus-infected cells and on the surface of virus particles. As a result, a vaccine containing the same is useful for protecting cattle and other cloven-hoofed animals against both herpesvirus diseases and foot and mouth disease.

The herpesvirus used to construct the herpesvirus-based viral vector is not critical to the present invention, although non-human, mammallian herpesviruses are preferred, particularly herpesviruses which infect animals that are susceptible to FMD. Examples of such herpesviruses include IBRV, useful for the insertion of an FMDV epitope to protect cattle against IBR and foot and mouth disease; BHV-4, useful for the insertion of an FMDV epitope to protect cattle against reproductive problems and foot and mouth disease; PRV, useful for the insertion of an FMDV epitope to protect swine against Aujetzky's disease and food and mouth disease; CapHV-1, useful for the insertion of an FMDV epitope to protect goats against caprine herpes and food and mouth disease; AHV-1, useful for the insertion of an FMDV epitope to protect exotic ruminant species, such as deer and wildebeests, against malignant catarrhal fever and foot and mouth disease; and BHV-2, useful for the insertion on an FMDV epitope to protect cattle against bovine mammillitis and foot and mouth disease.

The particular IBRV strain employed as a starting material in the present invention is not critical. Examples of such IBRV strains include the attenuated tk⁻ strains IBRV(B8-D53) (ATCC No. VR-2066) (U.S. Patent 4,569,840); IBRV(NG)dltk (ATCC No. VR-2112) (U.S. Patents 4,703,011 and 4,824,667); and tk⁺ strains Los Angeles (ATCC No. VR-188), Cooper (ATCC No. VR-864), IPV strain K22 (Kendrick et al, Cornell Vet., 48:458-495 (1958)), strains MO3, MO6, BFN-IH, BFN-IID, Gi 1 to 5, Bi, B4, BRV, LAE, V3 415, V3 416, V3 18 and V3 93 (Gergersen et al, Arch. Virol., 84:91-103 (1985)), BFA Wabu strain (Ackermann et al, Vet. Microbiol., 9:53-63 (1984)), strain P8-2 (Weinmaster et al, Virol 118:191-201 (1982)), strains P10 and P34 (Engels et al, Arch. Virol., 67:169-174 (1981)) or Alberta(Canada) isolates Nos. 1 to No. 122 (Misra et al, Arch. Virol., 76:341-354 (1983)).

The particular BHV-4 strain employed as a starting material in the present invention is not critical. Examples of such BHV-4 strains include strains DN5999 and MOVAR 33/66 (Mohanty, J. Am. Vet. Med. Assn., 163:855-857 (1973)); strains N123 and FHV-2 (Kit et al, Virus Res., 4:197-212 (1986)); strain V (Dubuisson et al, Vet. Microbiol., 21:97-114 (1989)); and strains LVR-140 and K10/82 (Augsburger et al, Arch. Virol. 104:309-321 (1989)).

The particular PRV strain employed as a starting material in the present invention is not critical. Examples of such PRV strains include the attenuated tk⁻strains PRV(BUK-5A) (ATCC No. VR-2078) (U.S. Patents 4,514,497 and 4,609,548); PRV(BUK)-dl 3 (ATCC No. VR-2074) (U.S. Patents 4,514,497 and 4,609,548); PRV(dlg92/dltk) (ATCC No. VR-2116) (U.S. Patents 4,711,850 and 4,753,884); the attenuated tk⁺ strains Bucharest, SUCH-1 (Skoda et al, Arch. Virol., 8:1-9 (1964)), Norden (Paul, et al, Arch. Virol., 73:193-198 (1982)); and virulent strains isolated directly from diseased animals or passaged frequently in the laboratory, such as the Aujeszky strain (ATCC No. VR-135), the P-2208 strain, the KC-152D strain (Maes et al, Am. J. Vet. Res., 44:2083-2086 (1983)), the S62/26 Iowa strain, the Ind-F strain, the Ind-S strain, the Ind-R strain and the Shope strain (Paul, et al, Arch. Virol., 73:193-198 (1982)).

The particular CapHV-1 strain employed as a starting material in the present invention is not critical. Examples of such CapHV-1 strains include strains E/CH and McK/US (Engels et al, J. Gen. Virol., 68:2019-2023 (1987)).

The particular AHV-1 strain employed as a starting material in the present invention is not critical. Examples of such AHV-1 strains include the attenuated WC1 strain and the virulent C500 strain (Bredgen et al, J. Gen Virol., 70:1141-1150 (1989)).

The particular BHV-2 strain employed as a starting material in the present invention is not critical. Examples of such BHV-2 strains include strain W-687 (O'Connor, Vet. Rec., 117:637 (1985)); strain NY-1 (Letchworth et al, Infection and Immunity, 43:1072-1079 (1984)); and strain 69/1LO (Lastrucci et al, Arch. Virol., 72:75-81 (1982)).

The particular FMDV strain from which the FMDV epitope is derived is not critical to the present invention. Examples of such strains include serotype $0_1$K (Piccone et al, J. Virol., 62:1469-1473 (1988); Dopazo et al, Proc. Natl. Acad. Sci. USA, 85:6811-6815 (1988); Gebauer et al, J. Virol., 62:2041-2049 (1988); Baxt et al, J. Virol., 63:2143-2151 (1989); and Pfaff et al, J. Virol., 62:2033-2040 (1988)); serotype A; serotype O; serotype C; serotype Asia-1; serotype South African Territories 1, 2, and 3 (SAT-1,2,3); and any one of the more than 65 subtypes (Grubman et al, Virol., 158:133-140 (1987). In a preferred embodiment, the FMDV VP1 epitope corresponds to that of FMDV strain $0_1$K.

In a more preferred embodiment of the present invention, specific codons for the sequence of amino acids, (200-213) - (proline-proline-serine) - (141-158), of the $0_1K$ epitope are employed. It is well known, however, that except for methionine (ATG) and tryptophan (TGG), there are 2 to 6 codons for each amino acid (e.g., the codons for leucine are CTA, CTC, CTG, CTT, TTA, and TTG). Thus, alternative codons to those used in the present invention could be used for the synthesis of the FMDV epitope sequence without departing from the spirit and scope of this invention.

In addition to the use of a single copy insert of the FMDV epitope, multiple copies of the same epitope or a copy of two or more different FMDV epitopes can be inserted as dimers, trimers, etc., into the herpesvirus glycoprotein gene.

The particular herpesvirus glycoprotein gene sequence employed in Step (1) is not critical to the present invention. Examples of such herpesvirus glycoprotein gene sequences include: the IBRV gIII gene (U.S. Patent Application S.N. 07/116,197, filed November 3, 1987), any of the 10 or more other IBRV glycosylated species have been identified (Van Drunen et al, J. Virol., 59:401-410 (1986); Fitzpatrick et al, Virol., 176:145-157 (1990)); the PRV gIII gene (U.S. Patents 4,711,850 and 4,753,884); the PRV gI gene; and the PRV gp63 gene (Thomsen et al, J. Virol., 61:229-232 (1987)).

As used herein, the expression "genetic locus" means a region of DNA encompassing one or more base pairs.

The non-essential herpesvirus genetic locus employed in which the DNA encoding the signal sequence-FMDV epitope-herpesvirus glycoprotein gene is inserted in Step (5) is not critical to the present invention. The non-essential herpesvirus genetic locus may be a non-essential herpesvirus coding sequence, a non-essential herpesvirus regulatory sequence or a non-essential non-coding intergenic sequence. Examples of such non-essential genetic loci include the IBRV tk gene (U.S. Patents 4,703,011 and 4,824,667), the IBRV gIII gene (U.S. Patent Application S.N. 07/116,197, filed November 3, 1987), within a single copy of the IBRV duplicated internal repeat sequences, or other non-essential IBRV genetic loci homolgous to non-essential genetic loci of PRV; the PRV tk gene (U.S. Patents 4,514,497 and 4,609,548), the PRV gIII gene (U.S. Patents 4,711,850 and 4,753,884), the PRV gI gene, the PRV gp63 gene (Mettenleiter et al, J. Virol., 61:4030-4032 (1987)), the PRV gX gene (Marchioli et al, Am. J. Vet. Res., 48:1577-1583 (1987)), within a single copy of the PRV duplicated internal repeats (PCT Patent Publication No. 87/04463); the tk gene or non-essential genes homologous to the afore-mentioned genes present in any of BHV-4, CapHV-1, AHV-1 or BHV-2.

The specific cloning vector employed to prepare the hybrid plasmid of Step (1), Step (2) or Step (4) is not critical to the present invention and will depend on the herpesvirus non-essential genetic locus employed as the target for insertion and the non-essential gylcoprotein gene employed as the target for fusion. Examples of such cloning vectors employed include pBR322 and pBR322-based vectors (Sekiguchi et al, Gene, 21:267-272 (1983)), pMB9, pBR325, pKH47 (Bethesda Research Laboratories), pBR328, pHC79, phage Charon 28 (Bethesda Research Laboratories, Boehringer Manneheim Biochemicals), pKB11, pKSV-10 (P-L Biochemicals) and, pMAR 420 (Otsuka et al, Virus, 113:196-213 (1981)), and oligo(dG)-tailed pBR322 (Bethesda Research Laboratories)). In the examples provided herein, pIBI30 was chosen as the cloning vector to subclone the IBRV gIII gene and flanking sequences from the HindIII-I fragment of IBRV so as to obtain pLAHK. This is because pIBI30 has unique HindIII and KpnI sites. Then, pIBI30 was again chosen as the cloning vector for further subcloning so as to obtain pLA(GIII)30. This is because pIBI30 has unique PvuII and EcoRI sites. The hybrid plasmid of Step (4) was constructed by recloning a modified IBRV gIII gene and flanking sequences back into pLAHK.

The specific host employed for growing the hybrid plasmids of the present invention is not critical to the present invention. Examples of such hosts include E. coli K12 RR1 (Bolivar et al, Gene, 2:95-113 (1977)), E. coli, K12 HB101 (ATCC No. 33694), E. coli MM21 (ATCC No. 336780), and E. coli DH1 (ATCC No. 33849). E. coli K12 RR1 is the preferred host and has an F⁻ hsd R hsd M genotype.

Similarly, alternative vector/cloning systems can be employed such as plasmid vectors which grow in E. coli or Saccharomyces cerevisiae, or both, or plasmid vectors which grow in B. subtillus, or even vectors such as bovine papilloma virus (ATCC No. 37112) which grow in animal cells such as mouse (ATCC No. RL-1616) (Elder et al, Ann. Rev. Gen., 15:295-340 (1981) and Ure et al, Methods in Enzymology, "Recombinant DNA", Vol. 101, Part C, Academic Press, NY (1983)).

As used herein, "flanking sequences" means the sequences upstream, downstream, or both upstream and downstream from the non-essential herpesvirus genetic locus. The upstream sequences can contain transcriptional control signals, i.e., promoters and enhancers of a non-essential herpesvirus gene. The downstream sequences can contain the polyadenylation signal for transcription of a non-essential herpesvirus gene.

In general, the size of the flanking sequences will be about 400 bp. In pLAHK/cl.13, described in detail below, the 3' and 5' sequences on both sides of the IBRV gIII gene were 1.6 kb and 4.3 kb in length.

The signal sequence employed in Step (2) is not critical to the present invention. Examples of such signal sequences include those derived from bGH (Woychik et al, Nucl. Acids Res., 10:7197-7210 (1982)), bovine immunoglobulin (GeneBank), IBRV gIII (U.S. Patent Application S.N. 07/116,197, filed November 3, 1987), BHV-1 gI (Whitbeck et al, J. Virol., 62:3319-3327 (1988)); PRV gIII (U.S. Patents 4,711,850 and 4,753,884); PRV gp50 (Petrovskis et al, J. Virol., 59:216-223 (1986)); PRV gp63 (Petrovskis et al, J. Virol., 60: 185-193 (1986)); PRV gI (Petrovskis et al, J. Virol., 60: 185-193 (1986)); and PRV gII (Whealy, J. Virol., 64: 1946-1955 (1990)).

In a preferred embodiment of the present invention, in Step (4), the nucleotide sequence (117 nucleotides) encoding the first 39 amino acids of the IBRV gIII have been deleted by exonuclease treatment and the remaining sequence of the IBRV gIII gene has been ligated to the bGH-FMDV sequence. The deletion of exactly 117 nucleotides of the gIII gene is not critical to the present invention. The deletion could be reduced to the 69 nucleotides encoding the IBRV gIII signal sequence or expanded in the 3' direction to about 450 nucleotides without departing from the spirit or scope of the present invention. There are 4 potential glycosylation sequences in the IBRV gIII polypeptide located at amino acids 93, 111, 173 and 297. Potential antigenic sites predicted by the method of Hopp and Woods are found between amino acid residues 103 and 146. With the aid of monoclonal antibodies, three gIII epitopes have been mapped between amino acid residues 22 and 150. One epitope was mapped between amino acid residues 140 and 240 and another epitope was mapped between amino acid residues 230 and 287 (Fitzpatrick et al, Virol., 16:145-157 (1990)). Thus, deletion of less than 69 to 450 nucleotides from the coding sequence of the IBRV gIII gene can be made to remove the IBRV gIII signal sequence and one or more IBRV gIII epitopes, while leaving intact other IBRV gIII epitopes.

The specific herpesvirus promoter employed in Step (5) is not critical to the present invention. Examples of such promoters include the following herpesvirus promoters: the promoter for an "immediate early" IBRV gene, or the IBRV tk "early" gene promoter which is located in the 400 bp PvuII to NcoI fragment upstream from the translation start signal of the IBRV tk gene (Figure 4, pLATKdlNdeI, map units of 1.4 to 1.8 of U.S. Patent 4,703,011), or the promoters of IBRV late genes, such as IBRV gIII gene promoter which is located in the 650 bp gIII to NcoI fragment upstream from the ATG translational start signal of the IBRV gIII gene (Figure 5 of U.S. Patent 4,703,011).

In addition to the above-recited IBRV gene promoters, other herpesvirus promoters could be used in Step (5) such as the IBRV gI gene promoter (Misra et al, Virol 166:542-549 (1988)); the BHV-2 tk gene promoter (Sheppard et al, J. Gen. Virol., 70:3067-3071 (1979)); or the BHV-2 gB gene promoter (Hammerschmidt et al, Virol., 165:388-405 (1988)); the PRV tk gene promoter (U.S Patent 4,514,497), the PRV gX gene promoter (Rea et al, J. Virol., 54:21-29 (1985)), the PRV gp50 gene promoter (Petrovskis et al, J. Virol., 59:216-223 (1986); and Petrovskis et al, J. Virol., 60:185-193 (1986), the PRV gII gene promoter (Robbins et al, J. Virol., 61:2691-2701 (1987)) or the PRV gIII gene promoter (U.S. Patent 4,711,850).

The recombinant herpesvirus of Step (5) can be constructed as described herein, for example, in U.S. Patent Application S.N. 07/148,725, filed March 4, 1988, and U.S. Patent Application S.N. 06/857,703, filed April 29, 1986.

As used herein, a "selectable gene" means a DNA sequence which encodes a gene product whose presence or absence can be easily detected. The selectable gene employed in Step (5) is not critical to the present invention. Examples of such selectable genes can include a tk gene, the transposon Tn5 gene (neo$^R$) and the E. coli lacZ gene.

The specific tk gene employed as a selectable gene is not critical to the present invention. That is, the tk gene can be derived from any of the tk$^+$ PRV strains discussed above or from any other viruses which contain a viral specific tk gene such as HSV-1, HSV-2 and marmoset herpesvirus (Kit et al, Biochem. Biophys. Acta, 741:158-170 (1983) and Wagner et al, Proc. Natl. Acad. Sci. USA, 78:1441-1445 (1981)). Alternatively, the tk gene can be of cellular origin such as the chicken and human tk genes (Merill et al, Mol. Cell. Biol., 4:2316-2320 (1984)).

The specific selection means for selecting for the presence or absence of a functional TK, i.e., tk$^+$ or tk$^-$ viruses is not critical. Examples of the selection means for a tk$^+$ virus include growth medium supplemented with $10^{-4}$ M hypoxanthine, $10^{-6}$ M aminopterin, $4 \times 10^{-5}$ M thymidine and $10^{-5}$ M glycine (hereinafter "HATG") (Dubbs et al, Virol 126:408-411 (1983)) and growth medium supplemented with $6 \times 10^{-7}$ M methotrexate, $1.6 \times 10^{-5}$ thymidine, $5 \times 10^{-5}$ M adenosine, $5 \times 10^{-5}$ M guanosine and $10^{-4}$ M glycine (hereinafter "MTAGG") (Munyon et al, J. Virol., 7:813-820 (1971)). Examples of the selection means for a tk$^-$ virus include growth medium containing 25 μg/ml 5-bromodeoxyuridine (hereinafter "BrdUrd"), growth medium containing 100 μg/ml 5-iododeoxyuridine (hereinafter "IdUrd"), or growth medium containing 100 μg/ml arabinosylthymine. Many modifications of the nucleoside analog selection techniques for tk$^-$ viruses can also be used. For example, the virus infected cells can be grown in media with about 2.5 to 25 μg/ml BrdUrd. Since the BrdUrd is incorporated into DNA of tk$^+$ viruses and BrdUrd-containing DNA is highly photosensitive, the virus harvests can be treated with about 0.5 μM Hoeschst 33258 to further photosensitive the DNA. The DNA is then exposed to "cool white" fluorescent light (General Electric, 15 w) for 4 min to deliver about 50 erg/s mm² /sec (Hillary et al, Biochem. Genet., 22:201-213 (1984)). Following this procedure, the infectivity of tk⁺ viruses is selectively destroyed, while tk⁻ viruses are resistant to this treatment.

As discussed above, selectable genes other than the tk gene can be used in Step (5). For example, the transposon Tn5 gene (neoᴿ) which encodes an aminoglycoside 3′-phosphotransferase capable of conferring resistance to neomycin and to the drug G418 (pNEO; Pharmacia P-L Biochemicals). To obtain expression of the transposon Tn5 gene, it is necessary that a herpesvirus promoter be positioned 5′ to the coding region of the transposon Tn5 gene (Franke et al, Mol. cell. Biol., 5:1918-1924 (1985)).

Alternatively, the E. coli lacZ gene can be used instead of the tk gene in Step (5) such that the E. coli lacZ gene is fused to a herpesvirus promoter. This hybrid plasmid can then be used to select for recombinant herpesvirus plaques expression β-galactosidase, which is detected by the blue color produced with O-nitrophenyl-β-D-galactopyranoside and X-gal (Boehringer-Mannheim) (Chakrabarti et al, Mol.Cell. Biol., 5:3403-3409 (1985)).

In the context of this invention, a temperature-resistant virus is a virus which is non-temperature sensitive. Thus, a temperature-resistant virus is capable of replicating, at a non-permissive temperature, i.e., at about 38.5°C to 40°C, preferably about 39.1°C, about as well as the parental virus or field isolates of herpesvirus replicate at a permissive temperature. By contrast, temperature-sensitive herpesvirus strains contain mutations in viral genes essential for replication, whereby functional gene products are produced at permissive temperatures, i.e., about 32°C to 37.5°C, preferably 34.5°C, but not at non-permissive temperatures. Therefore, in temperature-sensitive viruses, production of infectious virus particles is 4 to 7 logs lower at the non-permissive temperatures. With temperature-resistant virus strains, production of infectious virus particles is about the same at non-permissive temperatures as at permissive temperatures.

Temperature-resistant viruses are superior to temperature-sensitive viruses as modified live virus vaccines because (1) attenuation results from alterations in pathogenic viral genes rather than from crippling viral genes required for replication; and (2) temperature-resistant viruses can be safely administered intramuscularly, intranasally, or intravenously and can replicate in the deep tissues of the body so as to elicit a more complete and prolonged immunological response.

In contrast, temperature-sensitive viruses only replicate at low temperature sites, such as the upper respiratory tract, and thus can only be administered intranasally.

The particular herpesvirus host cell employed in Step (6) is not critical to the present invention and will depend of the herpesvirus employed. For example, for IBRV, host cells include Rab-9 (ATCC No. CRL-1414), primary rabbit kidney calls, secondary rabbit kidney cells, rabbit cornea (SIRC) cells (ATCC No. CCL-60), rabbit kidney (LLC-RKI) cells (ATCC No. CCL-106), embryo bovine trachea (EBTR) cells (ATCC No. CCL-44), bovine turbinate (BT) cells (ATCC No. CRL-1390), and bovine kidney (MDBK) cells (ATCC No. CCL-22) (The American Type Culture Collection Catalog indicates that some types of lamb, goat, cat and horse cells may be permissive for IBRV (Los Angeles) (ATCC No. VR-188)). MDBK and Rab-9 are the preferred IBRV host cells employed in the present invention. However, it should be noted that for the production of virus used for vaccination of animals in the field, a U.S. Department of Agriculture certified cell line permissive for IBRV, preferably of the same species as the animal to be vaccinated, and free of other infectious agents, should be used. For example, a suitable bovine cell line would be a certified diploid nontumorigenic bovine turbinate or kidney cell line free of mycoplasma and other viruses, such as MDBK cells.

For BHV-4, examples of suitable host cells include bovine kidney cells and Rab-9 cells (Kit et al, Virus Res., 4:197-212 (1986)).

For PRV, examples of suitable PRV host cells include porcine kidney (PK 15), porcine testicular (ST) cells, Rab-9, primary rabbit kidney cells, secondary rabbit kidney cells, monkey cells, i.e., CV-1 and OMK, human cells, e.g., and human embryonic kidney cells, and chick embryo fibroblast cells. Rab-9 are the preferred PRV host cells employed in the present invention. However, it should be noted that for the production of virus to be used for vaccination of animals in the field, a U.S. Department of Agriculture certified cell line permissive for PRV, preferably of the same species as the animal to be vaccinated, and free of other infectious agents, should be used. For example, a suitable porcine cell line would be a certified diploid nontumorigenic porcine testicle (ST) cell line free of mycoplasma and other viruses.

For CapHV-1, examples of suitable host cells include bovine kidney cells and bovine fetal lung cells (Engels et al, J. Gen. Virol., 68:2019-2023 (1987)).

For AHV-1, an example of suitable host cells is bovine kidney cells (Bridgen et al, J. Gen. Virol., 43:1141-1150 (1989)).

For BHV-2, examples of suitable host cells include bovine kidney cells and bovine testicular cells (O'Connor, Vet. Rec., 117:637 (1985)); and (Letchworth et al, Infection and Immunity, 43:1072-1079 (1984)).

The useful dosage of the herpesvirus-based viral vectors of the present invention to be administered as a live virus vaccine will vary depending on the age, weight and species of the animal vaccinated, the herpesvirus employed, and the mode of administration employed.

A suitable dose can be, for example, about $10^{5.0}$ to $10^8$ p.f.u./animal, preferably about $10^{7.5}$ p.f.u./animal, in a pharmaceutically acceptable carrier or diluent.

The particular pharmaceutically acceptable carrier or diluent is not critical to the present invention. Examples of pharmaceutically acceptable carriers and diluents include any physiological buffered media, i.e., about pH 7.0-7.4, containing from 2.5 to 15% (v/v) serum which do not contain antibodies to the herpesvirus employed or to FMDV. Agammaglobulin sera is preferred to sera that contains gammaglobulin. Examples of serum to be employed in the present invention include calf serum, fetal calf serum, and lamb serum. Serum protein, such as bovine serum albumin, in an amount of from about 0.5 to 3.0% (w/v) can be employed as a substitute for serum. However, it is desirable to avoid the use of foreign proteins in the carrier or diluent which may induce allergic responses in vaccinated animals.

Alternatively, the herpesvirus-based viral vectors of the present invention can be employed as inactivated virus vaccines against herpesvirus and foot and mouth disease. That is, inactivation of viral infectivity by ultraviolet light, formaldehyde, or binary ethylene imine treatment yields vaccines capable of eliciting protective antibodies and cytotoxic T cells against virus proteins. Adjuvants may be used with the recombinant viruses of the present invention to enhance the immunological responses to the herpesvirus and FMDV antigens.

Either the live recombinant viruses or the inactivated recombinant viruses of the present invention can be mixed with the adjuvants prior to immunization of animals. For example, cattle or pigs may be inoculated intramuscularly behind the ears with 4.0 ml of the recombinant virus emulsified with adjuvant "A" (or other suitable adjuvants) to give 2.0 ml of recombinant virus per 4.0 ml dose. To prepare 60 ml of the recombinant virus-adjuvant "A" emulsion, 30 ml of the recombinant virus is emulsified with 26.7 ml of Drakeol 6-VR (Pennsylvania Oil, Co.), 3.0 ml of Seppic 888 (Seppic Division, Cosmétique Pharmacie), and 0.3 ml of Emulgin 05 (Henkel Corp.). Animals immunized with the recombinant virus-adjuvant emulsion would thus be protected against virulent virus infection.

The recombinant viruses of the present invention can be administered intramuscularly, subcutaneously, intravenously or intranasally. Intramuscularly is the preferred mode of administration.

It is preferred that the recombinant viruses of the present invention be stored at a titer of at least $10^6$ to $10^7$ p.f.u./ml at -70° to 90°C or in a lyophilized state at 4° to -20°C. The lyophilized virus may be reconstituted for use with sterile distilled water or using an aqueous diluent containing preservative, such as gentamicin and amphotericin B or penicillin and streptomycin.

The following examples are provided for illustrative purposes only and are in no way intended to limit the scope of the present invention.

In the following examples, all media and buffer solutions were made up in glass distilled water unless otherwise indicated.

EXAMPLE 1

Construction of IBRV-FMDV

I. Construction of a Plasmid Containing the IBRV gIII Gene with a Cloning Cassette for Insertion of the bGH-FMDV Epitote DNA Sequence

A. Cloning of a Plasmid Containing the IBRV gIII Gene: Derivation of pLAHK

Plasmid pLAHK (see Figure 1) is comprised of a 7.6 kb KpnI/HindIII fragment of IBRV(LA) DNA inserted in plasmid pUC18 dl EcoRI. The IBRV DNA contains the IBRV gIII gene and flanking sequences thereof (U.S. Patent Application S.N. 07/116,197, November 3, 1987).

B. Subcloning of the IBRV gIII Gene: Derivation of pLA(GIII):30

The 4.3 kb EcoRI to PvuII fragment (map position 0.8 to 5.1 kb) containing the IBRV gIII gene (see Figure 1) was excised from pLAHK as follows.

5.0 μg of pLAHK was added to 250 μl of a buffer comprising 60 mM NaCl, 10 mM Tris-HCl (pH 7.5), 10 mM MgCl$_2$, 6.0 mM 2-mercaptoethanol, and 100 μg/ml of BSA (hereinafter "PvuII cutting buffer"). The DNA was digested for 2 hr at 37°C with 12 units of PvuII enzyme (New England Biolabs). The reaction was terminated by adding cyclohexanediamine tetraacetate (hereinafter "CDTA") to 20 mM and heating at 65°C for 30 min. Ammonium acetate was added to 2.5 M and the DNA precipitated with 2 volumes of ethanol. The DNA preci-

18

pitate was collected by centrifugation, then redissolved in 250 µl of a buffer comprising 50 mM NaCl, 100 mM Tris-HCl (pH 7.5), 5.0 mM $MgCl_2$ and 100 µg/ml of BSA (hereinafter "EcoRI cutting buffer"). The PvuII-digested pLAHK was digested for 2 hr at 37°C with 20 units of EcoRI enzyme (New England Biolabs). The reaction was terminated by adding CDTA to 20 mM and heating at 65°C for 30 min. The PvuII- and EcoRI-digested DNA fragments of pLAHK were purified by agarose gel electrophoresis on 0.5% (w/v) agarose slab gels in electrophoresis buffer comprising 30 mM $NaH_2PO_4$, 1.0 mM EDTA, and 40 mM Tris-Base (pH 8.1) (hereinafter "electrophoresis buffer") at 35 volts at 4°C for about 16 hr. After electrophoresis, DNA fragments were stained by soaking the gel in electrophoresis buffer comprising 0.5 µg/ml of ethidium bromide, visualized over a long-wave UV illuminator, and photographed. Then, the glassmilk method was used to recover the desired DNA fragment (Vogelstein et al, Proc. Natl. Acad. Sci. USA., 76:615 (1979)).

More specifically, the 4.3 kb size fragment was excised from the gel, and extracted with 3 volumes of NaI reagent (Geneclean™, Bio 101 Inc., La Jolla, CA), at 50°C for 3 min. The DNA was absorbed to "glassmilk" (Geneclean™) for 5 min at room temperature and separated from the agarose, etc., by a brief centrifugation in a microcentrifuge. The "glassmilk" was washed 3 times with NEW wash (Geneclean™). The DNA was desorbed from the "glassmilk" by adding 10 µl of a buffer comprising 10 mM Tris (pH 7.5), and 1.0 mM EDTA (hereinafter "1 X TE buffer") and heating to 55°C for 3 min. The DNA in solution was removed from the residual "glassmilk" by centrifugation and transferring to a new tube.

A cloning vector for insertion of the PvuII to EcoRI fragment was obtained as follows.

0.5 µg of pIBI 30 (International Biotechnologies, Inc.) was added to a buffer comprising 20 mM KCl, 6. 0 mM Tris-HCl (pH 8. 0), 6. 0 mM $MgCl_2$, 6. 0 mM 2-mercaptoethanol, and 100 µg/ml of BSA (hereinafter "SmaI cutting buffer"). The DNA was digested for 2 hr at 25°C with 5 units of SmaI enzyme (New England Biolabs, Inc.). The reaction was terminated by adding CDTA to 20 mM and heating at 65°C for 30 min. Ammonium acetate was added to 2.5 M and the DNA precipitated with 2 volumes of ethanol. The DNA precipitate was collected by centrifugation, then redissolved in 50 µl of EcoRI cutting buffer and digested with 10 units of EcoRI enzyme for 2 hr at 37°C. The reaction was terminated and the DNA precipitated, then collected as described above.

The 4.3 kb PvuII to EcoRI fragment of pLAHK and the 2.9 kb SmaI- and EcoRI-digested cloning vector pIBI30 were combined in a buffer comprising 50 mM Tris-HCl (pH 7.8), 10 mM $MgCl_2$, 20 mM dithiothreitol, 1.0 mM ATP, and 50 µg/ml of BSA (hereinafter "ligation buffer") and ligated by adding 1,000 units of T4 DNA ligase and incubating at 4°C overnight. The reaction was terminated by adding EDTA to 20 mM and heating the mixture for 10 min at 65°C. The ligation mixture was used to transform competent E. coli K12 strain NM522 (International Biotechnologies, Inc.) obtained as follows.

A starter culture of NM522 was prepared by inoculation 5.0 ml of 2YT media (16 g of bacto-trytone (Difco Laboratories, Inc.), 10 g of yeast extract (Difco Laboratories, Inc.), and 10 g of NaCl in 1.0 liter of $H_2O$) with a fresh colony of NM522 picked from M9 minimal media agar plates, and incubating the culture overnight at 37°C. 100 ml of 2YT media was inoculated with 1.0 ml of a starter culture of E. coli K12 strain NM522. The culture was incubated for 1 1/4 hr, at which time the $OD_{600} = 0.3$, then transferred to a centrifuge bottle and sedimented by centrifuging for 10 min at 6,000 x g at 4°C. The supernatant was decanted and the pellet gently resuspended in 50 ml of ice cold 10 mM $CaCl_2$ for 30 min on ice. The bacteria were pelleted by centrifuging for 20 min at 5,000 x g at 4°C. The supernatant was decanted, and the bacteria were resuspended in 10 ml of ice cold 10 mM $CaCl_2$ to obtain competent cells.

The competent bacteria were transformed with the ligation mixture as follows.

1.0 µl of the ligation mixture was added to 0.1 ml of $CaCl_2$ -treated E. coli K12 strain NM522 in glass vials, and stored on ice for 30 min. After a 5-min heat shock at 37°C, the mixture was kept for 5 min at room temperature. Then, 1.0 ml of LB broth (10 g of Bacto-tryptone, 5.0 g of yeast extract, 10 g of NaCl in 1.0 liter of $H_2O$) was added, and the mixture incubated at 37°C for 1 hr. 0.1 ml of aliquots of the bacteria plus DNA were spread onto LB agar plates containing 0.1 mg/ml of ampicillin in 10 cm diameter Petri dishes. The spread plates were incubated overnight at 37°C. Colonies were picked with toothpicks and transferred to tubes with 5.0 ml of 2YT media containing 0.1 mg/ml of ampicillin, and were incubated overnight at 37°C.

The candidate recombinant clones were screened for the desired hybrid plasmid DNA (hereinafter "rapid screening procedure") as follows.

An overnight culture of bacteria containing hybrid plasmid DNA was inoculated into 5.0 ml of 2YT media containing 0.1 mg/ml of ampicillin and incubated at 37°C to a density of about 1.5 ($A_{600}$). 1.0 ml of this bacterial culture was then transferred to a 1.5 ml microcentrifuge polypropylene tube and centrifuged in an Eppendorf microcentrifuge for about 1 min at room temperature to pellet the bacteria. Next, the bacteria were resuspended in 0.1 ml of a lysozyme solution comprising 2.0 mg/ml of egg lysozyme; 50 mM glucose; 10 mM CDTA; and 25 mM Tris-HCl buffer (pH 8.0) (hereinafter "lysozyme solution No. 1"), and then incubated for 30 min at 4°C. Next, 0.2 ml of 0.2 N NaOH plus 1.0% (w/v) sodium dodecylsulfate was added to the bacterial suspension and the tube was vortexed and kept at 4°C for 5 min. Thereafter, 0.15 ml of 3.0 M sodium acetate (pH 4.8) was added, and the tube was gently inverted, during which time a "clot" of DNA formed. The DNA was kept at 4°C for 1 hr to allow chromosomal DNA, protein, and high molecular weight RNA to precipitate. Next, the precipitate was centrifuged in an Eppendorf centrifuge for 5 min at room temperature and the clear supernatant fluid, approximately 0.4 ml, containing recombinant plasmid DNA, was transferred to a second microcentrifuge tube. Then, 2.5 volumes of ethanol (approximately 1.0 ml) were added to the second tube which was placed at -20°C for 30 min. The precipitated hybrid plasmid DNA was collected by centrifugation for 2 min at room temperature in an Eppendorf centrifuge. Then, the hybrid plasmid DNA was dissolved in 0.1 ml of 0.1 M sodium acetate, 0.05 M Tris-HCl (pH 8.0), reprecipitated with ethanol, collected by again centrifuging, and finally dissolved in 100 µl of 0.1 X TE buffer. A recombinant plasmid was obtained containing the 4.3 kb EcoRI-PvuII fragment from pLAHK inserted into the SmaI-EcoRI site of plasmid vector pIBI30. This recombinant plasmid was designated pLA-(GIII):30 (see Figure 1).

For large-scale preparation of hybrid plasmid DNA, 200 times the amount of plasmid-transformed bacteria were processed as compared with the bacteria used to produce hybrid plasmid DNA for the rapid screening procedure described above, except that after the first ethanol precipitation, the sample was treated at 37°C for 30 min, with 0.5 mg of pancreatic RNase A (Worthington Biochemical Corp.) from a stock solution comprising 1.0 mg/ml of RNase A in 5.0 mM Tris-HCl (pH 8.0) which had been heated at 100°C for 10 min. The treatment was followed by the addition of 500 µg of Proteinase K (E.M. Science) in TE buffer at 37°C for 30 min. Subsequently, an equal volume of phenol was added, and the sample was vortexed and centrifuged as described above to separate the phases. The aqueous phase was then removed, precipitated with ethanol, and collected by centrifugation as described above. The precipitate was then dissolved in 4. 4 ml of 1 X TE buffer with 4.4 g of CsCl and 0.2 ml of ethidium bromide (8.6 mg/ml stock). The sample was overlaid with paraffin oil and centrifuged at 44,000 rpm at 22°C for 44 hr in a Beckman 50 Ti rotor.

The lowermost band of superhelical plasmid DNA was collected by puncturing the side of the tube with a 20-gauge needle. The ethidium bromide was extracted from the plasmid DNA with n-butanol saturated with an aqueous solution comprising 5.0 M NaCl, 10 mM Tris (pH 8.5), and 1.0 mM EDTA followed by exhaustive dialysis against 0.1 X TE buffer.

## C. Removal of DNA Sequence from pLA(GIII):30 Containing Extra NcoI Site: Derivation of pLA(GIII):30 dl HindIII/HpaI

Mapping of pLA(GIII):30 indicated the presence of two NcoI restriction sites. The NcoI site at 3.9 map units was removed by deleting the HindIII to HpaI fragment (4.6 to 3.7 map units) in order to obtain a plasmid with a unique cloning site for insertion of the bGH-FMDV epitope DNA sequence. The deletion plasmid, designated pLA(GIII):30 dl HindIII/HpaI (see Figure 2), was constructed as follows.

1.0 µg of pLA(GIII):30 was added to 50 µl of a buffer comprising 50 mM NaCl, 50 mM Tris-HCl (pH 8.0), 10 mM MgCl$_2$, and 100 µg/ml of BSA (hereinafter "HindIII cutting buffer"). The DNA was digested for 2 hr with 20 units of HindIII enzyme (New England Biolabs, Inc.). The reaction was terminated by adding CDTA to 20 mM and heating at 65°C for 30 min. Ammonium acetate was added to 2.5 M and the DNA precipitated with 2 volumes of ethanol. The DNA precipitate was collected by centrifugation, and resuspended in 50 µl of a buffer comprising 20 mM KCl, 10 mM Tris-HCl$_2$ (pH 7.4), 1.0 mM dithiothreitol, and 100 µg/ml of BSA (hereinafter "HpaI cutting buffer"). The HindIII-digested pLA(GIII):30 DNA was digested with 3 units of HpaI enzyme (Pharmacia) for 1.5 hr at 37°C. The reaction was terminated and the DNA ethanol precipitated and collected by centrifugation as described above. The 3'-recessed HindIII terminus was filled in with Klenow's enzyme by redissolving the HindIII- and HpaI-digested pLA(GIII):30 in 25 µl of a buffer comprising 6.0 mM Tris-HCl (pH 7.5), 50 mM NaCl, 6.0 mM MgCl$_2$, 1.0 mM dithiothreitol, 0.2 mM dATP, dGTP, dCTP, TTP, and 1 unit of Klenow enzyme (Bethesda Research Laboratories, Inc.), then incubating at room temperature for 30 min. The reaction was terminated by heating the mixture at 70°C for 5 min. The HpaI-digested, HindIII-digested and filled in pLA-(GIII):30 plasmid DNA was added to 25 µl of 2 X ligation buffer and recircularized by an overnight incubation at 4°C with 1,000 units of T4 DNA ligase. The aliquots of the above ligation mixture were used to transform E. coli K12 strain NM522 as described above. Candidate colonies were picked and screened as described above to identify a recombinant plasmid of 6.3 kb from which the 0.9 kb HindIII to HpaI fragment of pLA(GIII):30 had been deleted. The deletion plasmid, designated pLA(GIII):30 dl HindIII/HpaI, was mapped by restriction enzyme methods (see Figure 2).

## D. Construction of a Plasmid with Cloning Sites for Insertion of the bGH-FMDV Epitope DNA Sequence: Derivation of pLA(GIII):30 dl HindIII/HpaI Oligo 171/172 Cassette

An oligonucleotide cloning/expression cassette was synthesized for insertion into the NcoI site of plasmid pLA(GIII):30 dl HindIII/HpaI. The cassette was designed so as to provide convenient cloning sites, optimal translation start signals for the bovine growth hormone (bGH) signal sequence, the FMDV epitope sequence, and suitable restriction nuclease sites to permit unidirectional nuclease digestion into the IBRV gIII gene. The plasmid obtained was designated pLA(GIII):30 dl HindIII/HpaI Oligo 171/172 (see Figure 2).

Two 32 mer oligonucleotides were synthesized on a Systec automated DNA synthesizer using CED-phosphoramidite chemistry according to the manufacturer's protocol. The trityl-on oligonucleotides were cleaved from the CPG-resin during incubation for 1.5 hr in concentrated $NH_4OH$, followed by incubation for 10 hr at 55°C to remove protecting groups. After lyophilization, the oligonucleotides were partially purified on Sea-Pak $C_{18}$ reverse phase chromatography cartridges (Waters Associates, Inc.). The Sea-Pak $C_{18}$ cartridges were wetted with 10 ml of acetonitrile, followed by 5.0 ml of 30% (v/v) acetonitrile and 100 mM triethylammonium bicarbonate (TEAB), followed by 10 ml of 25 mM TEAB. The oligonucleotides were dissolved in 10 ml of 25 mM TEAB and absorbed to the cartridges during filtration. The failure sequences lacking a 5′ terminal trityl group were eluted in a rinse of 15 ml of 10% (v/v) acetonitrile and 25 mM TEAB. The trityl-on oligonucleotide was then eluted with 5.0 ml of 30% (v/v) acetonitrile and 100 mM TEAB, and lyophilized. The residue was washed with 95% (v/v) ethanol and relyophilized. The trityl groups were removed by room temperature incubation for 45 min of the oligonucleotides in 0.5 ml of 80% (v/v) acetic acid. The acetic acid was removed by lyophilization, followed by a 95% (v/v) ethanol wash and lyophilization. The DNA concentration was determined by UV spectrophotometry. The oligonucleotides were further purified in 20 μg aliquots on 12% (w/v) polyacrylamide, 7.0 M urea gels (19% (w/v) acrylamide, 1.0% (w/v) BIS) using a Tris-borate-EDTA buffer system comprising 10.8 g of trizma base, 5.5 g of boric acid and 2.0 ml of 0.5 M EDTA, $Na_2$ (pH 8.0). The major band of DNA was visualized by UV shadowing, excised and transferred to a microcentrifuge tube. The gel was crushed and extracted during a 16-hr incubation at 37°C in 1.0 M TEAB. The extracted oligonucleotides were diluted 1:5 with water, and absorbed to a Sea-Pak $C_{18}$ cartridge by filtration. Contaminating gel material was rinsed through the filter with 10 ml of 25 mM TEAB. The purified oligonucleotide was eluted with 3.0 ml of 30% (v/v) acetonitrile and 100 mM TEAB, then lyophilized. The DNA residue was rinsed with 95% (v/v) ethanol and lyophilized. The DNA concentration was estimated by the extent of fluorescence enhancement of 5.0 μg/ml of ethidium bromide in 0.1 M NaCl using the crude oligonucleotide as standard (UV absorption standard). The quality of the purified oligonucleotide was verified on 8.0% (w/v) polyacrylamide gels after phosphorylation with gamma-$^{32}$P-ATP and T4 polynucleotide kinase.

The sequence of the oligonucleotides, arranged as a duplex was:

```
NcoI cohesive end (#1)
                      PstI    MluI  NsiI

Oligo 171   5'-CATGATGGCTGCAGCTACGCGTATGCATCTAC-3'

Oligo 172      3'-TACCGACGTCGATGCGCATACGTAGATGGTAC-5'

                             NcoI cohesive end (#2)
```

The NcoI cohesive end (#1) is not regenerated on ligation. NcoI cohesive end (#2) is regenerated on ligation. The $T_m$ of the duplex was calculated to be 52°C according to the formula:

$$T_m = 16.6 \, Log(M) + 0.41(\% \, G + C) + 81.5 - 820/L,$$

where M is the molarity of monovalent salts, %G+C is % guanine and cytosine content of the base paired duplex region, and L is the length of the base paired regions.

100 pmol of each oligonucleotide was dissolved in 20 μl of a buffer comprising 70 mM Tris-HCl (pH 7.6), 10 mM $MgCl_2$, 5.0 mM dithiothreitol, and 50 mM NaCl, and heated to 90°C for 3 min, then hybridized at 42°C overnight (10°C below the $T_m$). The hybridized oligonucleotides were stored at 4°C until time of use. The success of the annealing reaction was monitored in trial experiments by $^{32}$P-labeling of the oligonucleotides with polynucleotide kinase, and analysis of migration rates on non-denaturing 8.0% (w/v) polyacrylamide gels in the Tris-borate-EDTA buffer system.

2.0 μg of plasmid pLA(GIII):30 dl HindIII/HpaI DNA was dissolved in 100 μl of a buffer comprising 150 mM NaCl, 6.0 mM Tris-HCl (pH 7.9), 6.0 mM $MgCl_2$, and 100 μg/ml of BSA (hereinafter "NcoI cutting buffer"). The DNA was digested with 7 units of NcoI enzyme (New England Biolabs, Inc.) for 2 hr at 37°C. The reaction was terminated by adding CDTA to 20 mM and heating at 65°C for 30 min. The DNA was ethanol precipitated and collected by centrifugation as described above. The annealed oligonucleotides and the NcoI-digested pLA-(GIII):30 dl HindIII/HpaI were dissolved in ligation buffer with 1,000 units of T4 DNA ligase. After incubation for 16 hr at 4°C, the reaction was terminated by adding EDTA to 20 mM and heating to 65°C for 10 min. The oligonucleotide duplex tailed plasmid was separated from free oligonucleotides by centrifugal filtration through a miniature Sephadex G-25 column (5' Prime-3' Prime, Inc.). The oligonucleotide strand which was not covalently attached to the plasmid vector because of lack of a donating 5' prime phosphate group was released by denaturing the mixture at 80°C for 3 min. The complementary single strands which were attached to the respective NcoI termini of the vector through covalent bonds, which were formed from donation of the vector termini 5' prime phosphate groups, were annealed by incubation for 16 hr at 37°C. Aliquots of the ligation mixture were taken to transform E. coli K12 strain NM522 as described above. Colonies were picked and analyzed by the rapid plasmid screening procedure described above. The plasmid DNA of candidate recombinants was digested with MluI or PstI enzyme to identify clones linearized by both enzymes.

Clones which were linearized with these enzymes had the oligonucleotide cassette inserted into the NcoI site in one or the other orientations. The use of this linker tailer method assured that only a single cassette had been inserted because the oligonucleotides lacked 5'-phosphate groups to permit self-polymerization. In order to identify a clone with the cassette in the proper orientation relative to the IBRV gIII gene, single-stranded DNA was prepared for sequencing by the following method.

5.0 ml of 2YT media containing 0.1 mg/ml of ampicillin was inoculated with the recombinant clone and incubated overnight at 37°C. A fresh broth of 5.0 ml of 2YT media containing 0.1 mg/ml of ampicillin was inoculated in the morning with 0.7 ml of the starter culture, and incubated at 37°C for 6 hr. 2.0 ml of this log phase growing culture was taken to inoculate 100 ml of 2YT media containing 0.25 mg/ml of ampicillin, and incubated for 30 min at 37°C. Then, 0.4 ml of M13 K07 helper phage (International Biotechnologies, Inc.) was added to give a multiplicity of infection (hereinafter "m.o.i.") of 20, and incubation at 37°C was continued for another 30 min. Then, 0.14 ml of 50 mg/ml of kanamycin was added and the culture incubated at 37°C overnight. The bacteria were pelleted by centrifuging at 8,000 rpm at 4°C for 30 min in a Sorvall GSA rotor, and the supernatant subsequently collected by decanting into a second bottle. To the supernatant, 25 ml of 20% (w/v) PEG and 3.5 M ammonium acetate was admixed and incubated for 30 min on ice. The PEG precipitated phage was pelleted as described above, and the supernatant completely decanted and drained. The pellet was redissolved in 10 ml of 1 X TE buffer, and extracted with 5.0 ml of 90% (v/v) phenol. Then, the extraction was repeated after adding 5.0 ml of chloroform. The phases were separated by a 12,000 rpm centrifugation for 10 min in a Sorvall SS-34 rotor. The upper aqueous phase was removed and pooled with a 10 ml of 1 X TE buffer back extraction of the phenol/chloroform phase. The combined aqueous phase was again extracted with an equal volume of phenol/chloroform (1:1), and the phases separated by centrifugation. The aqueous phase was again extracted with an equal volume of chloroform, then brought to 2.5 M ammonium acetate. The DNA was ethanol precipitated and collected by centrifugation. The DNA was resuspended in 5.0 ml of water and reprecipitated as described above. The pellet was washed with ice cold 85% (v/v) ethanol, dried, and resuspended in 100 µl of TE buffer.

The M13 KO7 helper phage was prepared as follows.

A stock of phage was streaked on plates of LB agar containing 0.2% (w/v) glucose, then overlaid with a mid log phase NM522 culture in LB agar containing 0.2% (w/v) glucose. After an overnight incubation at 37°C, plaques were picked and a broth of 200 ml of 2YT media containing 70 µg/ml of kanamycin was inoculated. After an overnight incubation at 37°C, the bacteria was pelleted as described above, and the supernatant containing the helper phage used for single-stranded DNA preparation as described above.

The oligonucleotide cassette insert was sequenced using a synthetic primer-Oligo 164 prepared as described above and the Sequenase™ (United States Biochemicals, Inc.) DNA sequencing system.

The sequence of the primer-oligo 164 was:

5' - AAATGTAGCCCGCGCGCGGT - 3'

Primer-oligo 164 is 73 bp upstream of the NcoI site containing the inserted oligonucleotide cloning cassette.

0.01 µg of primer-oligo 164 was annealed to 1.0 µg of single-strand DNA in a 10 µl volume of sequencing buffer (United States Biochemicals, Inc.) at 65°C for 10 min, then slowly cooled to room temperature for 45 min in a buffer comprising 40 mM Tris-HCl (pH 7.5), 20 mM MgCl$_2$, and 50 mM NaCl. Then 1.0 µl of 0.1 M DTT, 0.5 µl of $^{35}$S-dATP(1200 Ci/mmole, 10 µCi/µl), 2.0 µl of 1.5 µM dGTP, dCTP, dTTP, and 2.0 µl of a 1:5 dilution in TE buffer of Sequenase™ (3 units/µl) was added, and incubated at room temperature for 5 min. Next, 3.5 µl of the above mixture was added to each of four tubes containing 2.5 µl of the individual dideoxyribonucleotides ddATP, ddGTP, ddCTP, ddTTP, and incubated at 37°C for 5 min. The reaction was terminated with 4.0 µl of 95% (v/v) formamide, 20 mM EDTA, 0.05% (w/v) bromophenol blue, and 0.02% (v/v) xylene cyanol FF. After heating to 85°C for 2 min and immersing in ice, 3.0 µl of the individual reaction mixture was added to a 8.0% (w/v) polyacrylamide, 7.0 M urea sequencing gel in the Tris-borate-EDTA buffer described above. The DNA was electrophoresed at 2,500 volts at 55°C for 2 hr. Then, the gel was washed in 10% (v/v) acetic acid, air-dried, and exposed to FUJI X-ray film.

The DNA sequence information obtained identified a recombinant with the oligonucleotide cassette inserted into the correct orientation and phase as desired (see Figure 2). The DNA sequence obtained is shown below in Table 1 below.

### Table 1
### Compiled Sequence for the pLA(GIII)30: dl HindIII/HpaI
### Oligo 171/172 cassette

```
TCGCGGCCATCTTGGATCCACCCGCGCGCACGACCGCCGAGAGACCGCCAGCCCGAGACCTCG
AGCGCCGGTAGAACCTAGGTGGGCGCGCCGTGCTGGCCGGCTCTCTGGCGGTCGGGCTCTGGAGC
```

```
            bgH                                    gIII structural
            signal→ PstI   MluI  NsiI      sequence →
CCGCGCGTCCGCCATGATGGCTGCAGCTACGCGTATGCATCTACCATGGGCCCGCTGGGGCGA
GGCGCGCAGGCGGTACTACCGACGTCGATGCGCATACGTAGATGGTACCCGGGCGACCCCGCT
```

```
GCGTGGCTGATCGCAGCTATTTTCGCCTGGG
CGCACCGACTAGCGTCGATAAAAGCGGACCC
```

II. Construction of a DNA Sequence for the bGH-FMDV Epitope Sequence and Cloning into M13Mp18

A. Synthesis Strategy

The following DNA sequence was chosen for synthesis.

### PstI Cohesive end

```
    179 →                                    146 →
    GGCCCCCGGACCTCCCTGCTCCTGGCTTTCGCCCTGCTCTGCCTGCCCTGGACTCAGGT
    ACGTCCGGGGGCCTGGAGGGACGAGGACCGAAAGCGGGACGAGACGGACGGGACCTGAGTCCA
                                                    ← 180
```

```
      148→                              183 →
GGTGGGCGCCAGACACAAACAGAAAATTGTGGCACCGGTGAAACAGACTCTGCCCCCCTCCGT
CCACCCGCGGTCTGTGTTTGTCTTTTAACACCGTGGCCACTTTGTCTGAGACGGGGGGGAGGCA
      ← 147                              ← 149
```

```
      152 →                              177 →
GCCCAACTTGAGAGGTGACCTTCAGGTGTTGGCTCAAAAGGTGGCACGGACGCCCGCCGGCGG
CGGGTTGAACTCTCCACTGGAAGTCCACAACCGAGTTTTCCACCGTGCCTGCGGGCGGCCGCC
      ← 151                              ← 153
```

### MluI
```
CCACGCGTG
GGTGCGCACTTAA
      ← 178
```

### Eco RI Cohesive end

The proximal end has a PstI cohesive terminus, whereas the distal end has an EcoRI cohesive terminus to facilitate cloning of the assembled oligonucleotides into an intermediate vector (M13mp18) suitable for rapid DNA sequencing of candidate recombinants (see Figure 3). The PstI site begins at nucleotide 8 of the bGH signal peptide. The FMDV sequence ends 4 bases before the MluI site which is adjacent to the EcoRI cohesive terminus. The plasmid pLA(GIII):30 dl HindIII/HpaI Oligo 171/172 (see Figure 2) contains the DNA sequence for the first eight nucleotides of bGH signal peptide. When the PstI to MluI region of the above assembled fragments is transferred into the pLA(GIII):30 dl HindIII/HpaI Oligo 171/172 cassette, the bGH-FMDV sequence will be in the same reading frame as the gIII protein.

B. Synthesis and Annealing of Oligonucleotides

The following oligonucleotides were synthesized and purified substantially as described above.

## Size:

42 mer Oligo 179  5' - GGCCCCCGGACCTCCCTGCTCCTGGCTTTCGCCCTGCTCTGC

51 mer Oligo 180  5' - GGCAGGCAGAGCAGGGCGAAAGCCAGGAGCAGGGAGGTCCGG

GGGCCTGCA

28 mer Oligo 146  5' - CTGCCCTGGACTCAGGTGGTGGGCGCCA

28 mer Oligo 147  5' - GTGTCTGGCGCCCACCACCTGAGTCCAG

28 mer Oligo 148  5' - GACACAAACAGAAAATTGTGGCACCGGT

28 mer Oligo 149  5' - GTTTCACCGGTGCCACAATTTTCTGTTT

31 mer Oligo 183  5' - GAAACAGACTCTGCCCCCCTCCGTGCCCAAC

31 mer Oligo 151  5' - CTCAAGTTGGGCACGGAGGGGGGCAGAGTCT

28 mer Oligo 152  5' - TTGAGAGGTGACCTTCAGGTGTTGGCTC

28 mer Oligo 153 5' -  CTTTTGAGCCAACACCTGAAGGTCACCT

37 mer Oligo 177  5' - AAAAGGTGGCACGGACGCCCGCCGGCGGCCACGCGTG

36 mer Oligo 178  5' - AATTCACGCGTGGCCGCCGGCGGGCGTCCGTGCCAC

All of the oligonucleotides, except 178 and 179, were phosphorylated as follows.
Oligo 178 and Oligo 179 were not 5'-phosphorylated to avoid self-ligation of the termini.
50 pmol of oligonucleotide was dissolved in 10 μl of a buffer comprising 70 mM Tris-HCl (pH 7.6), 10 mM MgCl$_2$, 5.0 mM dithiothreitol, and 30 μCi gamma-$^{32}$P-ATP (4300 Ci/mmole). 5 units of T4 polynucleotide kinase (New England Biolabs, Inc.) was added, and the reaction mixture was incubated for 15 min at 37°C. Then, the solution was brought to 0.7 mM ATP and the reaction continued for an additional 45 min. The reaction was terminated by heating to 70°C for 10 min, and NaCl was added to a final concentration of 50 mM. The reaction mixtures of the following pairs of oligonucleotides were combined:
(PK) Oligo 180 + Oligo 179 (No PK) T$_H$ = 60°C
(PK) Oligo 146 + (PK) Oligo 147 T$_H$ = 42°C
(PK) Oligo 148 + (PK) Oligo 149 T$_H$ = 32°C
(PK) Oligo 183 + (PK) Oligo 151 T$_H$ = 50°C
(PK) Oligo 152 + (PK) Oligo 153 T$_H$ = 37°C
(PK) Oligo 177 + Oligo 178 (No PK) T$_H$ = 58°C
The individual pairs of oligonucleotides were heated to 90°C for 3 min, then for 16 hr at the hybridization temperature which was 10°C below the T$_M$ calculated as described above. The annealed pairs of oligonucleotides were then stored at 4°C for 2 hr prior to use. The completeness of the annealing process was monitored by mobility changes on nondenaturing 8.0% (w/v) polyacrylamide gels as described above.

C. Cloning of Assembled Oligonucleotides: Derivation of M13mp18:bGH-FMDV Cl.11

The bGH-FMDV epitope DNA sequence was assembled and cloned into the PstI to EcoRI sites of M13mp18 during a single step in vitro ligation from pairs of annealed oligonucleotide duplexes with 5 bp overlapping cohesive ends as follows (see Figure 2).
3.8 μg of RF DNA of M13mp18 (New England Biolabs, Inc.) was digested with 50 units of PstI in 50 μl of a buffer comprising 100 mM NaCl, 10 mM Tris-HCl (pH 7.5), 10 mM MgCl$_2$, and 100 μg/ml of BSA (hereinafter "Pst I cutting buffer") at 37°C for 1 hr, and then brought to a 100 μl buffer comprising 100 mM Tris-HCl (pH 7.5), 50 mM NaCl, 4.0 mM MgCl$_2$, and 100 μg/ml of BSA, and digested with 50 units of EcoRI at 37°C for 1 hr.

The reaction was terminated by adding 10 μl of 0.25 M EDTA and heating at 65°C for 10 min. The cleaved DNA solution was layered on top of a 10% (w/v) to 40% (w/v) sucrose gradient (in 10 mM Tris-HCl (pH 7.6) and 50 mM NaCl) prepared in a 14 x 89 mm polyallomer tube and centrifuged in a Spinco SW41.1 rotor at 4°C at 3 9, 000 rpm for 20 hr. About 30 fractions were collected from the bottom of the tube, and each fraction was analyzed by 0. 8% (w/v) agarose gel electrophoresis to identify the fractions containing the M13mp18 Pst I-EcoRI fragment (7210 bp).

500 ng of the sucrose gradient purified RF DNA of M13mp18 PstI-EcoRI fragment was precipitated with 2 volumes of ethanol, dried, mixed with the six synthetic oligonucleotides (1.0 pmol each), and ligated in 40 μl of ligation buffer with 800 units of T4 DNA ligase at 4°C for 24 hr. The ligation was terminated by adding 160 μl of TE buffer.

A 100 ml culture of E. coli JM 103 cells in early exponential phase was centrifuged and cell pellets were suspended in 20 ml of 50 mM CaCl$_2$. Cells were pelleted again by centrifugation and resuspended in 5.0 ml of 50 mM CaCl$_2$. To a sterilized plastic tube, 50 μl of the ligation mixture described above and 200 μl of CaCl$_2$-treated E. coli JM 103 were added and kept at 4°C for 1 hr. The mixture was heated at 37°C for 5 min. Then, 200 μl of exponentially growing JM 103 cell suspension, 50 μl of 20 mg/ml X-gal, 10 μl of 0.1 M isopropyl-β-D-thiogalactopyranoside (hereinafter "ITPG") in 50% (v/v) dimethylformamide, and 3.0 ml of melted YT soft agar comprising 8.0 g of bactotryptone, 5.0 g of yeast extract, 5.0 g of NaCl, and 6.1 g of bacto-agar in 1.0 liter of H$_2$O (pH 7.0), were added sequentially, mixed and poured into a 60 mm Petri dish. The culture was kept at room temperature until the agar solidified, and then incubated at 37°C overnight.

Thirty colorless plaques were picked with toothpicks, inoculated into 0.2 ml of ML media comprising 10 g of bactotryptone, 5.0 g of yeast extract and 5.0 g of NaCl in 1.0 liter of H$_2$O (pH 7.3), in 96-well tissue culture clusters (Costar), and incubated at 37°C for 2 hr. Each plaque was then transferred to a sterile screw-capped tube (25 x 150 mm). Then, 10 ml of ML media, and 1.0 ml of an exponentially growing E. coli JM 103 culture were added, and the culture incubated at 37°C in a shaking incubator overnight.

The infected cultures were transferred to 15 ml glass Sorvall centrifuge tubes and centrifuged at 12,000 rpm in an SS-34 Sorvall rotor for 10 min. 8.0 ml of the clear supernatant was mixed with 4.0 ml of 1.5 M NaCl and 12% (w/v) polyethylene glycol (hereinafter "PEG" solution), kept at 4°C for 30 min, and centrifuged at 12,000 rpm for 10 min. The pellet of M13 phages was resuspended in 1.0 ml of TE buffer, transferred to a 1.5 ml Eppendorf plastic centrifuge tube, mixed with 0.5 ml of PEG solution, and centrifuged. The supernatant was discarded, the phage pellet was dissolved into 400 μl of a buffer comprising 10 mM Tris-HCl (pH 7.4), 1.0 mM EDTA, 0.3 M Na Acetate, 0.1% (w/v) SDS, and 200 μg/ml of Proteinase K, and incubated at 37°C for 1 hr. The solution was extracted once with 0.4 ml of a 1:1 mixture of phenol/chloroform, and single-stranded phage DNA was precipitated from the aqueous phase obtained after centrifugation by adding 2 volumes of ethanol. Precipitated DNA was washed once with ethanol, dried, and dissolved in 20 μl of TE buffer.

The sequences of cloned DNA were determined by Sanger's chain termination method. As a first step, only the T reaction was performed for all of the cloned samples (T test). After analyzing T bands of all of the candidates, clones 8, 9, 11, and 14, were subjected to a complete sequence determination. Clone 11 contained the desired sequence inserted into the PstI to EcoRI site of M13mp18 (see Figure 2). Clone 8 had an ambiguity at nucleotide 22. Clone 9 had two base substitutions, at nucleotide 140 A→C and at nucleotide 177 A→G. Clone 14 had a base substitution at nucleotide 172 C→A.

### III. Cloning of the bGH-FMDV Epitope Sequence From M13mp18:bGH-FMDV cl.11 into pLA(gIII):30 dl HindIII/HpaI Oligo 171(172: Derivation of pGIIIFM1

The bGH-FMDV epitope DNA sequence was inserted downstream of the IBRV gIII promoter as follows (see Figure 2).

The bGH-FMDV epitope DNA sequence was excised from RF DNA of M13mp18:bGH-FMDV cl.11 as a 0.2 kb PstI-MluI fragment and ligated into the PstI-MluI sites of pLA(GIII): 30 dl HindIII/HpaI Oligo 171/172 (map unit 2.6 to 2.7 of Figure 2).

14 μg of plasmid pLA(GIII):30 dl HindIII/HpaI Oligo 171/172 was digested with 100 units of PstI in 200 μl of PstI cutting buffer at 37°C for 5 min. Analysis by agarose gel electrophoresis revealed that the plasmid was partially cleaved in this condition. The linearized plasmid DNA was separated from the uncut superhelical DNA by 10% (w/v) to 40% (w/v) linear sucrose gradient centrifugation. The fractions containing linearized DNAs were identified by agarose gel electrophoresis. As shown in Figure 2, pLA(GIII):30 dl HindIII/HpaI Oligo 171/172 has two PstI sites, one in the synthetic cassette and the other at 90 bases upstream of the BamHI site. Therefore, this fraction contained DNAs cleaved at either of the PstI sites or at both sites. The DNAs precipitated by 2 volumes of ethanol from this fraction were cleaved with 50 units of MluI in 30 µl of a buffer comprising 50 mM NaCl, 10 mM Tris-HCl (pH 7.5), 7.0 mM $MgCl_2$, 6.0 mM 2-mercaptoethanol, and 100 µg/ml of BSA (hereinafter "MluI cutting buffer") at 37°C for 2 hr and were separated by 0.7% (w/v) agarose gel electrophoresis, stained by ethidium bromide. A 6.1 kb band was extracted from the agarose gel and purified by the glassmilk method described above.

5.0 µg of M13mp18:bGH-FMDV cl.11 was digested with PstI and MluI, extracted with phenol/chloroform (1:1) and mixed with the purified PstI-MluI fragment of pLA(GIII):30 dl HindIII/HpaI Oligo 171/172. The mixture was ligated with T4 DNA ligase and used to transform $CaCl_2$-treated E. coli K12 RR1 cells. The ampicillin-resistant colonies contained hybrid plasmid with the bGH-FMDV gene. However, the 160 bp PstI fragment which contains the regulatory sequence of the IBRV gIII gene was lost from all of the plasmids. Therefore, as a second step, this plasmid was digested with PstI, dephosphorylated with bacterial alkaline phosphatase, then ligated with the 160 bp PstI fragment, which was excised from the original pLA(GIII):30 dl HindIII/HpaI Oligo 171/172, separated by gel electrophoresis, and purified by the glassmilk method. The insertion of the PstI fragment in proper orientation was confirmed by sequencing as shown in Table 2 below. The resulting plasmid was designated pGIIIFM1 (see Figure 2).

## Table 2
## DNA Sequence Compilation

GCACGCCGCGCTGTGCTTCTGCGTCGGGGCCTGCGTGGGTCTGCGCCGACGAATGAACGGCTG

CCGCCGACGGTAACGCGCCTGCAGCCGCGCGTGTGCTCAATCCCGBACCACGAAAGCACAAAA

CGGACGCCCTTAAAAATGTAGCCCGCGCGCGGTCGCGGCCATCTTGGATCCACCCGCGCGCAC

```
                                                       bGH signal →
GACCGCCGAGAGACCGCCAGCCCGAGACCTCGCCGCGCGTCCGCC  MET MET ALA ALA
                                                   ATG ATG GCT GCA

GLY PRO ARG THR SER LEU LEU LEU ALA PHE ALA LEU LEU CYS LEU PRO
GGC CCC CGG ACC TCC CTG CTC CTG CGT TTC GCC CTG CTC TGC CTG CCC

                      FMDV epitope
                      (O₁K amino acids 200-213) →
TRP THR GLN VAL VAL GLY ALA ARG HIS LYS GLN LYS ILE VAL ALA PRO
TGG ACT CAG GTG GTG GGC GCC AGA CAC AAA CAG AAA ATT GTG GCA CCG

                                 FMDV epitope
                 ___spacer___  (O₁K amino acids 141-158) →
VAL LYS GLN THR LEU PRO PRO SER VAL PRO ASN LEU ARG GLY ASP LEU
GTG AAA CAG ACT CTG CCC CCC TCC GTG CCC AAC TTG AGA GGT GAC CTT

                                           ___spacer___
GLN VAL LEU ALA GLN LYS VAL ALA ARG THR PRO ALA GLY GLY HIS ALA
CAG GTG TTG GCT CAA AAG GTG GCA CGG ACG CCC GCC GGC GGC CAC GCG
                                                           MluI

            gIII →
TYR ALA SER THR MET GLY PRO LEU GLY ARG ALA TRP LEU ILE ALA ALA
TAT GCA TCT ACC ATG GGC CCG CTG GGG CGA GCG TGG CTG ATC GCA GCT
    NsiI        NcoI

ILE PHE ALA TRP ALA LEU LEU SER ALA ARG ARG GLY LEU ALA GLU GLU
ATT TTC GCC TGG GCG CTC CTG TCT GCC CGG CGG GGG CTC GCC GAG GAG

ALA GLU ALA SER PRO SER PRO PRO PRO SER PRO SER PRO THR GLU THR
GCG GAA GCC TCG CCC TCG CCT CCG CCC TCC CCG TCC CCA ACC GAG ACG

GLU SER SER ALA GLY
GAA AGC TCC GCT GGG ...
```

### IV. Variable Unidirectional Deletion of gIII Gene Sequence from bGH-FMDV Fusion Sequence

In plasmid pGIIIFM1, the bGH-FMDV epitope sequence was fused with the IBRV gIII coding sequence as shown in Table 2 above. Since the signal peptide of the fusion protein was provided by the bGH gene, and the signal peptide coded by the IBRV gIII might interfere with membrane translocation, the DNA sequence coding for the IBRV signal peptide was deleted by the following procedures.

### A. Subcloning of the Target Sequence: Derivation of M13mp18/pGIIIFM1 BamHI-SacI

Plasmid pGIIIFM1 was digested with BamHI and SacI and the resulting 793 bp fragment, which contains the entire bGH-FMDV epitope sequence and a part of the IBRV gIII gene, was cloned at the BamHI and SacI sites of M13mp18 as follows (see Figure 3).

27

0.4 μg of RF DNA M13mp18 (Bethesda Research Laboratories, Inc.) and 1.0 μg of pGIIIFM1 DNA were mixed and digested with 40 units of SacI in 20 μl of a buffer comprising 10 mM Tris-HCl (pH 7.4), 10 mM MgCl₂, 10 mM 2-mercaptoethanol, and 100 μg/ml of BSA (hereinafter "SacI cutting buffer") at 37°C for 2 hr. Then, the DNAs were digested with 50 units of BamHI in 100 μl of a buffer comprising 150 mM NaCl, 6.0 mM Tris-HCl (pH 7.9), 6.0 mM MgCl₂, 6.0 mM 2-mercaptoethanol, and 100 μg/ml of BSA (hereinafter "BamHI cutting buffer") at 37°C for 2 hr. The digested DNAs were treated once with 100 μl of phenol/chloroform mixture (1:1), and precipitated by adding 50 μl of 7.5 M ammonium acetate and 300 μl of ethanol. The DNA precipitates were rinsed with ethanol, dried, and dissolved in 40 μl of ligation buffer. Then, 400 units of T4 DNA ligase was added and the ligation was carried out at 4°C overnight.

CaCl₂ -treated E. coli, K12 JM 103 cells were transformed with the ligated DNA in the presence of IPTG and X-gal as described above. Colorless plaques were picked, phages were propagated, and the single-stranded DNA was prepared from each phage. The phages containing the 793 bp BamHI-SacI fragment were identified by sequencing using Oligo 183 synthetic primer described above, which is homologous to portions of the FMDV epitope sequence. From a confirmed M13mp18/pGIIIFM1 BamHI-SacI clone, RF DNA was prepared by the standard procedures.

### B. Unidirectional Exonuclease Digestion of M13mp18/pGIIIFM1 BamHI-SacI: Derivation of M13mp18/cl.13

RF DNA of M13mp18/pGIIIFM1 BamHI-SacI was digested at the NsiI and NcoI sites and then digested further with exonuclease III (E. coli) for 30 to 60 sec. NsiI and NcoI digestions created 3′ protruding ends and 5′ protruding ends, respectively. Exonuclease III preferentially digested the 5′ protruding ends towards the IBRV gIII gene while the bGH-FMDV epitope sequence was protected by the 3′ protruding NsiI site.

More specifically, 3.0 μg RF DNA of M13mp18/pGIIIFM1 BamHI-SacI was digested with 50 units of the restriction enzyme NsiI in 200 μl of a buffer comprising 150 mM NaCl, 10 mM Tris-HCl (pH 8.4), 10 mM MgCl₂ , and 100 μg/ml of BSA (hereinafter "NsiI cutting buffer") at 37°C for 16 hr. DNA was extracted once with phenol:chloroform (1:1), precipitated by adding 0.5 volumes of 7.5 M ammonium acetate and 3 volumes of ethanol, rinsed with ethanol, dried and digested again with 70 units of the restriction enzyme NcoI in 200 μl of buffer comprising 150 mM NaCl, 10 mM Tris-HCl (pH 7.9), 10 mM MgCl₂, 100 μg/ml of BSA (hereinafter "NcoI cutting buffer") at 37°C for 2 hr. DNA was precipitated with 0.5 volumes of 7.5 M ammonium acetate and 3 volumes of ethanol, rinsed with ethanol, and dried. The DNA pellet was dissolved in 30 μl of 66 mM Tris-HCl (pH 8.0) and 0.66 mM MgCl₂ to a concentration of about 100 μg/ml. One-tenth volume of exonuclease III containing 300 units (New England Biolabs, Inc.) was added to give an excess of enzyme over susceptible DNA ends of about 20 to 1. Incubation was carried out at 37°C, and 10 μl of aliquots were removed at 30, 60, and 120 sec and mixed with 50 μl of 0.2 M NaCl and 5.0 mM EDTA (pH 8.0) in 0.5 ml microcentrifuge tubes.

The enzyme was inactivated by incubation at 70°C for 10 min. The DNA was precipitated by addition of 120 μl of ethanol to each sample, chilled to -70°C, pelleted by centrifugation, rinsed with 100 μl of ethanol, decanted, and dried. The exonuclease III-digested DNA was then treated with Mung bean nuclease and T4 DNA polymerase to create flush ends which could be ligated in vitro as follows.

Each pellet was dissolved in 50 μl of 50 mM NaCl, 30 mM sodium acetate (pH 4.6), 1.0 mM ZnCl₂ and 155 units of Mung bean nuclease and incubated at room temperature for 30 min. Reactions were stopped by addition of 6.0 μl of 0.5 M Tris-HCl (pH 8.0). Portions of selected aliquots were examined by electrophoresis on 0.8% (w/v) agarose gels to determine the actual rate of exonuclease degradation and to decide which aliquots would be used.

Samples were vortexed with 25 μl of phenol and 25 μl of chloroform spun in microcentrifuge, and the aqueous layers transferred to fresh 0.5 ml,tubes. After addition of 140 μl of ethanol, the samples were chilled, and the DNA was precipitated, pelleted, rinsed, and dried as above.

Each pellet was dissolved in 2.0 μl of a buffer comprising 0.33 M Tris-HCl (pH 7-9), 0.66 M potassium acetate, 0.10 M magnesium acetate, 5.0 mM dithiothreitol, and 1.0 mg/ml of BSA (hereinafter "10 X T4 DNA polymerase buffer"), 1.0 μl of dNTP solution (2.0 mM each of 4 dNTP), 16 μl of water, and 1.0 μl of T4 DNA polymerase (5 units). The reaction was carried out at 37°C for 5 min and terminated by adding 1.0 μl of 0.5 M EDTA (pH 7.6). The mixture was extracted once with phenol:chloroform (1:1), and DNA was precipitated by adding 0.5 volumes of 7.5 M ammonium acetate and ethanol, dried, and ligated in 40 μl of ligation buffer, and 800 units of T4 ligase at 4°C overnight.

Exponentially growing E. coli K12 JM 103 cells were treated with 50 mM CaCl₂, mixed with the ligated DNA, plated with fresh JM103 cells and incubated at 37°C overnight. Forty plaques were picked, propagated, and single-stranded DNA was prepared from each of them. T tests were performed with each sample using synthetic primer Oligo 183, containing a part of FMDV epitope gene sequence described above. Out of 40 clones, 15 clones gave distinct bands and with these clones DNA sequences were determined. Clone 13 retained the bGH-FMDV sequence, lost the first 38 amino acids of IBRV gIII, but retained the remaining gIII amino acids in the same translational frame with the bGH-FMDV epitope. Similarly, clone 32 lost 78 amino acids of IBRV gIII, but the rest of the amino acids were in the same translational frame. Clone 33 lost 90 amino acids of the IBRV gIII gene. Clone 13 was designated M13mp18/cl.13 (see Figure 3).

## Table 3
### DNA Sequence of M13mp18/cl.13 from BamHI to SacI

```
bgH signal →
ATG ATG GCT GCA GGC CCC CCG ACC TCC CTG CTC CTG GCT TTC GCC CTG
MET MET ALA ALA GLY PRO ARG THR SER LEU LEU LEU ALA PHE ALA LEU

CTC TGC CTG CCC TGG ACT CAG GTG GTG GGC GCC AGA CAC AAA CAG AAA
LEU CYS LEU PRO TRP THR GLN VAL VAL GLY ALA ARG HIS LYS GLN LYS
                                        FMDV epitope
                                        (O₁K amino acids
                                        200-213) →

ATT GTG GCA CCG GTG AAA CAG ACT CTG CCC CCC TCC GTG CCC AAC TTG
ILE VAL ALA PRO VAL LYS GLN THR LEU PRO PRO SER VAL PRO ASN LEU
                                        spacer    FMDV epitope
                                                  (O₁K amino
                                                  acids 141-158)

AGA GGT GAC CTT CAG GTG TTG GCT CCA AAG GTG GCA CGG ACG CCC GCC
ARG GLY ASP LEU GLN VAL LEU ALA GLN LYS VAL ALA ARG THR PRO ALA
                                                            spacer

        39th amino acid gIII →
GGC GGC CCC CCG TCC CCA ACC GAG ACG GAA AGC TCC GCT GGG ACC ACC
GLY GLY PRO PRO SER PRO THR GLU THR GLU SER SER ALA GLY THR THR

GGC GCA ACG CCC CCC ACG CCC AAC AGC CCC GAC GCT ACG CCA GAG GAC
GLY ALA THR PRO PRO THR PRO ASN SER PRO ASP ALA THR PRO GLU ASP

AGC ACT CCC GGT GCT ACT ACG CCC GTG GGG ACG CCG GAG CCG CCG TCC
SER THR PRO GLY ALA THR THR PRO VAL GLY THR PRO GLU PRO PRO SER

GTG TCC GAG CAC GAC CCG CCC GTT ACC AAC AGC ACG CCG CCG CCC GCC
VAL SER GLU HIS ASP PRO PRO VAL THR ASN SER THR PRO PRO PRO ALA

CCG CCC GAG GAC GGG CGA CCC GGC GGC GCT GGC AAC GCG AGC CGC GAT
PRO PRO GLU ASP GLY ARG PRO GLY GLY ALA GLY ASN ALA SER ARG ASP

GGG CGA CCT AGC GGC GGG GGG CGG CCT CGC CCC CCG CGG CCG AGC AAA
GLY ARG PRO SER GLY GLY GLY ARG PRO ARG PRO PRO ARG PRO SER LYS

GCC CCG CCG AAG GAG CGC AAG TGG ATG CTC TGC GAG CGC GAG GCC GTG
ALA PRO PRO LYS GLU ARG LYS TRP MET LEU CYS GLU ARG GLU ALA VAL
```

Table 3 (Continued)

```
GCC GCC TCG TAC GCC GAG CCG CTG TAC GTG CAC TGC GGC GTG GCC GAC
ALA ALA SER TYR ALA GLU PRO LEU TYR VAL HIS CYS GLY VAL ALA ASP

AAC GCC ACT GGC GGT GCG CGC CTG GAG CTC
ASN ALA THR GLY GLY ALA ARG LEU GLU LEU ...
```

C. Replacement of BamHI-EcoRI Fragment of pGIIIFM1 with BamHI-EcoRI Fragment from M13mp18/cl.13 with Proximal gIII Gene Deletion: Derivation of pGIIIFM1/cl.13 (BamHI-EcoRI)

In order to increase the size of the sequences flanking the bGH-FMDV epitope, the BamHI-EcoRI fragment from M13mp18/cl. 13 (6. 9 to 6. 2 map units) was inserted into pGIIIFM1 lacking the BamHI-EcoRI fragment from 2. 8 to 0. 2 map units as follows (see Figure 3).

8.0 μg of plasmid pGIIIFM1 DNA was digested with 100 units of EcoRI in 200 μl of EcoRI cutting buffer at 37°C for 2 hr. The reaction mixture was extracted once with phenol:chloroform (1:1), DNA was precipitated with 2 volumes of ethanol and digested with 100 units of BamHI in 200 μl of BamHI cutting buffer. Digested pGIIIFM1 was separated by electrophoresis in a 0.8% (w/v) agarose gel and the 4.0 kb fragment was purified from the gel by the glassmilk method as described above.

RF DNA of M13mp18/cl.13, M13mp18/cl.32, or M13mp18/cl.33 was digested with BamHI and EcoRI and mixed with the purified 4.0 kb BamHI-EcoRI fragment of pGIIIFM1, ligated with T4 DNA ligase at 4°C overnight, and purified by phenol:chloroform (1:1) extraction and ethanol precipitation as described above. E. coli K12 RR1 cells were treated with CaCl$_2$ and transformed with the ligated DNAs as described above. Ampicillin-resistant colonies were screened and plasmids pGIIIFM1/cl.13 (BamHI-EcoRI), p G I I F M 1 / c l . 3 2 (B a m H I - E c o R I) , a n d pGIIIFM1/cl.33 (BamHI-EcoRI) were obtained (see Figure 3). These plasmids contain 5' flanking sequences of IBRV gIII gene, the regulatory sequence of IBRV gIII gene, bGH-FMDV epitope gene, and a part of IBRV gIII coding sequence in which the signal peptide is deleted.

D. Replacing SacI Fragment of pGIIIFM1 with SacI Fragment of pGIIIFM1/cl.13 (BamHI-EcoRI), pGIIIFM1/cl.32 (BamHI-EcoRI), pGIIIFM1/cl.33 (BamHI-EcoRI): Derivation of pGIIIFM1/cl.13, pGIIIFM1/cl.32 and pGIIIFM1/cl.33

The 3' flanking sequences of pGIIFM1/cl.13 (BamHI-EcoRI), pGIIIFM1/cl.32 (BamHI-EcoRI) and pGIIFM1/cl.33 (BamHI-EcoRI) are too short to facilitate the marker rescue of bGH-FMDV into IBRV. Therefore, plasmids which have longer arms of flanking sequences were constructed as follows (see Figure 4).

8.0 μg of pGIIIFM1 DNA in 100 μl of SacI cutting buffer was partially digested with 40 units of SacI at 37°C for 10 min. The reaction was terminated by adding 5.0 μl of 0.25 M EDTA, and extracting once with an equal volume of phenol:chloroform mixture (1:1), followed by DNA precipitation with 2 volumes of ethanol. The 5' phosphate ends of the partially digested DNA were hydrolyzed with 4 units of bacterial alkaline phosphatase (BAP) in 50 μl of a buffer comprising 0.05 M Tris-HCl (pH 8.0) and 0.05 M NaCl at 65°C for 1 hr. BAP was inactivated by adding Proteinase K to a final concentration of 100 μg/ml and incubating at 37°C for 30 min. Proteinase K was inactivated by phenol:chloroform extraction. The partially SacI-digested pGIIIFM1 fragments were separated by 0.8% (w/v) agarose gel electrophoresis and the 5.2 kb fragment was purified from the agarose gel by the glassmilk method described above.

8.0 μg of pGIIIFM1/cl.13 (BamHI-EcoRI), pGIIIFM1/cl.32 (BamHI-EcoRI), or pGIIIFM1/cl.33 (BamHI-EcoRI) was completely digested with 100 units of SacI in 100 μl of SacI buffer at 37°C for 2 hr. Digested fragments from each plasmid were separated by 0.8% (w/v) agarose gel electrophoresis. A 1.3 kb fragment from 0.2 to 1.4 map units of pGIIIFM1/cl.13 (BamHI-EcoRI), in which the bGH-FMDV gene sequence was located, was excised from the agarose gel, and purified by the glassmilk method described above.

The 5.2 kb SacI fragment of pGIIIFM1 lacking the SacI fragment from 2.0 to 3.2 map units was mixed with the 1.3 kb SacI fragment of pGIIIFM1/cl.13 (BamHI-EcoRI), or pGIIIFM1/cl.32 (BamHI-EcoRI), or pGIIIFM1/cl.33 (BamHI-EcoRI), and ligated with T4 DNA ligase as described above. Thereafter, E. coli K12 RR1 was transformed with the above DNA, ampicillin-resistant colonies were selected, and the plasmids present in these colonies were analyzed with restrictions enzymes. The clones were designated pGIIIFM1/cl.13, pGIIIFM1/cl.32, and pGIIIFM1/cl.33. The restriction map of pGIIIFM1/cl.13, which was utilized in subsequent constructions, is shown in Figure 4.

### V. Transfer of bGH-FMD-gIII Deletion Sequences to a Plasmid with Larger IBRV Flanking Sequences: Derivation of pLAHK/cl.13, pLAHK/cl.32, and pLAHK/cl.33

To increase the size of the IBRV flanking sequences further, the BamHI-EcoRI fragment of pGIIIFM1/cl. 13 from 2. 7 to 0. 2 map units was exchanged for the BamHI-EcoRI fragment of pLAHK from 3. 2 to 0.8 map units as follows (see Figure 4).

7.0 μg of pLAHK DNA was digested with BamHI and EcoRI, separated by 0.6% (w/v) agarose gel electrophoresis, and the 7.8 kb fragment was purified by the glassmilk method described above. This resulted in deleting the BamHI-EcoRI fragment of pLAHK from 3.2 to 0.8 map units.

1.0 μg of pGIIIFM1/cl.13, pGIIIFM1/cl.32, or pGIIIFM1/cl.33 was completely digested with BamHI and EcoRI. Each digested plasmid DNA was mixed with the purified 7.8 kb BamHI-EcoRI fragment of pLAHK, ligated by T4 DNA ligase, and added to CaCl$_2$ treated E. coli RR1 cells as described above. Many plasmids were screened from ampicillin-resistant colonies to obtain pLAHK/cl.13, pLAHK/cl.32, and pLAHK/cl.33. In pLAHK/cl.13, the BamHI-EcoRI fragment of pGIIIFM1/cl.13 from 2.7 to 0.2 map units was ligated to the 7.8 kb BamHI-EcoRI fragment of pLAHK (see Figure 4).

### VI. Construction of a Plasmid with the β-Gal Gene Expressible from the gIII Promoter

#### A. Construction of a Plasmid with an Adaptor at the Start Codon of the gIII Gene: Derivation of pLA(GIII):31 dl HindIII/HpaI dl NS

The IBRV gIII gene was subcloned as described above for the construction of pLA(GIII):30 dl HindIII/HpaI, except that the cloning vector employed was pIBI31 (International Biotechnologies, Inc.) instead of pIBI30 (see Figure 1 and Figure 2). These cloning vectors are identical, except that the cloning cassette is inverted in pIBI31 relative to pIBI30. The derived plasmid was designated pLA(GIII):31 dl HindIII/HpaI. This plasmid was further modified as follows, so as to provide convenient insertion sites and in-phase reading frame for the β-gal gene (see Figure 5).

2.0 μg of pLA(GIII):31 HindIII/HpaI was dissolved in 100 μl of NcoI cutting buffer, then digested with 7 units of NcoI enzyme for 2 hr at 37°C. The reaction was terminated and the DNA precipitated and collected by centrifugation as described above.

The NcoI-digested pLA(GIII): 31 dl HindIII/HpaI was redissolved in 100 μl of a buffer comprising 150 mM NaCl, 6.0 mM Tris-HCl (pH 7.9), 6.0 mM MgCl$_2$, 6.0 mM 2-mercaptoethanol, and 100 μg/ml of BSA (hereinafter "SalI cutting buffer"). The DNA was digested with 20 units of SalI enzyme (New England Biolabs, Inc.) for 2 hr at 37°C. The reaction was terminated and the DNA ethanol precipitated and collected by centrifugation as described above.

The following oligonucleotide adapters were synthesized by CED-phosphoramidite chemistry, and purified essentially as described above.

Oligo 138 5' - CATGGCAGAATCCTG
Oligo 139 5' - TCGACAGGATTCTGC

The purpose of the adaptor is to provide cloning sites for insertion of the β-gal gene which permit in-phase translation off of the gIII start codon at the NcoI site. When annealed, the oligonucleotides formed the following duplex adaptor:

```
            NcoI cohesive

        5' - CATGGCAGAATCCTG

             CGTCTTAGGACAGCT - 5'

                    SalI cohesive
```

The unphosphorylated oligonucleotides were annealed by heating 100 pmol of each in 50 μl of ligation buffer without ATP to 70°C for 5 min, then slowly cooling to room temperature. The oligonucleotide duplex was added to the NcoI and SalI-digested pLA(GIII): 31 dl HindIII/HpaI in ligation buffer, and ATP was added to a concentration of 1.0 mM. The oligonucleotide duplex was ligated to the vector by incubation with 1,000 units of T4 DNA ligase for a 16 hr at 3°C. The reaction was terminated by bringing the mixture to 20 mM EDTA and heating at 70°C for 10 min. The heating step denatured the oligonucleotide which was not covalently bound to the vector. The mixture was allowed to slowly cool to 4°C during which time the complementary single-stranded oligonucleotides at the different termini could anneal to recircularize the plasmid. Aliquots of the reaction mixture were used to transform E. coli K12 strain NM522 as described above. Plasmid DNA of recombinants were prepared as described above. Clones which were decreased in size from 6.3 kb to 4.9 kb due to deletion of the NcoI to SalI region of the gIII gene were analyzed further for the presence of regenerated NcoI and SalI sites due to the insertion of the synthetic adaptor. Plasmid DNA was dissolved in NcoI or SalI cutting buffer and digested with 2 or 7 units of enzyme, respectively. A clone was identified which was linearized by NcoI or SalI and designated pLA(GIII):31 dl HindIII/HpaI dl NS (see Figure 5).

## B. Insertion of β-gal Gene into Expression Plasmid: Derivation of pLA(PrGIII-β-gal:31

The β-gal gene was inserted into the pLA(GIII):31 dl HindIII/HpaI dl NS plasmid to obtain pLA(PrGIII-β-gal):31 as follows (see Figure 5).

2.0 μg of pMC1871 (Pharmacia) and 2.0 μg of pLA(GIII):31 dl Hind/Hpa dl NS were dissolved separately in 100 μl of SalI cutting buffer and digested with 20 units of SalI enzyme for 2 hr at 37°C. The reaction was terminated, and the DNA ethanol precipitated and collected by centrifugation as described above.

The DNA was redissolved in 50 μl of ligation buffer and ligated with 1,000 units of T4 DNA ligase and incubated for a 16 hr incubation at 4°C. The reaction was terminated and E. coli K12 strain NM522 transformed as described above. Plasmid DNA of transformants was prepared and analyzed for a 7.9 kb recombinant containing the 3.0 kb SalI β-gal gene from pMC1871 inserted into the SalI site of pLA(GIII):31 dl HindIII/HpaI dl NS. One such clone was designated pLA(PrGIII-β-gal):31 (see Figure 5).

In order to verify that the oligonucleotide adaptor had placed the β-gal gene in proper translation phase, the DNA region at the start of the β-gal gene was cloned into M13 and sequenced as follows.

1.5 μg of plasmid pLA(PrGIII-β-gal):31 and 0.5 μg of RF DNA of M13mp19 were mixed and digested with 20 units of BamHI in 20 μl of BamHI cutting buffer at 37°C for 1 hr. After the incubation, the reaction mixture was extracted with phenol:chloroform mixture (1:1), and the DNAs were precipitated from the aqueous phase with 2 volumes of ethanol. The DNA precipitates were rinsed with ethanol, dried, ligated in 40 μl of ligation buffer with 400 units of T4 DNA ligase at 4°C for 18 hr. CaCl₂-activated E. coli JM 103 cells were transformed with the ligated DNA and plated together with exponentially growing JM 103 cells in agar plates containing IPTG, and X-gal as described above. After overnight incubation at 37°C, colorless plaques were picked and single-stranded DNA was prepared from each plaque. The sequences were determined by the chain termination method as described above.

Three BamHI fragments of pLA(PrGIII-β-gal):31, which were 25 bp, 88 bp, and 3.0 kbp, were subcloned in M13mp19, and the shorter two fragments were sequenced. The reverse complement of the sequence spans the junction between the gIII promoter and initiation of the β-galactoside structural gene (see Table 4). The sequence confirmed that the β-gal gene had been inserted in phase with the start codon of gIII.

<div align="center">

Table 4
gIII Promoter Sequences Upstream
of gIII Start Codon at NcoI

</div>

```
                                       NcoI                    SalI
......CCCGAGACCTCGCCGCGCGTCCGCC ATG GCA GAA TCC TGT CGA CGG ATC

                                8   9
                                PRO VAL
        CGG GGA ATT CCC GGG GAT CCC GTC....
                                Lac Z →
```

## VII. Construction of IBRV Expressing the β-Gal Gene from the gIII Locus

An IBRV insertion mutant in the gIII gene expressing the β-gal gene from the gIII promoter was obtained by transfection of cells with DNA from both IBRV(NG)dltk and the plasmid pLA(PrGIII-β-gal):31 as follows.

Rab-9 cells were seeded in 60-mm Petri dishes (2.0 X 10⁵ cells per 5.0 ml of growth media per dish) and incubated at 37°C for 48 hr. Then, the following sterile solutions were added to a test tube in sequential order:

(1) 0.02 ml of 50 μg/ml of IBRV(NG)dltk DNA in TE buffer;
(2) 0.2 ml of 10 μg/ml of plasmid pLA(PrGIII-β-gal):31 in 0.1 X TE buffer;
(3) 0.65 ml of water;
(4) 1.0 ml of 20 μg/ml of salmon sperm DNA in buffer solution comprising 16 g/L of NaCl, 0.74 g/L of KCl, 0.25 g/L of Na$_2$HPO, 2H$_2$O, 2.0 g/L of glucose, 10 g/L of Hepes (pH 7.05) (hereinafter "2 X Hepes buffer solution"); and

(5) 0.13 ml of 2.0 M CaCl$_2$.

The resulting solution was mixed by inversion and kept at room temperature for 30 min while a DNA-calcium phosphate precipitate formed. Then, 0.5 ml of the suspension was added directly to the Rab-9 cells in the Petri dishes. The cells were incubated at 37°C for 5 hr. Then, the growth media was aspirated, and the monolayer rinsed with 5.0 ml of fresh growth media, followed by the addition of 5.0 ml of fresh growth media. The cultures were incubated at 37°C for 5 days until extensive cytopathiceffects occurred. The cultures were frozen and stored at -80°C.

Recombinant IBRV clones expressing β-galactosidase were isolated as follows.

The progeny virus obtained from the cotransfection above mainly comprised parental IBRV(NG)dltk and a small population of homologous recombinants in which substantially all of the coding sequence of the IBRV gIII gene was deleted and replaced by the β-gal gene downstream of the gIII gene promoter. To identify and screen for recombinant viruses among parental viruses, an enzymatic colorometric plaque assay was utilized. In the assay for β-galactosidase produced by the recombinant virus-infected cells, the colorless substrate X-gal is converted to an insoluble colored product by β-galactosidase. The virus harvested from the cotransfection was serially diluted in growth media. Then, 0.1 ml of aliquots of the serial dilutions were added to the wells of a 6-well cluster tissue culture tray containing near confluent BK cells from which growth media had just been aspirated. The virus inoculum was absorbed to the cell monolayer during incubation for 1 hr at 37°C. The infected cells monolayer was overlaid with 2.5 ml per well of 1.25% (w/v) agar, McCoy's 5A solution, 0.04% (w/v) protamine sulfate, 2.0 mM glutamine, 50 µg/ml of neomycin, 10 mM Hepes (pH 7.2) and, 50 units/ml of mycostatin plus 10% (v/v) fetal calf serum. The cultures were incubated for 5 days at 34.5°C, then overlaid with 2.5 ml per well of the above solution supplemented with 200 µg/ml of X-gal. After an overnight incubation at 34.5°C, colored plaques were identified and picked with a Pasteur pipette. The agar plug was suspended in 1.0 ml of growth media, and vortexed briefly. Then, a six-ounce prescription bottle containing near confluent BK cells from which the growth media had been just decanted was inoculated with the isolated plaque. After 3 days at 34°C, when complete cytopathic effects had occurred, the infected cell monolayer was frozen at -40°C, thawed, and harvested. A second and third plaque purification was repeated as described above.

IBRV DNA was prepared essentially as described by Pignatti et al for the preparation of HSV DNA (Pignatti et al, Virol., 93:260-264 (1979)).

More specifically, 20 eight-ounce prescription glass bottle monolayer cultures of BK cells (about 5.0 X 10$^6$ cells/culture) containing 20 ml of growth medium were infected at a m.o.i. of 5.0 p.f.u./cell of IBRV and incubated for 3 hr at 34.5°C, at which time cellular and DNA synthesis had been inhibited by the viral infection. Then, 1.0 µCi/ml and 0.25 µg/ml of $^3$H-thymidine was added to radioactively label the viral DNA, and incubation was continued at 34.5°C for another 17 hr. The cells were dislodged from the glass by scraping into the growth medium with a rubber policeman, centrifuged at 600 x g, washed with ice cold phosphate-buffered saline solution comprising 0.14 M NaCl, 0.003 M KCl, 0.001 M CaCl$_2$, 0.0005 M MgCl$_2$, and 0.01 M sodium phosphate (pH 7.5) (hereinafter "PBS"), containing 10 µg/ml of nonradioactive thymidine. Next, the cells were centrifuged at 600 x g and then frozen in an ethanol-dry ice bath.

After thawing, the cell pellet was resuspended in 9 volumes of lysing solution comprising 0.25% (w/v) Triton X-100, 10 mM EDTA, and 10 mM Tris-HCl (pH 7.9). Next, the cell suspension was transferred to a Dounce homogenizer, and incubated at room temperature for 20-30 min with gentle mixing.

Then, the cell suspension was transferred to a glass centrifuge tube and NaCl was added to a final concentration of 0.2 M. Next, the tube was inverted several times, and the solution was immediately centrifuged at 1,000 x g at 4°C for 10 min.

The resulting supernatant was decanted into a glass tube and deproteinized by incubating with 100 µg/ml of Proteinase K in TE buffer for 1 hr at 37°C. Then, 1 volume of 90% (v/v) redistilled phenol was added, the solution was mixed by inversion, centrifuged at 20,000 x g, and the aqueous phase, i.e., top phase, was transferred to a polyallomer centrifuge tube. Solid sodium acetate was then added to a concentration of 4.0% (w/v), the nucleic acids were precipitated with 2 volumes of ice cold ethanol, and incubated overnight at -20°C. Thereafter, the precipitate was collected by centrifugation at 16,000 rpm at 4°C in a Spinco SW25 rotor, dissolved in 2.0 ml of TE buffer, and dialyzed at 4°C against TE buffer.

The resulting DNA solution was then transferred to a polyallomer centrifuge tube and CsCl in TE buffer was added to 57% (w/v) (density = 1.715 g/cm$^2$). Next, the DNA was centrifuged for 46 hr at 22.5°C at 44,000 rpm in a Spinco No. 50 Ti rotor. Then, 12 drop fractions were collected from the bottom of the polyallomer tube and aliquots of 4.0 µl were counted in a liquid scintillation spectrometer to locate the IBRV DNA containing fractions (density = about 1.727 g/cm$^2$). When a total of 25 fractions were collected, generally fractions 13-15 contained the IBRV DNA.

The IBRV DNA-containing fractions were then pooled and dialyzed against several changes of TE buffer at 4°C for about 24 hr. The concentration of DNA was determined fluorometrically. The IBRV DNA yield was about 25 μg from $10^8$ cells.

The identity of IBRV DNA was verified by the pattern of restriction endonuclease-digested IBRV DNA fragments obtained after electrophoresis at 4°C in a submarine gel apparatus (Bethesda Research Laboratories, Inc.).

More specifically, the resulting DNA was digested with HindIII, BamHI, KpnI, SalI, under the reaction conditions recommended by the manufacturer (New England Biolabs, Inc.). Next, 1/10 volume of a solution comprising 0.4% (w/v) bromophenol blue, 125 mM EDTA, and 50% (v/v) glycerol was added to terminate the reaction, followed by heating at 65°C for 10 min. 20 μl of aliquots of each sample was applied into the sample wells of an agarose gel and electrophoresis was carried out as described below.

Electrophoresis of restriction endonuclease fragments was carried out on 0.6% (w/v) agarose slab gels (Kit et al, J. Med. Virol., 12:25-36 (1983)) in buffer comprising 30 mM $NaH_2PO_4$, 1.0 mM EDTA, and 40 mM Trizma base (pH 8.1) (hereinafter "electrophoresis buffer") at 45 volts, 4°C for 16 hr. After electrophoresis, DNA fragments were stained by soaking the gel in electrophoresis buffer containing 0.5 μg/ml of ethidium bromide, visualized over a longwave UV illuminator, and photographed.

The restriction patterns of the parental IBRV/(NG)dltk for HindIII, and SalI were not significantly distinct from that of the recombinant virus IBRV(PrGIII-β-gal). However, BamHI cleavage of IBRV(PrGIII-β-gal) revealed the appearance of a new 3.0 kb fragment; whereas an approximately 20 kb BamHI fragment of IBRV(NG)dltk was replaced by a fragment of about 18 kb. Measurements of fragments greater than 10 kb are rough approximations due to limitations of agarose gel electrophoresis. The KpnI cleavage of IBRV(PrGIII-β-gal) revealed that a 18 kb fragment present among KpnI fragments of IBRV(NG)dltk was replaced with an 21 kb fragment. These results for restriction nuclease digestion of IBRV(PrGIII-β-gal) were consistent with homologous recombination having occurred between IBRV(NG)dltk and pLA(PrGIII-β-gal).

## VIII. Construction of IBRV Expressing the bGH-FMDV Epitope as a gIII Fusion Protein: Derivation of IBVR-FMD Cl.1A

An IBRV insertion mutant expressing the bGH-FMDV epitope-gIII fusion protein from the gIII promoter was obtained by transfection of cells with DNA from both IBRV(PrGIII-β-gal) and plasmid pLAHK/cl.13.

### A. Transfection of IBRV(PrGIII-β-gal) and pLAHK/cl,13 DNA

Rab-9 cells were seeded in 60 mm Petri dishes (2.0 X $10^5$ cells per 5.0 ml of growth media per dish) and incubated at 37°C for 48 hr. Then, the following sterile solutions were added to a test tube in sequential order:
(1) 0.02 ml of 50 μg/ml of IBRV(PrGIII-β-gal) DNA in TE buffer;
(2) 0.02 ml of 10 μg/ml of plasmid pLAHK/cl.13 digested with KpnI and HindIII in 0.1 X TE buffer;
(3) 0.65 ml of water;
(4) 1.0 ml of 20 μg/ml of salmon sperm DNA in 2 X Hepes buffer solution; and
(5) 0.13 ml of 2.0 M $CaCl_2$.

The resulting solution was mixed by inversion and kept at room temperature for 30 min while a DNA-calcium phosphate precipitate formed. Then, 0.5 ml of the suspension was added directly to each Petri dish containing Rab-9 cells. The cells were incubated at 37°C for 4 hr. Then, the growth media was aspirated, and the dishes rinsed with 5.0 ml of fresh growth media. Next, 1.0 ml of 20% (v/v) glucose in 1 X Hepes buffer solution plus 10% (v/v) DMSO was added for 5 min at room temperature. The cells were rinsed with growth media, then 5.0 ml of fresh growth media was added, containing 0.5 X $10^6$ BK cells. The cultures were incubated at 37°C for 4 days until extensive cytopathic effects occurred. The cultures were frozen and stored at -80°C.

### B. Identification and Isolation of Recombinant Virus: Derivation of IBRV-FMD

Recombinant IBRV clones no longer expressing β-galactosidase were isolated as follows.

The progeny virus obtained from the transfection above mainly comprised parental IBRV(PrGIII-β-gal) and a small population of homologous recombinants in which the coding sequence of the β-gal gene was deleted and replaced by the bGH-FMDV-gIII fusion sequence. To identify and screen for recombinant viruses among parental viruses, an enzymatic colorometric plaque assay was utilized. In the assay for β-galactosidase produced by the recombinant virus-infected cells, the colorless substrate X-gal is converted to an insoluble colored product by β-galactosidase. The virus harvested from the cotransfection was serially diluted in growth media. Then, 0.1 ml of aliquots of the serial dilutions were added to the wells of a 6-well cluster tissue culture tray containing near confluent BK cells from which growth media had just been aspirated. The virus inoculum was absorbed to the cell monolayer for 1 hr at 37°C. The infected cell monolayer was overlaid with 2.5 ml per well of 1.25% (w/v) agar, McCoy's 5A solution, 0.04% (w/v) protamine sulfate, 2.0 mM glutamine, 50 µg/ml of neomycin, 10 mM Hepes (pH 7.2), and 50 units/ml of mycostatin plus 10% (v/v) fetal calf serum. The cultures were incubated for 5 days at 34.5°C, then overlaid with 2.5 ml per well of the above solution supplemented with 200 µg/ml of X-gal. After an overnight incubation at 34.5°C, colorless plaques were identified and picked with a Pasteur pipette. The agar plug was suspended in 1.0 ml of growth media, and vortexed briefly. Then, a six-ounce prescription bottle containing near confluent BK cells from which the growth media had been just decanted was inoculated with the isolated plaque. After 3 days at 34.5°C, when complete cytopathiceffects had occurred, the infected cell monolayer was frozen at -40°C, thawed and harvested. A second and third plaque purification was repeated as described above.

## C. Preparation of the IBRV-FMD DNA and Analysis of DNA Structure

Three plaque isolates from the titration of the harvested transfection of IBRV(PrGIII-β-gal) and pLAHK/cl.13 were propagated in six-ounce bottles of BK cells until complete cytopathic effects were present. These virus pools were used to prepare viral DNA as described above.

0.5 µg of viral DNA from the parental viruses IBRV(NG)dltk and IBRV(PrGIII-β-gal), and the three candidate recombinants, IBRV-FMD cl.1A, cl.2A, and cl.3A, were dissolved in 50 µl of BamHI, SalI, or SacI buffer. Then, 5 to 10 units of each enzyme was added to separate reactions, and the DNA digested for 1 hr at 37°C. The reactions were terminated by adding 1/10th volume of a solution comprising 0.4% (w/v) bromophenol blue, 125 mM EDTA, and 50% (v/v) glycerol, followed by heating at 65°C for 10 min. Then, 20 µl of aliquots of each sample was applied to the wells of an agarose gel, and electrophoresis carried out as described above. After electrophoresis, the separated DNA restriction fragments in the agarose gel were transferred to nitrocellulose filters (Schleicher and Schuell) in the following manner: The agarose gel was placed in a glass baking tray containing 1.0 M KOH for 30 min at room temperature and, then, in a glass baking tray containing 1.0 M Tris-HCl (pH 7.0) and 0.6 M NaCl for 60 min at room temperature. The treated gel was then transferred to a blot apparatus (Bethesda Research Laboratories).

A nitrocellulose filter was prewetted in water for 10 min and then 20 X SSC for 5 min. Next, the filter was placed on the gel. Using 20 X SSC as the transfer fluid, blotting was allowed to proceed for about 24 hr. The adherent gel was removed from the nitrocellulose filter and the filter was rinsed with 6 X SSC, dried at room temperature for several hours and then in a vacuum desiccator at 60°C overnight. This was followed by 2 hr of baking at 80°C. The nitrocellulose filters were removed from the desiccator and placed in Dazey Seal-A-Meal cooking bags (Southern, J. Mol. Biol., 98:503-513 (1975)).

The filter was first pretreated overnight at 60°C with 50 ml of modified Denhard's solution comprising 3 X SSC, 0.02% (w/v) polyvinylpyrollidone, 0.02% (w/v) Ficoll, 0.02% (w/v) BSA, 50 µg/ml of alkaline salmon sperm DNA, and 10 µg/ml of poly(A). The alkaline salmon sperm DNA was added from a stock solution of about 5.0 µg/ml prepared by dissolving 50 mg of salmon sperm DNA in 10 ml of 0.2 N NaOH, heating at 100°C for 20 min to denature and shear the DNA to about 0.4 kb segments, and then neutralizing with 0.2 ml of 10 N HCl.

The modified Denhard's solution was then replaced with 50 ml of hybridization buffer comprising 50% (v/v) formamide, 0.6 M NaCl, 0.2 M Tris-HCl (pH 8.0), 0.02 M EDTA, 0.1% (w/v) sodium dodecylsulfate, 50 µg/ml of alkaline salmon sperm DNA, and 10 µg/ml of poly(A). Next, air bubbles were squeezed out of the bag which was then sealed using an Oster Touch-A-Matic bag sealer, and incubated at 37°C for 1 hr on a shaker.

To prepare the probes, 2.0 µg of purified single-stranded DNA from M13mp18: bGH-FMDV cl. 11 was annealed with 5.0 ng of M13 hybridization probe primer in 30 µl of a buffer comprising 83 mM NaCl, 10 mM Tris-HCl (pH 7.5), 10 mM MgCl$_2$, and 20 mM dithiothreitol, at 65°C for 10 min, cooled to room temperature, and then the following substances were added: 20 µl each of gamma-$^{32}$P-dTTP and gamma-[32] P-dCTP (about 400 mCi/mmole, 10 mCi/ml in a stabilized aqueous solution, Amersham); 1.0 µl of 10 mM dATP; 1.0 µl of 10 mM dGTP; and 4 units of E. coli DNA polymerase, large fragment (Klenow enzyme, Bethesda Research Laboratories). The reaction mixture was incubated at room temperature for 2 hr, 10 µl of 0.25 M EDTA was added, and the mixture was heated at 65°C for 10 min. The labeled probe was purified by Sephadex G-50 column chromatography (0.5 x 15 cm). This procedure routinely yielded probes with a specific activity of more than $10^8$ cpm/µg of DNA.

Thereafter, about 1.0 ml, containing about $10^7$ cpm and 50 ng, of single-stranded [32] P-labelled DNA obtained as described above, was added to the bag with a 3.0 ml syringe by piercing the side of the bag at a corner. Next, the bag was resealed and incubated at 37°C for up to 48 hr on a shaker to allow for hybridization. After hybridization had been accomplished, the bag was cut and the solution was decanted. The filter was then carefully removed and placed into a tray containing about 100 ml of hybridization buffer containing 50 µg/ml of denatured salmon sperm DNA for the first wash only, but no poly(A) in any wash. The filter was washed for 30 min at 37°C five times with gentle shaking. Next, the filter was washed for 30 min at 37°C with 0.3 X SSC, and placed on filter paper to dry overnight at room temperature.

For autoradiography, the filter was replaced on a thin piece of cardboard covered with Saran-Wrap, and exposed to Kodak X-Omat XAR film with an intensifying screen for periods of 5 hr to 2 days at -70°C. The autoradiography of the gel probed with 'P-labeled M13mp18:bGH-FMDV cl.11 revealed that all three plaque isolates no longer had the hybridization pattern of the parental virus IBRV(PrGIII-β-gal), but, instead, had the hybridization pattern predicted by a recombination of the parental virus with the hybrid IBRV-FMDV sequences. That is, the probe did not hybridize to any of the restriction nuclease fragments of IBRV(NG)dltk. The probe did, however, hybridize to a 20 kb BamHI fragment, a 3.3 kb SalI fragment, and a 1.2 kb SacI fragment. These results were consistent with homologous recombination having occurred between IBRV(PrGIII-β-gal) and pLAHK/cl.13. IBRV-FMDV cl.1A was deposited at the American Type Culture Collection on February 23, 1989, under ATCC No. VR-2236.

## EXAMPLE 2

### Construction of IBRV-FMDV(Dimer)

#### I. Construction of a DNA Sequence for the FMDV Dimer Epitope Sequence and Cloning Into Phagescript

##### A. Synthesis Strategy

The following DNA sequence was chosen for synthesis:

```
BamHI cohesive end

219 →                                        221 →
GATCCACGCGTCTAGACACAAACAGAAAATTGTGGCACCGGTGAAACAGACTCTGCCCCCCTC
    GTGCGCAGATCTGTGTTTGTCTTTTAACACCGTGGCCACTTTGTCTGAGACGGGGGGAG
                                        ← 220


    223 →                                    225 →
CGTGCCCAACTTGAGAGGTGACCTTCAGGTGTTGGCTCAAAAGGTGGCACGGACGCCCGCCGG
GCACGGGTTGAACTCTCCACTGGAAGTCCACAACCGAGTTTTCCACCGTGCCTGCGGGCGGCC
    ← 222                                    ← 224

CTATGCATG
GATACGTACTTAA
        ← 226
            EcoRI cohesive end
```

The proximal end has a BamHI cohesive terminus, whereas the distal end has an EcoRI cohesive terminus to facilitate cloning of the assembled oligonucleotides into an intermediate M13-derived vector; i.e., Phagescript (Stratagene Cloning Systems, La Jolla, CA), suitable for rapid DNA sequencing of candidate recombinants.

##### B. Synthesis and Annealing of Oligonucleotides

The following oligonucleotides were synthesized and purified as described above.

```
34 mer  Oligo 219   5' - GATCCACGCGTCTAGACACAAACAGAAAATTGTG
```

```
35 mer  Oligo 220  5' - GGTGCCACAATTTTCTGTTTGTGTCTAGACGCGTG

34 mer  Oligo 221  5' - GCACCGGTGAAACAGACTCTGCCCCCCTCCGTGC

34 mer  Oligo 222  5' - GTTGGGCACGGAGGGGGGCAGAGTCTGTTTCACC

34 mer  Oligo 223  5' - CCAACTTGAGAGGTGACCTTCAGGTGTTGGCTCA

34 mer  Oligo 224  5' - CCTTTTGAGCCAACACCTGAAGGTCACCTCTCAA

34 mer  Oligo 225  5' - AAAGGTGGCACGGACGCCCGCCGGCTATGCATG

32 mer  Oligo 226  5' - AATTCATGCATAGCCGGCGGGCGTCCGTGCCA
```

All of the oligonucleotides, except Oligo 219 and Oligo 226, were phosphorylated as follows.

Oligo 219 and Oligo 226 were not 5'-phosphorylated to avoid self-ligation of the termini.

50 pmole of oligonucleotide was dissolved in 10 μl of a buffer comprising 70 mM Tris-HCl (pH 7.6), 10 mM MgCl$_2$, 5.0 mM dithiothreitol, and 30 μCi gamma-$^{32}$P-ATP (4,300 Ci/mmole). Then, 5 units of T4 polynucleotide kinase (New England Biolabs, Inc.) was added, and the reaction mixture was incubated for 15 min at 37°C. Next, the solution was brought to 0.7 mM ATP and the reaction continued for an additional 45 min. The reaction was terminated by heating to 70°C for 10 min, and NaCl was added to a final concentration of 50 mM. The reaction mixtures of the following pairs of oligonucleotides were combined:

```
(PK) Oligo 220 + Oligo 219 (No PK)     T_H = 40°C

(PK) Oligo 221 + (PK) Oligo 222        T_H = 48°C

(PK) Oligo 223 + (PK) Oligo 224        T_H = 43°C

(PK) Oligo 225 + Oligo 226 (No PK)     T_H = 48°C
```

The individual pairs of oligonucleotides were heated to 90°C for 3 min, then for 16 hr at the hybridization temperature which was 10°C below the $T_m$, calculated as described above. The annealed pairs of oligonucleotides were then stored at 4°C for 2 hr prior to use. The completeness of the annealing process was monitored by mobility changes on nondenaturing 8.0% (w/v) polyacrylamide gels as described above.

C. Cloning of Assembled Oligonucleotides: Derivation of PS:FMDV

The FMDV epitope DNA sequence was assembled and cloned into the BamHI to EcoRI sites of Phagescript DNA during a single step in vitro ligation from pairs of annealed oligonucleotides duplexes with 5 bp overlapping cohesive ends as follows (see Figure 6).

2.0 μg of Phagescript DNA was digested with 20 units of BamHI in 120 μl of buffer comprising 100 mM potassium glutamate, 25 mM Tris-acetate (pH 7.6), 10 mM magnesium acetate, 0.5 mM 2-mercaptoethanol, and 50 μg/ml of BSA at 37°C for 1 hr. The BamHI-digested DNA was then digested with 20 units of EcoRI added directly to the reaction mixture and incubated at 37°C for 1 hr.

The reaction was terminated by adding Proteinase K to a final concentration of 100 μg/ml and incubating at 37°C for 1 hr. An equal volume of 90% (v/v) phenol was added, the reaction mixture vortexed, and phases separated by centrifugation. The aqueous phase was removed, extracted five times with water-saturated ether, then dialyzed against four changes of 500 ml of 0.1 X TE buffer at 4°C over a 2-day period. The DNA was precipitated by the addition of an equal volume of 7.5 M ammonium acetate and 2 volumes of ethanol. The precipitated DNA was collected by centrifugation as described above and air dried.

2.0 μg of the EcoRI- and BamHI-digested Phagescript DNA was combined with 2.5 pmole of each of the four pairs of annealed oligonucleotides in 50 μl of ligation buffer and ligated by adding 800 units of T4 DNA ligase and incubating at 4°C overnight. The reaction was terminated by adding 160 μl of 1 X TE buffer. Competent E. coli K12 strain JM103 were transformed by aliquots of the ligation mixture as described above.

Twenty-four colorless plaques were picked with toothpicks and single-stranded DNA prepared as described above for M13mp18 recombinants. The sequences of cloned DNA was determined by Sanger's chain termination method. A clone was identified containing the intended sequence inserted as expected. The clone was designated PS:FMDV (see Figure 6)

## II. Construction of a Plasmid Containing the bGH-FMDV(Dimer) Epitope as the N-Terminal Sequence of the IBRV gIII Protein: Derivation of pGIIIFM1(Dimer)

The FMDV epitope DNA sequence was inserted in phase between the bGH-FMDV and gIII DNA sequence of pGIIIFM1 to produce a plasmid containing the bGH-FMDV-FMDV-gIII sequence as shown in Figure 6. The FMDV epitope DNA sequence was excised from RF DNA of PS:FMDV as a 0.13 kb MluI-NsiI fragment and ligated into the MluI-NsiI site of pGIIIFM1 at 2.6 map units.

More specifically, 2.0 µg of pGIIIFM1 plasmid DNA and 2.0 µg of PS:FMDV RF DNA were separately dissolved in 100 µl of MluI cutting buffer. The DNA was digested with 12 units of MluI enzyme for 2 hr at 37°C. The reaction was terminated and the DNA ethanol precipitated and collected by centrifugation as described above.

The MluI-digested pGIIIFM1 and MluI-digested PS:FMDV DNA were separately dissolved in 100 µl of NsiI cutting buffer. Each of the plasmids was digested with 10 units of NsiI enzyme for 2 hr at 37°C. The reaction was terminated after incubation for 1 hr with 100 µg/ml of Proteinase K at 37°C, then by vortexing with an equal volume of phenol. After phase separation by centrifugation, the aqueous phase containing the DNA was extracted with ether, then dialyzed exhaustively against 0.1 X TE buffer. The plasmid DNA was brought to 0.3 M sodium acetate and precipitated by 2 volumes of ethanol. The DNA was collected by centrifugation as described above.

The MluI- and NsiI-digested pGIIIFM1 and PS:FMDV DNA was dissolved in ligation buffer and ligated by 800 units of T4 DNA ligase for 16 hr at 4°C. Aliquots of the reaction mixture were used to transform E. coli K12 strain XL-1 Blue as described above. A clone was identified with a 0.12 kb insertion and designated pGIIIFM1(Dimer) after DNA sequence confirmation (see Figure 6). The compiled sequence is shown in Table 5 below.

## Table 5
## Compiled Sequence of pGIIIFM1(Dimer)

CTGCAGCCGCGCGTGTGCTCAATCCCGGACCACGAAAGCACAAAACGGACGCCCTTAAAAATG

TAGCCCGCGCGCGGTCGCGGCCATCTTGGATCCACCCGCGCGCACGACCGCCGAGAGACCGCC

```
                                       bGH signal →
AGCCCGAGACCTCGCCGCGCGTCCGCC MET MET ALA ALA GLY PRO ARG THR SER
                            ATG ATG GCT GCA GGC CCC CGG ACC TCC


LEU LEU LEU ALA PHE ALA LEU LEU CYS LEU PRO TRP THR GLN VAL VAL
CTG CTC CTG GCT TTC GCC CTG CTC TGC CTG CCC TGG ACT CAG GTG GTG


      FMDV epitope (O₁K amino acids 200-213) →
GLY ALA ARG HIS LYS GLN LYS ILE VAL ALA PRO VAL LYS GLN THR LEU
GGC GCC AGA CAC AAA CAG AAA ATT GTG GCA CCG GTG AAA CAG ACT CTG


    spacer     FMDV epitope (O₁K amino acids 141-158) →
PRO PRO SER VAL PRO ASN LEU ARG GLY ASP LEU GLN VAL LEU ALA GLN
CCC CCC TCC GTG CCC AAC TTG AGA GGT GAC CTT CAG GTG TTG GCT CAA


                                           FMDV epitope
                                           O₁K amino acids
                         spacer             200-213) →
LYS VAL ALA ARG THR PRO ALA GLY GLY HIS ALA SER ARG HIS LYS GLN
AAG GTG GCA CGG ACG CCC GCC GGC GGC CAC GCG TCT AGA CAC AAA CAG


                                           FMDV epitope
                                           (O₁K amino
                                           acids
                                 spacer     141-158) →
LYS ILE VAL ALA PRO VAL LYS GLN THR LEU PRO PRO SER VAL PRO ASN
AAA ATT GTG GCA CCG GTG AAA CAG ACT CTG CCC CCC TCC GTG CCC AAC


LEU ARG GLY ASP LEU GLN VAL LEU ALA GLN LYS VAL ALA ARG THR PRO
TTG AGA GGT GAC CTT CAG GTG TTG GCT CAA AAG GTG GCA CGG ACG CCC


spacer                        gIII →
ALA GLY TYR ALA SER THR MET GLY PRO LEU GLY ARG ALA TRP LEU ILE
GCC GGC TAT GCA TCT ACC ATG GGC CCG CTG GGG CGA GCG TGG CTG ATC


ALA ALA ILE PHE ALA TRP ALA LEU LEU SER ALA ARG ARG GLY LEU ALA
GCA GCT ATT TTC GCC TGG GCG CTC CTG TCT GCC CGG CGG GGG CTC GCC


GLU GLU ALA GLU ALA SER PRO SER PRO PRO PRO SER PRO SER PRO THR
GAG GAG GCG GAA GCC TCG CCC TCG CCT CCG CCC TCC CCG TCC CCA ACC
```

Table 5 (Continued)

```
GLU THR GLU SER SER ALA GLY THR THR GLY ALA THR PRO PRO THR PRO
GAG ACG GAA AGC TCC GCT GGG ACC ACC GGC GCA ACG CCC CCC ACG CCC

ASN SER PRO ASP ALA THR PRO GLU ASP SER THR PRO GLY ALA THR THR
AAC AGC CCC GAC GCT ACG CCA GAG GAC AGC ACG CCC GGT GCT ACT ACG

PRO VAL GLY THR PRO GLU PRO PRO SER VAL SER GLU HIS ASP PRO PRO
CCC GTG GGG ACG CCG GAG CCG CCG TCC GTG TCC GAG CAC GAC CCG CCC

. . .
```

III. Construction of a Deletion Derivative of pGIIIFM1(Dimer): Derivation of pGIIIFM1(Dimer) Del-21

In plasmid pGIIIFM1(Dimer), the bGH-FMDV-FMDV epitope sequence is fused with the IBRV gIII coding sequence as shown in Table 5 above. Since the signal peptide of the fusion protein is provided by the bGH gene, and since the signal peptide coded by the IBRV gIII gene may interfere with membrane translocation, the DNA sequence coding for the IBRV signal peptide was deleted. An oligonucleotide mediated deletion method, modified from that of Su, Gene, 69:81-89 (1988) was used to delete 21 amino acids of the IBRV gIII signal peptide sequence, as follows.

A. Preparation of Single-stranded Circular DNA of pGIIIFM1(Dimer)

E. coli K12 XL-1 Blue containing plasmid pGIIIFM1(Dimer) from a trypticase soy agar slant was used to inoculated 5.0 ml of ML media containing 50 µg/ml of ampicillin and 10 µg/ml of tetracycline. The next day, 50 ml of ML media containing 50 µg/ml of ampicillin and 0.001% (w/v) thiamine was inoculated with 1.0 O.D.$_{600}$ of the overnight culture. The fresh culture was incubated at 37°C with shaking until the O.D.$_{600}$ was about 0.2 O.D., or 5.0 x 10$^7$ bacteria/ml. Into a fresh tube were added 0. 5 ml of the above culture and 1.0 ml of pre-warmed M13K07 helper phage (titer: 5.0 x 10$^8$ p.f.u./ml) at an m.o.i. of 20. The phage was allowed to absorb to the bacteria at 37°C for 30 min with gentle mixing., The infected culture was used to inoculate 50 ml of Circle Grow™ media (Bio 101, Inc.) containing 70 µg/ml of kanamycin and 50 µg/ml of ampicillin, and 0.001% (w/v) thiamine. The culture was incubated at 37°C with shaking for 24 to 48 hr until a dense culture was present.

The bacterial cells were pelleted in a Sorvall GSA rotor at 8,000 rpm for 30 min at 4°C. The supernatant containing the phage was precipitated by adding one-fourth volume of 20% (w/v) PEG, 3.5 M ammonium acetate solution, and incubating on ice for 30 min. The precipitated phage was pelleted in a GSA rotor at 9,000 rpm for 15 min at 4°C. The pellet was thoroughly drained, then resuspended in 5.0 ml of 1 X TE buffer. The sample was vortexed for 2 min with one-half volume of 90% (v/v) phenol, then vortexed a further 2 min after adding 2.5 ml of chloroform. The phases were separated by centrifuging in a Sorvall SS-34 rotor at 15,000 rpm for 15 min at room temperature. The aqueous phase was removed, and the extraction repeated as above. The DNA was precipitated by adding one-half volume of 7.5 M ammonium acetate and 2 volumes of ethanol. After 1 hr at -20°C, the DNA precipitate was collected by centrifugation in a Sorvall SS-34 rotor at 16,000 rpm for 20 min at 4°C. The DNA was resuspended in distilled water, then again precipitated and collected as described above. The DNA pellet was rinsed twice with ice cold 85% (v/v) ethanol, air dried, and then redissolved in 0.3 ml of distilled water.

B. Oligonucleotide Mutagenesis

The following 28-mer oligonucleotide was synthesized by CE-phosphoramidite chemistry on a Systec 1450 automated DNA synthesizer according to the manufacturer's instructions, then purified by polyacrylamide gel electrophoresis.

5' - GGCTATGCATCTACC CGGCGGGGGCTCG - 3'

"Clamp Oligonucleotide"

The first 15 nucleotides correspond to the sequence coding for the five amino acids preceding the start codon of the IBRV gIII signal peptide, whereas the remaining 13 nucleotides correspond to the sequence coding for amino acids 22 through 25 of the IBRV gIII protein. The absent intervening sequence codes for the first 21 amino acids of the IBRV gIII gene containing all but the last amino acid of the predicted IBRV gIII signal peptide. The length of the arms of the oligonucleotide clamp were chosen to have a $T_H$ of 43°C to 47°C, according to the formula:

$$T_H = 2°C (A + T) + 4°C (C + G) - 3°C$$

50 pmoles of oligonucleotide was phosphorylated in 25 μl of a buffer comprising 70 mM Tris-HCl (pH 7.6), 10 mM $MgCl_2$, 5.0 mM dithiothreitol, 1.0 mM ATP, and 10 units of T4 polynucleotide kinase (New England Biolabs, Inc.) at 37°C for 30 min, then heat inactivated at 70°C for 10 min.

Next, 0.66 pmole of the phosphorylated oligonucleotide was added to 0.2 pmole of pGIIIFM(Dimer) single-stranded DNA (0.9 μg) in 10 μl of a buffer comprising 40 mM Tris-HCl (pH 7.5), 20 mM $MgCl_2$, and 50 mM NaCl (hereinafter "T7 DNA pol buffer"), and annealed at 65°C for 5 min followed by slow cooling to 4°C.

The oligonucleotide was extended and the new strand sealed by adding 1.0 μl of T7 DNA polymerase (14 units Sequenase; U.S. Biochemical), 1.0 μl of T4 DNA ligase (400 units; New England Biolabs, Inc.), 1.5 μl of 10 mM ATP, and 2.0 μl of a mixture of 0.5 mM dATP, 0.5 mM dGTP, 0.5 mM dCTP, 0.5 mM TTP, and 50 μM dithiothreitol in T7 DNA pol buffer, then incubating at 37°C for 15 min followed by heat inactivation at 65°C for 5 min.

The heteroduplex DNA was separated from parental single-stranded pGIIIFM1(Dimer) DNA by absorption of the latter onto nitrocellulose filter as follows.

To the above reaction were added 205 μl of water and 30 μl of 5.0 M NaCl. The sample was applied to a Centrex™ spin filter (Shleicher & Schuell), which was centrifuged at 500 x g for 10 min at room temperature. The nitrocellulose filter was rinsed with 100 μl of 0.5 M NaCl, and the above step repeated. Then, a 20 μl aliquot of the eluate was used to transform $CaCl_2$-treated E. coli XL-1 Blue as described above. Transformants were picked and minipreps of plasmid DNA prepared and analyzed for loss of the NcoI site present in the sequence targeted for deletion. About 40% of the transformants had lost the NcoI site. Single-stranded DNA was prepared from several clones and sequenced as described above. The results verified the precise deletion of the sequence coding for the first 21 amino acids of the signal peptide of the IBRV gIII protein. Table 6 below shows the compiled sequence in the modified region of pGIIIFM1(Dimer).

## Table 6
### Compiled Sequence of pGIIIFM1(Dimer)-Del 21

CTGCAGCCGCGCGTGTGCTCAATCCCGGACCACGAAAGCACAAAACGGACGCCCTTAAAAATG

Oligo 315

TAGCCCGCGCGCGGTCGCGGCCATCTTGGATCCACCCGCGCGCACGACCGCC<u>GAGAGACCGCC</u>

bGH signal →
<u>AGCCCGAGACCTC</u>GCCGCGCGTCCGCC MET MET ALA ALA GLY PRO ARG THR SER
                           ATG ATG GCT GCA GGC CCC CGG ACC TCC

LEU LEU LEU ALA PHE ALA LEU LEU CYS LEU PRO TRP THR GLN VAL VAL
CTG CTC CTG GCT TTC GCC CTG CTC TGC CTG CCC TGG ACT CAG GTG GTG

FMDV epitope ($O_1K$ amino acids 200-213) →
GLY ALA ARG HIS LYS GLN LYS ILE VAL ALA PRO VAL LYS GLN THR LEU
GGC GCC AGA CAC AAA CAG AAA ATT GTG GCA CCG GTG AAA CAG ACT CTG

<u>spacer</u> FMDV epitope ($O_1K$ amino acids 141-158) →
PRO PRO SER VAL PRO ASN LEU ARG GLY ASP LEU GLN VAL LEU ALA GLN
CCC CCC TCC GTG CCC AAC TTG AGA GGT GAC CTT CAG GTG TTG GCT CAA

                                          FMDV epitope
                                          ($O_1K$ amino acids
                      <u>spacer</u>        200-213) →
LYS VAL ALA ARG THR PRO ALA GLY GLY HIS ALA SER ARG HIS LYS GLN
AAG GTG GCA CGG ACG CCC GCC GGC GGC CAC GCG TCT AGA CAC AAA CAG

                                          FMDV epitope
                                          ($O_1K$ amino acids
                                 <u>spacer</u>    141-158) →
LYS ILE VAL ALA PRO VAL LYS GLN THR LEU PRO PRO SER VAL PRO ASN
AAA ATT GTG GCA CCG GTG AAA CAG ACT CTG CCC CCC TCC GTG CCC AAC

LEU ARG GLY ASP LEU GLN VAL LEU ALA GLN LYS VAL ALA ARG THR PRO
TTG AGA GGT GAC CTT CAG GTG TTG GCT CAA AAG GTG GCA CGG ACG CCC

<u>spacer</u>               amino acid 22 of gIII →
ALA GLY TYR ALA SER THR ARG ARG GLY LEU ALA GLU GLU ALA GLU ALA
GCC GGC TAT GCA TCT ACC CGG CGG GGG CTC GCC GAG GAG GCG GAA GCC

SER PRO SER PRO PRO PRO SER PRO SER PRO THR GLU THR GLU SER SER
TCG CCC TCG CCT CCG CCC TCC CCG TCC CCA ACC GAG ACG GAA AGC TCC
           Oligo 316   <u>AGG GGT TGG CTC TGC CTT TCG AGG</u>

ALA GLY THR THR GLY ALA THR PRO PRO THR PRO ASN SER PRO ASP ALA
GCT GGG ACC ACC GGC GCA ACG CCC CCC ACG CCC AAC AGC CCC GAC GCT

<u>Table 6</u> (Continued)

```
THR PRO GLU ASP SER THR PRO GLY ALA THR THR PRO VAL GLY THR PRO
ACG CCA GAG GAC AGC ACG CCC GGT GCT ACT ACG CCC GTG GGG ACG CCG

GLU PRO PRO SER VAL SER GLU HIS ASP PRO PRO VAL THR ASN SER THR
GAG CCG CCG TCC GTG TCC GAG CAC GAC CCG CCC GTT ACC AAC AGC ACG

PRO PRO PRO ALA PRO PRO GLU ASP GLY ARG PRO GLY GLY ALA GLY ASN
CCG CCG CCC GCC CCG CCC GAG GAC GGG CGA CCC GGC GGC GCT GGC AAC

. . .
```

## IV. Construction of IBRV Expressing the bGH-FMDV(Dimer)-GIII Fusion Sequence

An IBRV-based viral vector expressing the bGH-FMDV(Dimer) epitope-gIII fusion protein from the IBRV gIII promoter was obtained by transfection of cells with DNA from both IBRV(PrGIII-β-gal) and pGIIIFM1(Dimer)-Del 21.

### A. Transfection of IBRV(PrGIII-β-gal) and pGIIIFM1(Dimer)-Del 21 DNA

Rab-9 cells were seeded in 60-mm Petri dishes (2.0 x 10⁵ cells per 5.0 ml of growth media per dish) and incubated at 37°C for 48 hr. Then, the following sterile solutions were added to a test tube in sequential order:
(1) 0.02 ml of 50 μg/ml of IBRV(PrGIII-β-gal) DNA in TE buffer;
(2) 0.2 ml of 10 μg/ml of plasmid pGIIIFM1(Dimer)-Del 21 in 0.1 X TE buffer;
(3) 0.65 ml of water;
(4) 1.0 ml of 20 μg/ml of salmon sperm DNA in 2 X Hepes buffer solution; and
(5) 0.13 ml of 2.0 M CaCl₂.
The resulting solution was mixed by inversion and kept at room temperature for 30 min while a DNA-calcium phosphate precipitate formed. Then, 0.5 ml of the suspension was added directly to each Petri dish containing Rab-9 cells. The cells were incubated at 37°C for 4 hr. Next, the growth media was aspirated, and the dishes rinsed with 5.0 ml of fresh growth media. Then, 1.0 ml of 20% (w/v) glucose in 1 X Hepes buffer solution plus 10% (v/v) DMSO was added for 5 min at room temperature. The cells were rinsed with growth media, then 5.0 ml of fresh growth media was added, containing 0.5 x 10⁶ BK cells. The cultures were incubated at 37°C for 5 days until extensive cytopathic effects occurred. The cultures were frozen and stored at -80°C.

### B. Identification and Isolation of Recombinant Virus: Derivation of IBRV-FMDV(Dimer)

Recombinant IBRV clones no longer expressing β-galactosidase were isolated as follows.
The progeny virus obtained from the transfection above mainly comprised parental IBRV(PrGIII-β-gal) and a small population of homologous recombinants in which the coding sequence of the β-gal gene was deleted and replaced by the bGH-FMDV(Dimer)-gIII fusion sequence. To identify and screen for recombinant viruses among parental viruses, an enzymatic colorometric plaque assay was utilized as described above. The virus harvested from the co-transfection was serially diluted in growth media and titrated in BK cells as described above. The infected monolayers were overlaid with X-gal, and after an overnight incubation at 34°C, three colorless plaques were identified (designated IBRV-FMD(Dimer) cl. 1 or 2 or 3), picked with a Pasteur pipette and propagated in BK cells as described above. Clones 1 and 3 were plaque purified a second time, and new virus pools prepared as described above.

### C. Preparation of IBRV-FMD(Dimer) DNA Structure

Candidate recombinants, IBRV-FMD(Dimer) cl.1 and IBRV-FMD(Dimer) cl.3, were used to prepare viral DNA for further analysis according to the method described above. To confirm the DNA sequence of the recombinant viruses, the DNAs were subjected to enzymatic amplification by polymerase chain reaction (hereinafter "PCR"). Amplified samples were subjected to sequencing with DNA polymerase from <u>Thermus aquaticus</u> (Taq polymerase) and terminal labeled oligonucleotides primers.

## 1. Enzymatic amplification of IBRV-FMD(Dimer) DNA by the polymerase chain reaction (PCR)

The positions of the PCR primers is shown in Table 6. Before PCR, 40 ng of IBRV-FMD(Dimer) cl.1 DNA or IBRV-FMD(Dimer) cl.3 DNA in 10 μl of a buffer comprising 10 mM Tris-HCl (pH 8.0), 1.0 mM EDTA (pH 8.0), and 10 mM NaCl was denatured in boiling water for 10 min just before PCR. IBRV-FMD(Dimer) cl.1 and IBRV-FMD(Dimer) cl.3 were subjected to amplification with 2.5 units of Taq polymerase. The reaction mixture, including 50 pmole of Oligo primer 315 (5' - GAGAGACCGCCAGCCCGAGACCTC), 50 pmole of Oligo primer 316 (5' - GGAGCTTTCCGTCTCGGTTGGGGA) and 1.25 mM dNTPs mix was subjected to repeated cycles of 1 min at 94°C (denaturing), 2 min at 45°C (annealing) and 3 min at 72°C (extension) 25 times using a Perkin-Elmer Cetus DNA thermocycler. Each sample was overlaid with 100 μl of mineral oil (Perkin-Elmer No. 186-2302) in a 0.5 ml capped polypropylene tube. The PCR product was extracted with 100 μl of chloroform. The aqueous phase, containing the sample, was collected.

## 2. Purification of PCR Products by Microconcentrator (Centricon-100™)

To purify the PCR products (cl.1 and cl.3) from contaminating primers, the samples were filtered through Centricon-100™ (Amicon). Each sample was added to the sample reservoir (90 μl of sample + 910 μl of 1 X TE buffer), and was spun at 3,000 rpm (1,000 x g) for 30 min (2 times). Then, the retentate cup was placed over the sample reservoir. The unit was inverted and centrifuged at 3,000 rpm (1,000 x g) for 5 min to transfer concentrate into retentate cup. Aliquots of the purified PCR product were electrophoresed through a 0.8% (w/v) agarose for 16 hrs at 35 volts.

## 3. Terminal Labeling of Oligonucleotide Primers with gamma-$^{32}$P-dATP

Oligo primer 315 and Oligo primer 316 were labeled with gamma-$^{32}$P-dATP using T4 polynucleotide kinase. The reactive mixture comprising 50 pmol of oligonucleotide, 17 μl of gamma-$^{32}$P-dATP, and 10 units of T4 polynucleotide kinase in polynucleotide kinase buffer was incubated for 30 min at 37°C. Labeled oligonucleotides were purified through P4 gel filtration media (Bio-Rad Laboratories) using elution buffer comprising 10 mM Tris (pH 7.6), 10 mM NaCl, and 2.0 mM EDTA.

## 4. Sequencing IBRV-FMD(Dimer) PCR Products with Taq Polymerase

Before sequencing, the mixture of 17 μl of PCR product and 100 μl of $^{32}$ P-labeled primer was heated in boiling water for 10 min and immediately cooled on ice. The following four sets of annealing mixture were made:

    cl.1 + Oligo primer 315
    cl.1 + Oligo primer 316
    cl.3 + Oligo primer 315
    cl.3 + Oligo primer 316

Each set of annealing mixtures contained 17 μl of PCR product plus 100 μl of the oligo primer and 11 μl of 10 X Hin buffer comprising 60 mM Tris (pH 7.5), 60 mM MgCl$_2$, 10 mM DTT and 0.5 M NaCl. The mixture was incubated at 45°C for 30 min and cooled to room temperature. After annealing, 260 μl of ethanol were added to each sample. The samples were kept at -20°C for 30 min, then spun down for 5 min. The precipitates were rinsed with 200 μl of ethanol and dried <u>in vacuo.</u> 3.0 μl of 10 X Taq polymerase buffer comprising 0.5 M KCl, 15 mM MgCl$_2$, 0.1% (w/v) gelatin and 0.1 M Tris (pH 8.3), and 7.0 μl of water were added to the dried precipitate. To the 10 μl annealing reaction mixture were added 2.0 μl of labeling mix comprising 10 μM dGTP, 5.0 μM dCTP, 5.0 μM TTP in 10 mM Tris-HCl (pH 8.0), 6.0 μl of water and 2.0 μl of Taq polymerase (5 units/μl). To 2.0 μl of the appropriate termination mix comprising 30 μM dNTP, 0.25-1.5 mM ddNTP, and 0.37-1.62 mM MgCl$_2$ in separate tubes, were added 4.0 μl of the labeling reaction, followed by incubation at 70°C for 3 min. The reaction was stopped by adding 4.0 μl of 95% (v/v) formamide, 20 mM EDTA, 0.05% (w/v) xylene cyanol and 0.05% (w/v) bromophenol blue. Before loading, the samples were heated at 80°C for 2 min. 2.0 μl of the samples were loaded into wells on a 8.0% (w/v) polyacylamide sequencing gel, then electrophoresed at 55°C for 2.5 hrs, at 2,800 volts. The sequence of IBRV(Dimer) cl.1 and cl.3 was confirmed as expected from recombination of pGIIIFM1(Dimer)-Del-21 and IBRV(PrGIII-β-gal). IBRV-FMD(Dimer) cl.3 has been deposited at the American Type Culture Collection on May 16, 1990, under ATCC No. VR-2271.

EXAMPLE 3

### 1. Immunochemical Reactivity of IBRV-FMD and IBRV-FMDV(Dimer) with Anti-FMDV Peptide

### A. Plaque Immunoassay

#### 1. IBRV-FMDV cl.1A

In situ immunostaining of virus plaques of the IBRV-FMD cl.1A was performed as follows.

Monolayers of BK cells in 24-well tissue culture trays (Falcon No. 3047) were infected with about 10 p.f.u. of virus per well in a 0.05 ml inoculum volume. After absorption for 1 hr at 34.5°C, the infected cells were overlaid with 0.7 ml per well of 1.0% (w/v) methylcellulose in growth medium, and the trays incubated at 34.5°C for 5 days.

The overlay was aspirated and the wells rinsed with 1 X PBS. Then, the infected cells were fixed with 0.25% (v/v) glutaraldehyde in 1 X PBS for 5 min at room temperature or with methanol for 10 min at room temperature. The fixative was aspirated, and the wells rinsed twice with 1 X PBS, then once with 1 X PBS containing 3.0% (w/v) BSA for 5 min at room temperature. Next, the solution was replaced with 0.25 ml per well of the primary antibody diluted 1:100 for the bovine and guinea pig anti-FMDV peptide sera and 1:200 for the IBRV gIII monoclonal antibody, in 1 X PBS containing 3.0% (w/v) BSA. The antibody was absorbed for 2 hr at room temperature, and removed by three rinses with 1 X PBS. 0.25 ml per well of secondary antibody-alkaline phosphatase conjugates (Kirkegaard and Perry Laboratories) at 2.5 μg/ml in 1 X PBS containing 3.0% (w/v) BSA were absorbed for 2 hr at room temperature, and the unbound fraction removed by three rinses with 1 X PBS. Then, 0.25 ml per well of alkaline phosphate reagent comprising 6 volumes of 0.1 M Tris (pH 9.5), 0.75 volumes of bromochloroindolyl phosphate concentrate solution and 0.75 volumes of nitroblue tetrazolium concentrate solution (Kirkegaard and Perry Laboratories) was added, and the plates incubated for 10 min at room temperature in subdued light. The plates were rinsed with water and dried.

The results demonstrated that IBRV-FMDV cl.1A reacted specifically with either bovine of guinea pig anti-FMDV peptide sera and the IBRV gIII monoclonal antibody, whereas the IBRV(NG)dltk virus, which has only a deletion in the IBRV tk gene and no inserts of FMDV DNA sequences, failed to react with the anti-FMDV peptide sera, but did react, as expected, with the IBRV gIII monoclonal antibody.

#### 2. IBRV(Dimer) cl.1, cl.2 and cl.3

In situ immunostaining of virus plaques of the IBRV-FMD(Dimer) cl.1, cl.2 and cl.3 was performed as follows.

Monolayers of BK cells in 24-well tissue culture trays (Falcon No. 3047) were infected with about 10 p.f.u. of virus per well in a 0.05 ml inoculum volume. After absorption for 1 hr at 34.5°C, the infected cells were overlaid with 0.7 ml per well of 1.0% (w/v) methylcellulose in growth medium, and the trays incubated at 34.5°C for 5 days.

The overlay was aspirated and the wells rinsed with 1 X PBS. Then, the infected cells were fixed with 0.25% (v/v) glutaraldehyde in 1 X PBS for 5 min at room temperature or with methanol for 10 min at room temperature. The fixative was aspirated, and the wells rinsed twice with 1 X PBS, then once with 1 X PBS containing 3.0% (w/v) BSA for 5 min at room temperature. Then, the solution was replaced with 0.25 ml per well of the primary antibody diluted 1:100 for the bovine and guinea pig anti-FMDV peptide sera and 1:200 for the IBRV gIII monoclonal antibody, in 1 X PBS containing 3.0% (w/v) BSA. The antibody was absorbed for 2 hr at room temperature, and removed by three rinses with 1 X PBS. 0.25 ml per well of secondary antibody-alkaline phosphatase conjugates (Kirkegaard and Perry Laboratories) at 2.5 μg/ml in PBS containing 3.0% (w/v) BSA were absorbed for 2 hr at room temperature, and the unbound fraction removed by three rinses with 1 X PBS. Then, 0.25 ml per well of alkaline phosphate reagent (Kirkegaard and Perry Laboratories) was added, and the plates incubated for 10 min at room temperature in subdued light. The plates were rinsed with water and dried.

The results demonstrated that both IBRV-FMD(Dimer) cl.1 and cl.3 reacted specifically with either bovine or guinea pig anti-FMDV peptide sera and the IBRV gIII monoclonal antibody, whereas IBRV(NG)dltk, which has only a deletion in the IBRV tk gene and no inserts of FMDV DNA sequences, failed to react with the anti-FMDV peptide sera, but did react, as expected, with the IBRV gIII monoclonal antibody.

### B. Immunogold Staining of Permeabilized and Non-permeabilized Infected Cells

Immunocytochemical staining of permeabilized versus non-permeabilized infected cells demonstrates the cytological locations of antigens. Surface membrane proteins can react with antibodies in non-permeabilized cells, whereas intracellular antigens remain inaccessible. Hence, staining of infected cells was carried out.

BK cells were seeded and grown to confluency in 8-well Lab-Tek slides (Miles Laboratories), then infected with variable multiplicities of IBRV-FMD cl.1A or IBRV(NG)dltk. After incubation for 16 hr at 34.5°C, the infected cell monolayers were processed as two groups: (a) permeabilized or (b) non-permeabilized. The former group was first rinsed 3 times for 5 min at room temperature with 1 X PBS⁻ (with no $Mg^{++}$ or $Ca^{++}$ salts), then fixed in ice cold 95% (v/v) ethanol-5.0% (v/v) glacial acetic acid for 3 min at room temperature. Thereafter, both groups were processed the same, with all reagent incubations held at room temperature. All PBS solutions were without heavy metal salts.

The wells were rinsed 3 times for 5 min with PBS containing 1.0% (w/v) BSA and 0.02 M sodium azide. The secondary antibody, biotin-labeled anti-guinea pig IgG or biotin-labeled anti-mouse IgG, was diluted 1:200 in PBS containing 5.0% (w/v) BSA and 0.02 M sodium azide, and 0.1 ml per well was absorbed for 30 min. The wells were rinsed 3 times for 5 min with PBS containing 1.0% (w/v) BSA and 0.02 M sodium azide. Then, 0.1 ml/well of gold-streptavidin (Janseen Laboratories) diluted 1:100 in PBS containing 0.1% (w/v) BSA and 0.02 M sodium azide was absorbed for 1 hr. Next, the wells were rinsed 3 times for 5 min with PBS containing 1.0% (w/v) BSA and 0.02 M sodium azide. Next, fixed for 2 min with 4.0% (v/v) formaldehyde in absolute ethanol. The wells were rinsed 3.0 times for 5 min with distilled water. Then, several drops per slide of silver developing reagent IntenSe-M (Janseen Laboratories) were added and held for 15 min. Next, the slides were rinsed with water, dried, and mounted under buffered 50% (v/v) glycerin (pH 7.0).

The results demonstrated that, in the non-permeabilized group, there was abundant circumferential staining of the IBRV-FMD cl.1A-infected cells with the guinea pig anti-FMDV peptide sera, but no staining with the normal guinea pig sera, nor staining of the IBRV(NG)dltk-infected cells with either sera. In the non-permeabilized group, the IBRV gIII monoclonal antibody circumferentially stained both the IBRV-FMD cl.1A- and IBRV(NG)dltk-infected cells. In the permeabilized cell group, there was circumferential and concentric circumferential staining plus cytoplasmic staining of the IBRV-FMD cl.1A-infected cells with the guinea pig anti-FMDV peptide sera, but no staining with the normal guinea pig sera, nor staining of the IBRV(NG)dltk-infected cells with either sera. In the permeabilized group, the IBRV gIII monoclonal antibody similarly stained both the IBRV-FMDV cl.1A- and IBRV(NG)dltk-infected cells, as described for the anti-FMDV sera.

The circumferential pattern of staining non-permeabilized cells was characteristic of cell surface labeling of antigen.

## C. Virus Neutralization Studies

The capability of anti-FMDV peptide sera to neutralize infectivity of IBRV-FMD cl.1A selectively would be functional evidence of localization of the antigen on the surface of virus particles. Hence, virus neutralization studies were performed as follows.

BK cells were grown to confluency in 6-well multiwell tissue culture trays (Falcon No. 3046). IBRV-FMD cl.1A and IBRV(NG)dltk were sonicated for 1 min on ice, then diluted in APMEM and 10% (v/v) fetal calf serum to about 2,000 p.f.u./ml. The guinea pig anti-FMDV peptide, guinea pig normal sera, polyclonal anti-IBRV and fetal calf serum control were diluted 1:4, 1:8, and 1:16 in 1 X PBS (with $MgCl_2$ and $CaCl_2$).

An equal volume of diluted virus and diluted sera was mixed, then incubated at 37°C for 1 hr. Then, 0.1 ml per well inoculum of virus plus serum was added and absorbed for 1 hr at 34.5°C. The infected cell monolayers were overlaid with 1.0% (w/v) methylcellulose in growth medium. After incubation for 4 days at 34.5°C, the media was aspirated, and the wells rinsed with 1 X GKN. The cells were fixed with ethanol for 5 min at room temperature, and stained with 0.1% (w/v) crystal violet for 5 min. Then, the wells were rinsed with tap water, dried, and plaques counted.

The results are shown in Tables 7 and 8 below.

### Table 7
### Virus Neutralization of IBRV(NG)dltk

| Anti-sera | Anti-sera Dilution | Plaque Count | Average Number of Plaques | Percent Inhi-bition |
|---|---|---|---|---|
| Normal Guinea Pig Sera | No sera | 254-298-308 | 287 | --- |
|  | 1:4 | 269-260-270 | 266 | 7% |
|  | 1:8 | 260-308-295 | 288 | 0% |
|  | 1:16 | 279-288-297 | 288 | 0% |
| Guinea Pig Anti-FMDV Sera | No sera | 284-274-296 | 284 | --- |
|  | 1:4 | 340-348-342 | 343 | <0% |
|  | 1:8 | 329-317-316 | 312 | <0% |
|  | 1:16 | 309-295-311 | 305 | <0% |
| Anti-IBRV Sera | No sera | 279-257-239 | 258 | --- |
|  | 1:4 | 0-0-0 | 0 | 100% |
|  | 1:8 | 2-0-1 | 1 | 99% |
|  | 1:16 | 9-17-13 | 13 | 95% |
| Fetal Calf Sera | No sera | 244-314-235 | 264 | --- |
|  | 1:4 | 264-261-242 | 256 | 3% |
|  | 1:8 | 236-250-232 | 239 | 9% |
|  | 1:16 | 269-281-234 | 261 | 1% |

$$* \text{ Percent Inhibition} = 100 - 100 \times \frac{\text{Plaque count sera}}{\text{Plaque count with no sera}}$$

Table 8
Virus Neutralization of IBRV-FMD

| Anti-sera | Anti-sera Dilution | Plaque Count | Average Number of Plaques | Percent Inhi-bition |
|---|---|---|---|---|
| Normal Guinea Pig Sera | No sera | 93-103-74 | 90 | --- |
| | 1:4 | 125-120-113 | 119 | <0% |
| | 1:8 | 118-99-111 | 109 | <0% |
| | 1:16 | 97-107-108 | 104 | <0% |
| Guinea Pig Anti-FMDV Sera | No sera | 127-112-96 | 112 | --- |
| | 1:4 | 23-44-26 | 27 | 75% |
| | 1:8 | 55-58-58 | 57 | 49% |
| | 1:16 | 66-93-60 | 73 | 35% |
| Anti-IBRV Sera | No sera | 81-82-93 | 85 | --- |
| | 1:4 | 0-0-0 | 0 | 100% |
| | 1:8 | 4-4-3 | 4 | 95% |
| | 1:16 | 20-23-20 | 21 | 75% |
| Fetal Calf Sera | No sera | 98-82-89 | 90 | --- |
| | 1:4 | 99-86-85 | 90 | 0% |
| | 1:8 | 80-69-90 | 80 | 9% |
| | 1:16 | 105-89-95 | 96 | <0% |

$$* \text{ Percent Inhibition} = 100 - 100 \times \frac{\text{Plaque count sera}}{\text{Plaque count with no sera}}$$

As shown in Tables 7 and 8 above, the guinea pig anti-FMD peptide sera neutralized the infectivity of IBRV-FMD cl.1A, but not the infectivity of IBRV(NG)dltk. On the other hand, guinea pig normal serum had no neutralizing activity against either virus.

In addition, as shown in Tables 7 and 8 above, the polyclonal anti-IBRV sera neutralized the infectivity of both IBRV-FMD cl.1A and IBRV(NG)dltk, whereas the normal fetal calf sera had no neutralizing activity against either virus.

These results provide functional evidence of the presence of FMDV antigen on the surface of IBRV-FMD cl.1A.

EXAMPLE 4

Experiments in Calves to Evaluate Safety and Induction of Antibodies by IBRV-FMDV cl.1A

Seven Holstein calves, each weighing about 600 pounds, were tested and shown to be negative to IBRV by the virus neutralization test. The calves were divided into the following groups and vaccinated as indicated below.

Group A. Calves Nos. 905 and 906 were each immunized intravenously (IV) with 2.0 ml of the IBRV-FMD cl.1A (about $10^8$ p.f.u./calf) and challenged intranasally on day 35 post vaccination (PV) with 2.0 ml of the Cooper strain of IBRV, furnished by the USDA (virus titer of $10^{7.49}$ TCID$_{50}$/ml).

Group B. Calves Nos. 903 and 904 were each immunized intramuscularly (IM) with 2.0 ml of IBRV-FMD cl.1A and challenged intranasally on day 35 PV with 2.0 ml of the Cooper strain of IBRV, furnished by the USDA (virus titer of $10^{7.49}$ TCID$_{50}$/ml).

Group C. Calves Nos. 901 and 902 were each immunized IM with 2.0 ml of IBRV-FMD cl.1A as with Group B, but Group C calves were revaccinated on day 35 PV with 2.0 ml of IBRV-FMD cl.1A (about 108 p.f.u./calf) and not challenged with 2.0 ml of the Cooper strain of IBRV, furnished by the USDA (virus titer of $10^{7.49}$ TCID$_{50}$/ml).

Group D. Calf No. 907 used as a control was not vaccinated, but challenged on days 35 PV with 2.0 ml of the Cooper strain of IBRV, furnished by the USDA (virus titer of $10^{7.49}$ TCID$_{50}$/ml).

The calves were observed daily for clinical signs of IBR (respiratory distress, rapid breathing, nasal or ocular discharge, or nasal ulcerations). No clinical signs were noted in the vaccinated calves. All of the animals continued to eat normally and appeared normal, except for Calf No. 903, which was a chronic bloater. On necropsy, the mediastinal lymph nodes were found to be enlarged, creating pressure on the esophagus, thereby preventing normal eructation of rumen gas.

Table 9 below shows the PV temperature observations.

Table 9
Immunization of Calves With IBRV-FMDV cl.1A:
Temperature Observations Post-Vaccination *

| Day | 901 (IM) | 902 (IM) | 903 (IM) | 904 (IM) | 905 (IV) | 906 (IV) |
|---|---|---|---|---|---|---|
| -4 | 100.7 | 100.6 | 100.8 | 101.3 | 100.6 | 101.3 |
| -3 | 100.9 | 100.9 | 101.3 | 101.2 | 101.7 | 102.5 |
| -2 | 101.0 | 100.6 | 101.2 | 101.4 | 101.3 | 100.9 |
| -1 | 100.6 | 101.4 | 102.3 | 101.0 | 100.6 | 101.5 |
| **0 | 99.8 / 100.6 | 100.4 / 101.3 | 100.6 / 101.8 | 100.1 / 100.6 | 100.7 / 101.2 | 100.7 / 101.6 |
| 1 | 102.8 / 103.0 | 103.1 / 102.6 | 104.7 / 105.1 | 102.0 / 102.6 | 104.5 / 105.1 | 100.0 / 102.7 |
| 2 | 101.5 / 102.0 | 101.4 / 103.2 | 103.2 / 104.0 | 101.1 / 102.0 | 100.4 / 101.4 | 100.6 / 100.6 |
| 3 | 102.0 / 103.5 | 102.8 / 102.6 | 103.3 / 104.3 | 101.9 / 105.5 | 101.2 / 105.6 | 104.1 / 103.4 |
| 4 | 101.4 / 100.8 | 100.6 / 101.6 | 104.3 / 103.8 | 101.3 / 103.2 | 103.0 / 102.5 | 104.2 / 103.8 |
| 5 | 101.1 / 101.0 | 101.0 / 101.5 | 103.2 / 103.5 | 101.0 / 102.5 | 103.1 / 103.5 | 102.4 / 102.5 |
| 6 | 102.4 / 102.2 | 101.0 / 101.0 | 103.8 / 103.3 | 101.1 / 101.2 | 102.4 / 102.8 | 102.2 / 102.6 |
| 7 | 101.0 / 100.6 | 100.4 / 100.4 | 103.4 / 105.2 | 101.8 / 102.3 | 101.4 / 102.2 | 101.6 / 102.5 |
| 8 | 101.3 / 101.3 | 101.1 / 102.0 | 105.6 / 106.0 | 104.6 / 106.3 | 103.2 / 104.2 | 102.7 / 102.8 |
| 9 | 105.4 / 104.2 | 103.6 / 103.4 | 102.6 / 102.8 | 103.8 / 103.0 | 105.2 / 106.2 | 101.5 / 102.0 |

---

*     Temperatures taken daily, a.m. is top line of Table 9 and p.m. is bottom line of Table 9.

**     Day of Vaccination

## Table 9 (Continued)

| Day | 901 (IM) | 902 (IM) | 903 (IM) | 904 (IM) | 905 (IV) | 906 (IV) |
|-----|------|------|------|------|------|------|
| 10 | 101.2 | 100.4 | 101.7 | 102.6 | 105.0 | 101.4 |
|    | 102.6 | 102.5 | 101.9 | 102.6 | 102.7 | 104.7 |
| 11 | 101.4 | 101.2 | 101.3 | 101.0 | 101.0 | 101.2 |
|    | 102.0 | 102.8 | 101.8 | 101.5 | 101.9 | 101.2 |
| 12 | 100.6 | 101.0 | 101.5 | 100.7 | 101.2 | 100.7 |
|    | 100.1 | 101.0 | 101.4 | 101.8 | 100.8 | 101.0 |
| 13 | 100.2 | 100.5 | 99.9 | 101.2 | 100.7 | 101.2 |
|    | 101.0 | 100.2 | 101.0 | 101.6 | 101.4 | 101.6 |
| 14 | 100.2 | 100.2 | 100.1 | 100.9 | 100.7 | 101.0 |
|    | 100.4 | 100.2 | 101.0 | 101.2 | 101.0 | 101.2 |

As shown in Table 9, all of the calves showed varying degrees of temperature elevation, indicative of viral reproduction by the nature of the pyrexia.

Table 10 below shows the PV leucocyte counts.

## Table 10
### Immunization of Calves With IBRV-FMDV cl.1A
### Leukocyte Counts Post-Vaccination

| Day | 901 (IM) | 902 (IM) | 903 (IM) | 904 (IM) | 905 (IV) | 906 (IV) |
|-----|----------|----------|----------|----------|----------|----------|
| -3 | 7.2 | 9.4 | 8.4 | 9.0 | 7.3 | 7.7 |
| -2 | 8.7 | 11.7 | 8.1 | 8.3 | 7.1 | 9.4 |
| -1 | 7.5 | 10.5 | 8.4 | 8.1 | 8.3 | 7.9 |
| *0 | 7.7 | 10.3 | 7.5 | 8.4 | 7.3 | 8.7 |
| 1 | 5.3 | 7.4 | 6.0 | 6.5 | 5.6 | 8.3 |
| 2 | 7.4 | 10.0 | 6.9 | 8.7 | 7.6 | 4.4 |
| 3 | 8.6 | 10.5 | 9.0 | 9.1 | 5.2 | 5.9 |
| 4 | 7.2 | 9.4 | 7.7 | 5.5 | 5.6 | 7.9 |
| 5 | 4.7 | 6.2 | 5.7 | 5.4 | 5.1 | 6.7 |
| 6 | 4.3 | 5.5 | 5.1 | 5.1 | 3.4 | 5.2 |
| 7 | 4.8 | 5.1 | 5.9 | 4.0 | 4.4 | 4.9 |
| 8 | 5.4 | 5.2 | 6.2 | 3.4 | 3.9 | 5.3 |
| 9 | 5.9 | 10.6 | 8.9 | 5.8 | 4.9 | 6.9 |
| 10 | 9.7 | 11.4 | 9.7 | 7.8 | 8.6 | 7.0 |
| 11 | 9.7 | 12.5 | 7.6 | 7.1 | 11.6 | 7.0 |

\*     Day after challenge.

## Table 10 (Continued)

| Day | 901 (IM) | 902 (IM) | 903 (IM) | 904 (IM) | 905 (IV) | 906 (IV) |
|---|---|---|---|---|---|---|
| 12 | 7.3 | 8.8 | 8.9 | 6.9 | 6.8 | 7.1 |
| 13 | 7.4 | 9.7 | 7.1 | 8.3 | 6.7 | 7.7 |
| 14 | 7.8 | 10.4 | 7.4 | 7.7 | 6.5 | 7.2 |
| | **7.8 | 10.5 | 8.1 | 8.5 | 7.5 | 8.4 |

** Average leukocyte count 4 days before vaccination.

As shown in Table 10 above, all of the calves showed some degree of leukopenia, also indicative of PV virus replication.

Tables 11 and 12 below show the temperature and leucocyte counts of the calves challenged with IBRV(Cooper) (Calf Nos. 903-906), and the response after a second booster vaccination (Calf Nos. 901 and 902).

### Table 11
### Immunization of Calves IBRV-FMDV cl.1A
### Temperature Observations Post-Challenge*

| Day | 901 (IM) | 902 (IM) | 903 (IM) | 904 (IM) | 905 (IV) | 906 (IV) |
|---|---|---|---|---|---|---|
| -3 | 99.6 | 99.7 | 99.6 | 100.7 | 100.6 | 99.5 |
| -2 | 99.9 | 100.1 | 100.4 | 100.8 | 100.7 | 100.3 |
| -1 | 100.1 | 99.7 | 101.6 | 101.2 | 101.0 | 100.9 |
| **0 | 100.0 | 100.5 | 100.4 | 100.6 | 100.4 | 99.5 |
|  | 100.2 | 100.6 | 100.0 | 100.1 | 100.8 | 100.2 |
| 1 | 100.3 | 100.3 | 100.3 | 101.6 | 99.8 | 101.3 |
|  | 100.6 | 100.3 | 100.4 | 102.0 | 100.6 | 101.7 |
| 2 | 99.5 | 99.5 | 102.0 | 100.6 | 100.8 | 100.9 |
|  | 100.8 | 100.7 | 102.6 | 102.0 | 101.2 | 102.1 |
| 3 | 98.8 | 99.1 | 101.2 | 101.2 | 100.1 | 100.0 |
|  | 99.9 | 100.6 | 101.8 | 100.8 | 101.0 | 101.1 |
| 4 | 97.8 | 98.0 | 102.6 | 102.1 | 100.1 | 100.6 |
|  | 98.6 | 99.5 | 102.1 | 102.1 | 100.7 | 101.0 |
| 5 | 99.6 | 100.0 | 99.6 | 99.4 | 100.4 | 100.6 |
|  | 100.2 | 100.2 | 100.6 | 100.4 | 99.8 | 100.4 |
| 6 | 99.5 | 99.3 | 100.4 | 100.0 | 100.3 | 100.4 |
|  | 100.0 | 100.2 | 101.0 | 101.2 | 99.7 | 100.2 |
| 7 | 99.6 | 99.9 | 101.0 | 99.5 | 99.7 | 99.4 |
|  | 99.5 | 100.6 | 100.6 | 99.6 | 99.5 | 100.3 |
| 8 | 100.5 | 99.9 | 100.8 | 100.8 | 99.4 | 98.3 |
|  | 100.2 | 100.0 | 100.3 | 100.1 | 100.1 | 100.4 |
| 9 | 100.1 | 100.0 | 99.3 | 100.2 | 99.9 | 99.8 |
|  | 100.4 | 100.7 | 99.8 | 100.4 | 99.5 | 100.4 |
| 10 | 100.3 | 98.5 | 99.4 | 100.4 | 100.0 | 98.5 |
|  | 99.7 | 100.3 | 100.2 | 100.3 | 100.1 | 100.1 |

---

\*    Temperatures taken daily; a.m. is top line of
Table 11 and p.m. is bottom line of Table 11.

\*\*   Day after Challenge.

## Table 11 (Continued)

| Day | 901 (IM) | 902 (IM) | 903 (IM) | 904 (IM) | 905 (IV) | 906 (IV) |
|-----|----------|----------|----------|----------|----------|----------|
| 11  | 99.1     | 99.7     | 98.2     | 100.4    | 99.3     | 99.7     |
|     | 100.3    | 100.7    | 100.0    | 101.0    | 100.8    | 99.9     |
| 12  | 99.8     | 99.4     | 99.8     | 100.2    | 100.6    | 100.2    |
|     | 100.3    | 100.5    | 100.5    | 100.2    | 100.7    | 100.3    |
| 13  | 100.1    | 100.3    | 99.4     | 100.6    | 100.7    | 99.9     |
|     | 101.2    | 100.6    | 100.5    | 101.5    | 100.5    | 99.3     |
| 14  | 101.0    | 100.2    | 99.5     | 100.5    | 99.8     | 99.4     |

## Table 12
### Leucocyte Counts Post Challenge

| Day | 901 (IM) | 902 (IM) | 903 (IM) | 904 (IM) | 905 (IV) | 906 (IV) |
|---|---|---|---|---|---|---|
| -3 | 7.1 | 11.7 | 8.1 | 8.7 | 7.1 | 8.2 |
| -2 | 8.2 | 11.8 | 9.4 | 9.1 | 7.6 | 8.7 |
| -1 | 8.3 | 12.6 | 12.1 | 9.2 | 7.6 | 9.3 |
| *0 | 11.1 | 12.1 | 8.3 | 8.9 | 8.2 | 6.9 |
| 1 | 8.6 | 13.6 | 7.3 | 9.1 | 12.1 | 8.4 |
| 2 | 9.1 | 12.3 | 9.1 | 10.3 | 7.1 | 8.7 |
| 3 | 7.9 | 11.5 | 6.2 | 7.9 | 7.5 | 8.4 |
| 4 | 7.3 | 11.3 | 6.4 | 7.9 | 5.9 | 7.8 |
| 5 | 7.8 | 11.4 | 5.6 | 10.3 | 4.8 | 7.7 |
| 6 | 7.2 | 11.6 | 9.2 | 9.2 | 6.8 | 7.7 |
| 7 | 7.6 | 10.6 | 6.6 | 8.1 | 6.2 | 7.5 |
| 8 | 7.7 | 11.3 | 5.6 | 8.8 | 7.4 | 7.9 |
| 9 | 10.5 | 12.0 | 6.2 | 8.8 | 8.3 | 10.2 |
| 10 | 9.4 | 11.0 | 7.0 | 4.1 | 10.2 | 8.8 |
| 11 | 8.7 | 12.0 | 6.2 | 8.6 | 9.9 | 5.6 |
| 12 | 8.7 | 11.7 | 6.2 | 8.3 | 7.1 | 8.6 |
| 13 | 10.8 | 11.8 | 6.0 | 8.3 | 7.1 | 9.1 |
| 14 | 8.3 | 10.8 | 6.6 | 9.0 | 7.9 | 8.8 |

\*      Day after challenge.

As shown in Tables 11 and 12, the results indicate a protective response, except for Calf No. 903 which developed an enlarged lymph node in the area of the esophagus that resulted in chronic bloating, and stress. No animals showed any clinical signs of disease. Table 11 represents the temperature observations in the vaccinated calves and indicate that vaccinated calves were protected from pyrexia.

Tables 13 below shows the leukocyte counts and temperature observations in control Calf No. 907.

**Table 13**
**Leukocyte Count and Temperature**
**Chart of non-vaccinated Control Calf No. 907**
**<u>Before and After Challenge*</u>**

| <u>Date</u> | <u>Leukocyte Count</u> | <u>Temperature</u> |
|---|---|---|
| 2-12 | 12.1 | ** |
| 2-13 | 11.2 | |
| 2-14 | <u>10.4</u> | |
| *** 2-15 | 10.1 | |
| 2-16 | 14.2 | 101.0 |
| 2-17 | 11.8 | 100.8 |
| | | 102.1 |
| 2-18 | 12.6 | 104.2 |
| | | 103.1 |
| 2-19 | 11.8 | 103.6 |
| | | 104.5 |
| 2-20 | 13.9 | 103.0 |
| | | 103.5 |
| 2-21 | 12.9 | 102.0 |
| | | 103.2 |
| 2-22 | 14.0 | 99.5 |
| | | 108.8 |
| 2-23 | 10.1 | 100.6 |
| | | 101.0 |
| 2-24 | 9.3 | 99.3 |
| | | 100.2 |
| 2-25 | 9.2 | 98.3 |
| | | 100.2 |

\*   Temperatures taken daily, a.m. is top line
of Table 13 and p.m. is bottom line of
Table 13.

\*\*   Temperature recording was not started
until 1 day after challenge.

\*\*\*   Day of challenge.

## <u>Table 13</u> (Continued)

| <u>Date</u> | <u>Leucocyte</u> | <u>Temp</u> |
|---|---|---|
| 2-26 | 10.3 | 100.0 |
| | | 101.2 |
| 2-27 | 11.1 | 99.8 |
| | | 100.2 |
| 2-28 | 12.5 | 100.5 |
| | | 100.3 |
| 3-1 | <u>13.0</u> | 100.5 |
| | **** 11.2 | |

**** Average leukocyte count of days 12-14.

As shown in Tables 13 above, non-vaccinated control Calf No. 907 exhibited a temperature elevation from 4 to 11 days after challenge.

Table 14 below shows the ELISA titers of calves vaccinated with the IBRV-FMDV cl.1A.

Table 14
Enzyme-linked Immunosorbent Assay (ELISA) for Antibodies
to the FMDV Peptide of Sera from Calves Immunized with IBRV-FMDV cl.1A

| Inj. Route | Calf No. | Pre-Inoculation | ELISA to FMDV (Days post vaccination) | | | | | | |
|---|---|---|---|---|---|---|---|---|---|
| | | | <u>7</u> | <u>14</u> | <u>21</u> | <u>28</u> | <u>35**</u> | <u>42</u> | <u>49</u> |
| IV | 905 | 0.30* | 0.26 | 0.82 | <u>1.89</u> | <u>1.79</u> | 0.75 | 0.68 | <u>1.76</u> |
| IV | 906 | 0.28 | 0.33 | <u>1.94</u> | <u>2.16</u> | <u>2.04</u> | 0.81 | 0.42 | 0.58 |
| IM | 903 | 0.35 | 0.34 | 0.70 | <u>2.88</u> | <u>1.57</u> | 0.92 | <u>1.75</u> | 0.48 |
| IM | 904 | 0.34 | 0.30 | 0.99 | <u>1.91</u> | <u>1.75</u> | 0.48 | 0.44 | 0.61 |
| IM | 901 | 0.28 | 0.53 | 0.58 | 0.70 | 0.72 | <u>1.72</u> | 0.67 | 0.40 |
| IM | 902 | 0.53 | 0.36 | <u>1.84</u> | <u>2.54</u> | <u>2.37</u> | <u>1.90</u> | <u>1.82</u> | 0.67 |
| | 907+ | --- | --- | --- | --- | --- | 0.62 | 0.67 | 0.97 |

*   The results listed above are the $\log_{10}$ the reciprocal of the antipeptide antibody titers of calf serum samples evaluated by ELISA. Titers ≥ or > 1.699 are given as logs and underlined. Lesser values are given as O.D. readings at 1:50 serum dilution.

**   All calves were challenged IM on day 35 PV with IBRV (Cooper), except for Calf Nos. 901 and 902, which were immunized a second time with IBRV-FMDV cl.1A.

+   Control calf not vaccinated.

As shown in Table 14 above, antibodies to the FMDV epitope were produced, thereby demonstrating the expression of the FMDV epitope by IBRV-FMDV cl.1A. These levels of antibody are at the level where protection from FMDV challenge would be expected.

Figure 7 shows anti-FMDV neutralization titers (SNT) of Calves Nos. 901 to 906 after vaccination (day 0) with IBRV-FMDV cl.1A and challenge with IBRV(Cooper) on day 35 (C) or immunized a second time on day 35 with IBRV-FMDV cl.1A (B). The SNT titers confirm the induction of protective anti-FMDV antibodies by the immunized calves.

The above results demonstrate that IBRV-FMDV cl.1A is able to induce protection in calves to IBRV challenge and that the calves develop antibody titers indicative of protection levels to FMDV.

EXAMPLE 5

Immunization of Pigs with IBRV-FMDV cl.1A

Pigs are highly susceptible to FMDV. Since IBRV has occasionally been isolated from pigs (Kit et al., "High Technology Route to Virus Vaccines" (An ASM Publication), p. 82-99 (1985)), the possibility was investigated that anti-FMDV antibodies might be induced in pigs immunized with the recombinant IBRV-FMDV cl.1A which expresses the FMDV epitope.

Thirty pigs were placed in each of three rooms of a containment facility. The pigs were divided into the following groups (A to C) and vaccinated as indicated below.

Group A. 10 pigs were each vaccinated IM with 2.0 ml of IBRV-FMDV cl. 1A (8.0 p. f. u.).

Group B. 10 pigs were each vaccinated IM behind each ear with 5.0 ml of IBRV-FMDV cl.1A (20 p.f.u.) (total of 10 ml each) of IBRV-FMDV cl.1A.

Group C. 10 pigs were each vaccinated IM behind the ear with 4.0 ml of IBRV-FMDV cl.1A emulsified with Adjuvant "A" to give 2.0 ml of virus (8.0 p.f.u.) per 4.0 ml dose. In this immunization, 30 ml of IBRV-FMDV cl.1A was emulsified with Adjuvant "A" comprising 26.7 ml of Drakeol 6-VR (Pennsylvania Oil Co.), 3.0 ml of Seppic 888 and 0.3 ml of Emulgin 05.

To compare the induction of antibodies by IBRV-FMDV cl.1A with the induction of antibodies by the synthetic FMDV epitope ($O_1K$ serotype) (U.S. Patent 4,732,971; and Doel et al, J. Virol., 64:2260-2264 (1990)), Group D, comprising 10 pigs, were each vaccinated IM behind the ear with 2.0 ml of the synthetic FMDV epitope (133 µg) emulsified with Adjuvant "A". In this inoculation, 200 µl of 10 mg/ml of FMDV epitope solution and 14.8 ml of PBS was emulsified with 15 ml of Adjuvant "A" comprising 13.35 ml Drakeol 6-VR, 1.5 ml of Seppic 888, and 0.15 ml of Emulgin 05.

Blood samples for serum were taken prior to vaccination, and at 7, 14, 21, 28, and 35 days PV. The results with serum taken at day 21 PV are shown in Table 15 below.

Table 15
ELISA and SNT Results on 21-Day Swine Sera vs. FMDV

| Group | Vaccine | Animal No. | SNT | ELISA* |
|-------|---------|------------|-----|--------|
| A | 2.0 ml of IBRV-FMDV c1.1A | 637 | 11 | 0.347 |
| | | 638 | 16 | 0.226 |
| | | 639 | 8 | 0.205 |
| | | 640 | <6 | 0.172 |
| | | 641 | 11 | 0.228 |
| | | 642 | 8 | 0.671 |
| | | 643 | 16 | 0.558 |
| | | 644 | <6 | 0.602 |
| | | 645 | <6 | 0.781 |
| | | 646 | <6 | 0.257 |
| B | 10 ml of IBRV-FMDV c1.1A | 647 | 16 | 1.994 |
| | | 648 | ND | 0.513 |
| | | 649 | 8 | 0.412 |
| | | 650 | <6 | 0.365 |
| | | 651 | 8 | 0.410 |
| | | 652 | 45 | 1.750 |
| | | 653 | <6 | 0.314 |
| | | 654 | 11 | 0.510 |
| | | 655 | <6 | 0.311 |
| | | 656 | 32 | 1.978 |
| C | 2.0 ml of IBRV-FMDV c1.1A in 2.0 ml of Adjuvant A | 657 | 45 | 2.491 |
| | | 658 | 45 | 2.190 |
| | | 659 | 90 | 3.193 |
| | | 660 | 128 | 3.326 |
| | | 661 | 22 | 2.332 |
| | | 662 | 64 | 3.283 |
| | | 663 | 8 | 1.778 |
| | | 664 | 128 | 3.214 |
| | | 665 | 256 | 3.176 |
| | | 666 | 708 | 3.635 |

Table 15 (Continued)

| Group | Vaccine | Animal No. | SNT | ELISA* |
|-------|---------|-----------|-----|--------|
| D | 2.0 ml of FMDV $O_1K$ epitope in 15 ml of Adjuvant A | 667 | 32 | 2.562 |
| | | 668 | <6 | 0.178 |
| | | 669 | 8 | 2.273 |
| | | 670 | 64 | 2.785 |
| | | 671 | 22 | 2.875 |
| | | 672 | 32 | 2.491 |
| | | 673 | 128 | 3.602 |
| | | 674 | 45 | 2.491 |
| | | 675 | 64 | 2.973 |
| | | 676 | 90 | 2.851 |

* Log 10 of the reciprocal of the antibody titers of swine serum. Titers ≥ or > 1.699 are given as logs. Lesser values are given as O.D. readings at 1:50 serum dilution.

As shown in Table 15 above, protective levels (greater than 2.0) of FMDV antibodies, as measured by the ELISA, were induced in pigs vaccinated with IBRV-FMDV cl.1A emulsified with Adjuvant "A", but not by IBRV-FMDV cl.1A alone.

EXAMPLE 6

Evaluation of the Safety, Protection and Induction of Antibodies by IBRV-FMDV Cl.1A

Seventeen Danish cattle, approximately 12 months of age, were serologically tested and shown to be free of FMD and IBR/IPV antibodies. The cattle were divided into the following groups (A to C) and vaccinated as indicated below.

Group A. Cattle Nos. 9286, 9287, 9289 and 9290 were each vaccinated IM with 2.0 ml of IBRV-FMDV cl. 1A (about $10^8$ p.f.u./calf)and challenged at 21 days PV by intradermal administration of $10^4$ $TCID_{50}$ of $0_1$ Kaufbeuren ($O_1K$) virus.

Group B. Cattle Nos. 9291, 9292, 9293, 9294 and 9295 were each vaccinated IM with 4.0 ml of IBRV-FMD cl.1A ($10^8$ p.f.u./calf) emulsified with Adjuvant A (50:50), and challenged at 21 days PV as in Group A.

Group C. Cattle Nos. 9296, 9297, 9298, 9299 and 9300 were each vaccinated subcutaneously with 5.0 ml of trivalent inactivated FMDV vaccine (Coopers Animal Health, Ltd., Berkhamsted, England) and challenged at 21 days PV as in Group A.

Group D. Cattle Nos. 9301 and 9302 were held as nonvaccinated controls and challenged at 21 days as in Group A.

The cattle were observed daily for temperature and signs of rhinitis for 20 days PV. Seven days after challenge, all of the cattle were killed and checked for clinical FMD lesions. Serological studies were also run on all of the animals. The results are shown in Table 16 below.

Table 16
IBRV-FMDV cl.1A Challenge

| Group | Animal No. | FMD Challenge Results 7 days post Challenge | | | | | Protection | 21-day Serology | Anti-Virus | Anti-Peptide |
|---|---|---|---|---|---|---|---|---|---|---|
| | | PA | RF | LF | RH | LH | | SNT | | |
| A IBRV-FMDV cl.1A | 9286 | + | + | + | + | + | - | 22 | 36 | <20 |
| | 9287 | + | - | - | - | - | + | 22 | 302 | <20 |
| | 9288 | + | - | - | - | - | + 2/5 protected | 22 | 277 | 20 |
| | 9289 | + | + | + | + | + | - | 16 | 510 | <20 |
| | 9290 | + | + | + | + | + | - | 64 | 109 | <20 |
| B IBRV-FMDV cl.1A + Adju-vant A | 9291 | + | - | - | - | - | + | 22 | 47 | <20 |
| | 9292 | + | - | + | + | + | - | 11 | 28 | <20 |
| | 9293 | + | - | - | - | + | - 1/5 protected | 32 | 495 | 22 |
| | 9294 | + | - | + | + | + | - | 45 | 4012 | 25 |
| | 9295 | + | - | - | + | + | - | 22 | 168 | 20 |
| C Inacti-vated Vaccine | 9296 | - | - | - | - | - | + | 64 | 579 | 21 |
| | 9297 | + | - | - | - | - | + | 90 | 2214 | 11 |
| | 9298 | - | - | - | - | - | + 5/5 protected | 64 | 1401 | 316 |
| | 9299 | - | - | - | - | - | + | 90 | 351 | 17 |
| | 9300 | - | - | - | - | - | + | 90 | 1073 | 408 |
| D Unvac-cinated Controls | 9301 | + | + | + | + | + | - 0/2 protected | - | - | - |
| | 9302 | + | + | + | + | + | - | - | - | - |

PA - primary aphtae; RF - right forefoot; LF - left forefoot; RH - right hind; LH - left hind

As shown in Table 16 above, two of five cattle immunized with IBRV-FMDV cl. 1A demonstrated solid protection against a severe $O_1K$ needle challenge. In comparison, the trivalent inactivated FMDV vaccine protected all of the animals against challenge. In addition, significant antibody titers to FMD were observed in the IBRV-FMDV cl.1A immunized animals, although the development was slower than the trivalent inactivated vaccine and may not have reached peak titers at the time of challenge.

EXAMPLE 7

Lack of Detection of FMDV-gIII Fusion Sequence in Trigeminal Ganglia of Infected Calves

After wild-type IBRV infection, IBRV DNA can be detected in trigeminal ganglion during latency by in situ hybridization (Homan et al, Am. J. Vet. Res., 44:309-313). However, latent tk⁻ IBRV infection has never been demonstrated. Thus, latent infection of IBRV-FMDV cl.1A, a tk⁻ IBRV, was evaluated to determine whether the presence of FMDV-gIII fusion sequences could be found in trigeminal ganglion.

More specifically, calves from Example 4 were sacrificed and the trigeminal ganglion excised and placed at -70°C for storage. Approximately 100-200 mg of tissue was process for DNA extraction essentially as described by Hogan et al, Manipulating the Mouse Embryo, Cold Spring Harbor Laboratory, p. 175-176 (1986). The resulting DNA was resuspended in approximately 200 μl of water. 2.0 μl of this DNA suspension was added to 20 μl of PCR reaction mixtures comprising 4.0 pmoles each of primers:

```
Oligo EJ48     5' - CCCTCCGTGCCCAACTTGAGAGG

Oligo EJ47     5' - CGGCGGCGTGCTGTTGG

Oligo EJ49     5' - TGACCTTCAGGTGTTGGCTCAAAAGGTGG

Oligo EJ50     5' - GGCGGGTCGTGCTCGGACACGG
```

in 10 mM Tris-HCl (pH 8.3), 50 mM KCl, 1.5 mM MgCl₂, 0.01% (w/v) gelatin (Difco) and 200 μM dCTP, dATP, dTTP and deazaGTP:dGTP (3:1) (McConlogue et al, Nucl. Acid. Res., 16:9869 (1988)), and 0.5 units of Taq polymerase. The resulting reaction mixture was overlayed with 15 μl of mineral oil for 40 or 45 cycles (95°C, 30 sec, 65°C, 30 sec, 72°C, 60 sec). An aliquot of each reaction mixture was analyzed on a 4.0% (w/v) NuSieve (FMC, Rockland, ME): SeaKem (FMC, Rockland, ME) (3:1) agarose gel and Southern blot analysis was performed.

The primers were designed to copy a unique sequence of IBRV-FMDV cl.1A. All of the primers were synthesized by Research Genetics, Huntsville, Alabama. Oligo EJ48 was used to prime polymerase activity from the 5' FMDV sequence. Oligo EJ47 was used to prime polymerase activity from the 3' IBRV gIII sequence. Oligo EJ49 and Oligo EJ50 are were used to prime polymerase activity internal to Oligo EJ48 and Oligo EJ47.

After 40 PCR cycles, no DNA product of a predicted size, 251 bp, was detected in the DNA from IBRV-FMDV cl.1A-infected calves. None of the PCR products of any size hybridized to a PCR-generated probe using Oligo EJ49 or Oligo EJ50. In order to determine copy number, included in this analysis were DNAs extracted from trigeminal ganglia tissue on a different day and a dilution of target DNA: RF M13mp18/gIIIFM1 BamHI-Sac I with and without DNA from Calf No. 90. 2-20 copies of target DNA was detected in 1 x 10⁵ cell equivalents (approximately 2.0 μg of DNA).

Thus, if latency occurred using IBRV-FMDV cl.1A, the viral DNA was not detected, by the highly sensitive technique involving PCR-genetated probes, in the tissue of reported latency 45 days post infection.

While this invention has been described in detail with reference to specific embodiments thereof, it will be apparent to one skilled in the art that various changes and modifications can be made therein without departing from the spirit and scope thereof.

## Claims

1. A herpesvirus-based viral vector which expresses a foot and mouth disease virus epitope on the surface of virus-infected cells and on the surface of virus particles.

2. The herpesvirus-based viral vector as claimed in Claim 1, wherein said herpesvirus is selected from the group consisting of IBRV, BHV-4, PRV, CapHV-1, AHV-1, or BHV-2.

3. The herpesvirus-based viral vector as claimed in Claim 2, wherein said IBRV is derived from a strain selected from the group consisting of IBRV(B8-D53) ATCC No. VR-2066), IBRV(NG)dltk (ATCC No. VR-2112), Los Angeles (ATCC No. VR-188), Cooper (ATCC No. VR-864), IPV strain K2, MO3, MO6, BFN-IH, BFN-IID, Gi 1 to 5, Bi, B4, BRV, LAE, V3 415, V3 416, V3 18, V3 93, BFA Wabu strain, strain P8-2, P10, P34 or alberta (Canada) isolates Nos. 1 to No. 122.

4. The herpesvirus-based viral vector as claimed in Claim 1, wherein said foot and mouth disease virus epitope corresponds to that of a member selected from the group consisting of serotype $O_1K$, serotype A, serotype O, serotype C, serotype Asia-1 or serotype South african Territories 1, 2, or 3.

5. The herpesvirus-based viral vector as claimed in Claim 4, wherein said foot and mouth disease virus epitope corresponds to amino acids (200-213)-(proline-proline-serine)-(141-158) of the $O_1K$ epitope.

6. The herpesvirus-based viral vector as claimed in Claim 1, wherein said vector is selected from the group consisting of virus having the identifying characteristics of IBRV-FMDV cl.1A (ATCC No. VR-2236) or virus having the identifying characteristics of IBRV-FMDV(Dimer) cl.3 (ATCC No. VR-2271).

7. A vaccine against foot and mouth disease comprising:
(A) a pharmaceutically effective amount of a herpesvirus-based viral vector which expresses a foot and mouth disease virus epitope on the surface of virus-infected cells and on the surface of virus particles; and
(B) a pharmaceutically effective carrier or diluent.

8. The vaccine as claimed in Claim 7, wherein said herpesvirus is selected from the group consisting of IBRV, BHV-4, PRV, CapHV-1, AHV-1 or BHV-2.

9. The vaccine as claimed in Claim 8, wherein said IBRV is derived from a strain selected from the group consisting of IBRV(B8-D53) (ATCC No. VR-2066), IBRV(NG)dltk (ATCC No. VR-2112), Los Angeles (ATCC No. VR-188), Cooper (ATCC No. VR-864), IPV strain K2, MO3, MO6, BFN-IH, BFN-IID, Gi 1 to 5, Bi, B4, BRV, LAE, V3 415, V3 416, V3 18, V3 93, BFA Wabu strain, strain P8-2, P10, P34 or alberta (Canada) isolates Nos. 1 to No. 122.

10. The vaccine as claimed in Claim 7, wherein said foot and mouth disease virus epitope corresponds to that of a member selected from the group consisting of serotype $O_1K$, serotype A, serotype O, serotype C, serotype Asia-1 or serotype South african Territories 1, 2, or 3.

11. The vaccine as claimed in Claim 10, wherein said foot and mouth disease virus epitope corresponds to amino acids (200-213)-(proline-proline-serine)-(141-158) of the $O_1K$ epitope.

12. The vaccine as claimed in Claim 7, wherein said vector is selected from the group consisting of virus having the identifying characteristics of IBRV-FMDV cl.1A (ATCC No. VR-2236) or virus having the identifying characteristics of IBRV-FMDV(Dimer) cl.3 (ATCC No. VR2271).

13. The vaccine as claimed in Claim 7, wherein said pharmaceutically acceptable carrier or diluent is a physiological buffered media containing from 2.5 to 15% (v/v) serum which do not contain antibodies to the herpesvirus employed or to FMDV.

14. The vaccine as claimed in Claim 7, wherein said pharmaceutically acceptable amount is about $10^{5.0}$ to $10^8$ p. f. u. /animal.

15. A vaccine against foot and mouth disease comprising:
(A) a pharmaceutically effective amount of a herpesvirus-based viral vector which expresses a foot and mouth disease virus epitope on the surface of virus-infected cells and on the surface of virus particles produced by a process comprising:
(a) Constructing a hybrid plasmid containing a herpesvirus glycoprotein gene sequence and flanking sequences of a non-essential herpesvirus genetic locus;
(b) Constructing a hybrid DNA sequence encoding a signal sequence-foot and mouth disease virus epitope sequence;
(c) Inserting the signal sequence-foot and mouth disease virus epitope sequence of Step (b) into the resulting hybrid plasmid of Step (a) so as to obtain a hybrid plasmid containing the signal sequence-foot and mouth disease virus epitope sequence as the N-terminal sequence of the herpesvirus glycoprotein gene;
(d) Deleting N-terminal herpesvirus glycoprotein gene sequences unidirectionally from the resulting hybrid plasmid of Step (c) so as to remove the non-essential herpesvirus glycoprotein signal sequence, and so as to obtain a hybrid plasmid comprising DNA encoding a fusion protein comprising the signal sequence-FMDV epitope sequence in phase with the herpesvirus glycoprotein sequence and said flanking sequences;

(e) Constructing a recombinant herpesvirus which contains a DNA sequence encoding a promoter adjacent to a selectable gene inserted in said non-essential herpesvirus genetic locus and which expresses the selectable gene from the promoter;

(f) Co-transfecting the DNA of the resulting recombinant herpesvirus of Step (e) and the resulting hybrid plasmid of Step (d) into herpesvirus host cells; and

(g) Selecting for a recombinant herpesvirus which does not express the selectable gene so as to obtain a herpesvirus-based viral vector which expresses an FMDV epitope on the surface of virus-infected cells and on the surface of virus particles; and

(B) a pharmaceutically effective carrier or diluent.

16. The vaccine as claimed in Claim 15, wherein the hybrid DNA sequence of Step (b) encodes a signal sequence-FMDV epitope dimer so as to produce, in Step (g), a herpesvirus-based viral vector which expresses a foot and mouth disease virus epitope dimer on the surface of virus-infected cells and on the surface of virus particles.

17. The vaccine as claimed in Claim 15, wherein said glycoprotein gene sequence of Step (a) is selected from the group consisting of the IBRV gIII gene, the PRV gIII gene, the PRV gI gene or the PRV gp63 gene.

18. The vaccine as claimed in Claim 15, wherein said non-essential herpesvirus gene of Step (e) is selected from the group consisting of the IBRV tk gene, the IBRV gIII gene, the PRV tk gene, the PRV gIII gene, the PRV gp63 gene, the PRV gI gene or the PRV gX gene.

19. The vaccine as claimed in Claim 15 wherein said signal sequence employed in Step (b) is derived from a member selected from the group consisting of bGH, bovine immunoglobulin, IBRV gIII, BHV-1, gI, PRV gIII, PRV gp50, PRV 63, PRV gI or PRV gII.

20. The vaccine as claimed in Claim 15, wherein said promoter employed in Step (e) is selected from the group consisting of the IBRV tk gene promoter, the IBRV gIII gene promoter, the IBRV gI gene promoter, the BHV-2 tk gene promoter, the BHV-2 gB gene promoter, the PRV tk gene promoter, the PRV gX gene promoter, the PRV gp50 gene promoter, the PRV gII gene promoter or the PRV gIII gene promoter.

21. The vaccine as claimed in Claim 15, wherein said herpesvirus is selected from the group consisting of IBRV, BHV-4, PRV, CapHV-1, AHV-1 or BHV2.

22. The vaccine as claimed in Claim 21, wherein said IBRV is derived from a strain selected from the group consisting of IBRV(B8-D53) (ATCC No. VR-2066), IBRV(NG)dltk (ATCC No. VR-2112), Los Angeles (ATCC No. VR-188), Cooper (ATCC No. VR-864), IPV strain K2, MO3, MO6, BFN-IH, BFN-IID, Gi 1 to 5, Bi, B4, BRV, LAE, V3 415, V3 416, V3 18, V3 93, BFA Wabu strain, strain P8-2, P10, P34 or Alberta (Canada) isolates Nos. 1 to No. 122.

23. The vaccine as claimed in Claim 61, wherein said foot and mouth disease virus epitope corresponds to that of a member selected from the group consisting of serotype $O_1K$, serotype A, serotype O, serotype C, serotype Asia-1 or serotype South African Territories 1, 2, and 3.

24. The vaccine as claimed in Claim 23, wherein said foot and mouth disease virus epitope corresponds to amino acids (200-213)-(proline-proline-serine)-(141-158) of the $O_1K$ epitope.

25. The vaccine as claimed in Claim 15, wherein said pharmaceutically acceptable carrier or diluent is a physiological buffered media containing from 2.5 to 15% (v/v) serum which do not contain antibodies to the herpesvirus employed or to FMDV.

26. The vaccine as claimed in Claim 15, wherein said pharmaceutically acceptable amount is about $10^{5.0}$ to $10^8$ p.f.u./animal.

FIG. I

FIG. 2

FIG. 3

FIG. 4

FIG. 5

FIG. 6

FIG.7A

FIG.7B

FIG.7C